# EUROPEAN PATENT SPECIFICATION

(11) **EP 0 601 129 B1**
(45) Date of publication and mention of the grant of the patent: **15.11.2000**
(21) Application number: 92921799.0
(22) Date of filing: 28.08.1992
(51) Int. Cl.: C12Q 1/02, G01N 33/68

(54) **MORPHOGENIC PROTEIN SCREENING METHOD**
Screeningverfahren von morphogenischen Proteinen
PROCEDE DE TRIAGE DE PROTEINES MORPHOGENIQUES

(30) Priority: 30.08.1991 US 752861
(43) Date of publication of application: 15.06.1994
(60) Divisional application: 97202681.9
(73) Proprietor: CREATIVE BIOMOLECULES, INC., Hopkinton, MA 01748 (US)
(72) Inventor: SMART, John, E., Weston, MA 02193 (US); OPPERMANN, Hermann, Medway, MA 02053 (US); OZKAYNAK, Engin, Milford, MA 01757 (US); KUBERASAMPATH, Thangavel, Medway, MA 02053 (US); RUEGER, David, C., Hopkinton, MA 01748 (US); PANG, Roy, H., L., Etna, NH 03750 (US); COHEN, Charles, N., Medway, MA 02053 (US)
(74) Representative: Price, Vincent Andrew
(86) International application number: US9207359
(87) International publication number: WO9305172

(56) References cited:
- WO-A-90/00619
- WO-A-91/02744
- JOURNAL OF BONE AND MINERAL RESEARCH vol. 6, no. 7, July 1991, pages 767 - 777 H.ZHOU ET AL.
- PROCEEDINGS OF THE NATIONAL ACADEMY OF SCIENCES OF USA. vol. 86, June 1989, WASHINGTON US pages 4554 - 4558 K.LYONS ET AL. cited in the application
- PROCEEDINGS OF THE NATIONAL ACADEMY OF SCIENCES OF USA. vol. 88, May 1991, WASHINGTON US pages 4250 - 4254 S.-J. LEE
- BIOCHEMICAL AND BIOPHYSICAL RESEARCH COMMUNICATIONS. vol. 179, no. 1, 30 August 1991, DULUTH, MINNESOTA US pages 116 - 123 E. \ZKAYANAK ET AL.

## Description

The invention relates to a method of screening drugs for the ability to modulate the level in mammals of proteins which can induce tissue morphogenesis and to methods of determining which animal tissue(s) and/or cell types within a tissue express a particular morphogenic protein.

### Background of the Invention

Cell differentiation is the central characteristic of morphogenesis which initiates in the embryo, and continues to various degrees throughout the life of an organism in adult tissue repair and regeneration mechanisms. Members of the TGF-β superfamily include subfamilies of highly-related genes that now are suspected to play important roles in cell differentiation and morphogenesis during development and/or during adult life. For example, the Drosophila decapentaplegic gene product (DPP) has been implicated in formation of the dorsal-ventral axis in fruit flies; activins induce mesoderm and anterior structure formation in mammals; Müllerian inhibiting substance (MIS) may be required for male sex development in mammals; growth/differentiation factor-1 (GDF-1) has been implicated in nerve development and maintenance; other morphogenic proteins (BMP-2, -3, -4 and OP-1) induce bone formation.

The development and study of a bone induction model system has identified the developmental cascade of bone differentiation as consisting of chemotaxis of mesenchymal cells, proliferation of these progenitor cells, differentiation of cartilage, ossification and hypertrophy of this cartilaginous tissue, vascular invasion, bone formation, remodeling, and finally, marrow differentiation (Reddi (1981) Collagen Rel. Res. 1:209-206). This bone model system, which is studied in adult mammals, recapitulates the cascade of bone differentiation events that occur in formation of bone in the developing fetus. In other studies, the epithelium of the urinary bladder has been shown to induce new bone formation. Huggins (1931, Arch. Surg. 22:377-408) showed that new bone formation could be induced by surgical transplantation of urinary bladder epithelium onto the parietal fascia. Urist (1965, Science 150:893-899) demonstrated that implantation of demineralized bone segments resulted in endochondral bone formation. The latter study and observation suggested the existence of an osteogenic protein and that bovine diaphyseal bone was a source of enriched preparations of osteogenic protein (Sampath et al., J. Biol. Chem. 265:13198-13205, 1990; Urist, ibid; Reddi et al., Proc. Nat. Aca. Sci. 69:1601-1605, 1972; Sampath et al., Proc. Natl. Acad. Sci. 80:6591-6595, 1983). Proteins capable of inducing endochondral bone formation in mammals when implanted in association with a matrix now have been identified in a number of different mammalian species, as have the genes encoding these proteins, (see, for example, U.S. Patent No. 4,968,590. Human OP-1 DNA has been cloned from various cDNA and genomic libraries using a consensus probe (Ozkaynak et al., EMBO J. 9:2085-2093, 1990). Purified human recombinant OP-1, expressed in mammalian cells, has been shown to induce new bone formation in vivo. Like other members of the TGF-β superfamily, OP-1 is produced as a precursor, glycosylated, processed and secreted as a mature dimer. Mature OP-1 is cleaved at a maturation site following a sequence with the pattern of RXXR (Panganiban et al., Mol. Cell. Biol. 10:2669-2677, 1990).

The degree of morphogenesis in adult tissue varies among different tissues and depends on, among other factors, the degree of cell turnover in a given tissue. On this basis, tissues can be divided into three broad categories: 1) tissues with static cell populations such as nerve and skeletal muscle where there is little or no cell division and most of the cells formed during development persist throughout adult life and, therefore, possess little or no ability for normal regeneration after injury; 2) tissues containing conditionally renewing populations such as liver where there is generally little cell division but, in response to an appropriate stimulus or injury, cells can divide to produce daughters of the same differentiated cell type; and 3) tissues with permanently renewing populations including blood, bone, testes, and stratified squamous epithelia which are characterized by rapid and continuous cell turnover in the adult. Here, the terminally differentiated cells have a short life span and are replaced through proliferation of a distinct subpopulation of cells, known as stem or progenitor cells.

Lyons et al in P.N.A.S USA, 86, 4554-58, 1989 disclose a method comprising the culturing of F9 cells in the presence of retinoic acid and cAMP and the assessment of the effect of the said compounds by Northern Analysis on the cell RNA.

It is an object of this invention to provide a method of screening compounds which, when administered to a given tissue from a given organism, cause an alteration in the level of morphogenic protein ("morphogen") produced by the tissue. Such compounds, when administered systemically, will result in altered systemic or local levels of morphogenic activity. This morphogenic activity includes the ability to induce proliferation and sequential differentiation of progenitor cells, and the ability to support and maintain the differentiated phenotype or sequence of phenotypes through the progression of events that results in the formation of normal adult tissue (including organ regeneration). Thus, broadly, the invention provides a key to development of additional modalities of therapies involving modulation of morphogenic protein production in animals or adult mammals, e.g., humans, and consequent correction of conditions involving pathologic alteration of the balance of tissue cell turnover. Another object of the invention is to provide methodologies for identifying or selecting a combination of compound(s) which may increase a progenitor cell population in a mammal, stimulate progenitor cells to differentiate in vivo or in vitro, maintain the differentiated phenotype or sequence of phenotypes of a tissue, induce tissue-specific growth in vivo, or replace diseased or damaged tissues or organs in vivo. Another object of the invention is to determine the tissue(s) or organ(s) of origin of a given morphogen. Another object of the invention is to determine the specific cell type(s) within the tissue(s) or organ(s) of origin, or cell line(s) derived from the tissue(s), or organ(s) of origin, that is responsible for the synthesis and production of a given morphogen. These and other objects and features of the invention will be apparent from the description, drawing, and claims which follow.

### Summary of the Invention

The invention is defined by the appended claims - it features a method of screening candidate compounds for the ability to modulate the effective local or systemic concentration or level of morphogenic protein in an organism. The method is practiced by incubating one or more candidate compound(s) with epithelial cells from a test tissue type of an organism known to produce a given morphogen for a time sufficient to allow the compound(s) to affect the production, i.e., expression and/or secretion, of morphogen by the cells; and then assaying cells and the medium conditioned by the cells for a change in a parameter indicative of the level of production of the morphogenic protein. The procedure may be used to identify compounds showing promise as drugs for human use capable of increasing or decreasing morphogen production in vivo, thereby to correct or alleviate a diseased condition.

In a related aspect, the invention features a method of screening tissue(s) of an organism to assess whether or at what level cells of the tissue(s) produce a particular morphogen, thereby to determine a tissue(s) of origin of the morphogen. This permits selection of the tissue cell type to be used in the screening. As used herein, "tissue" refers to a group of cells which are naturally found associated, including an organ.

As an example of tissue(s) or organ(s) which produce high levels of morphogen relative to the level produced by other types of tissues, it has been discovered that OP-1, first found in bone tissue is produced at relatively high levels in cells derived from renal, e.g., kidney or bladder, or adrenal tissue; that GDF-1 is produced at relatively high levels in cells derived from nerve, e.g., brain tissue; that DPP is produced at relatively high levels in cells derived from one of the following drosophila tissues: dorsal ectoderm, epithelial imaginal disc, visceral mesoderm, or gut endoderm; that Vgr-1 is produced at relatively high levels in cells derived from mouse lung tissue; and that Vgl is produced at relatively high levels in cells derived from xenopus fetal endoderm tissue. In addition, BMP3 and CBMP2B transcripts have been identified in abundance in lung tissue. As used herein, "derived" means the cells are the cultured tissue itself, or are a cell line whose parent cells are the tissue itself.

Preferred methods for determining the level of or a change in the level of a morphogen in a cultured cell include using an antibody specific for the morphogen, e.g., in an immunoassay such as an ELISA or radioimmunoassay; and determining the level of nucleic acid, most particularly mRNA, encoding the morphogen using a nucleic acid probe that hybridizes under stringent conditions with the morphogen RNA, such as in an RNA dot blot analysis. Where a change in the presence and/or concentration of morphogen is being determined, it will be necessary to measure and compare the levels of morphogen in the presence and absence of the candidate compound. The nucleic acid probe may be a nucleotide sequence encoding the morphogen or a fragment large enough to hybridize specifically only to RNA encoding a specific morphogen under stringent conditions. As used herein, "stringent conditions" are defined as conditions in which non-specific hybrids will be eluted but at which specific hybrids will be maintained, i.e., incubation at 0.1X SSC (15mM NaCl, 5mM Na citrate) at 50°C for 15 minutes.

Examples of morphogens whose levels may be determined according to the invention include OP-1, OP-2, GDF-1, Vgr-1, DPP, 60A CBMP2A, CBMP2B, BMP 2, 3, 4, 5, 6, or Vgl. Thus, if an immunoassay is used to indicate the presence and/or concentration of a morphogen, an antibody specific for one of these morphogens only, and which will not detect the presence of other morphogens, will be used. Similarly, if nucleic acid hybridization is used to indicate the level of RNA encoding the morphogen, a nucleotide probe specific for one of these morphogens only will be used under hybridization conditions such that the probe should not be capable of hybridizing with RNA encoding a different morphogen. A morphogen includes an active C-terminal core region, which includes at least six cysteine residues, and a region N-terminal to the C-terminal region that is relatively non-homologous to the equivalent N-terminal regions of other morphogens. In addition, the 3' noncoding region of the mRNA is unique to each morphogen. Thus, a nucleic acid probe encoding all or a portion of the sequences N-terminal to the C-terminal core region of a morphogen, or encoding all or a portion of the sequences C-terminal to or 3' to the core region of a morphogen may be used as a probe which detects mRNA encoding that morphogen only.

"Morphogenic proteins" or "morphogens", as used herein, include naturally-occurring osteogenic proteins capable of inducing the full developmental cascade of bone formation, as well as polypeptide chains not normally associated with bone or bone formation, but sharing substantial sequence homology with osteogenic proteins. Such proteins, as well as DNA sequences encoding them, have been isolated and characterized for a number of different species. See. for example, U.S. Patent No. 4,968,590 and U.S. Patent Number. 5,011,691, WO91/05802 and WO92/07073; Sampath et al., (1990) J. Biol. Chem. 265:13198-13205; Ozkaynak et al. (1990) EMBO J. 9:2085-2093; and Lee, Proc. Nat. Aca. Sci. 88:42504254 (1991). Many of these proteins subsequently were discovered to have utility beyond bone morphogenesis. The mature forms of morphogens share substantial amino acid sequence homology, especially in the C-terminal core regions of the proteins. In particular, most of the proteins share a seven-cysteine skeleton in this region, in addition to other apparently required amino acids. Table II, infra, shows the amino acid sequence homologies for nine morphogens over the carboxy terminal 102 amino acids.

Among the morphogens useful in this invention are proteins originally identified as osteogenic proteins, such as the OP-1, OP-2 and CBMP2 proteins, as well as amino acid sequence-related proteins such as DPP (from Drosophila), Vgl (from Xenopus), Vgr-1 (from mouse, see U.S. 5,011,691 to Oppermann et al.), GDF-1 (from mouse, see Lee (1991) PNAS 88:4250-4254), all of which are presented in Table II and Seq. ID Nos.5-14), and the recently identified 60A protein (from Drosophila, Seq. ID No. 24, see Wharton et al. (1991) PNAS 88:9214-9218.) The members of this family, which include members of the TGF-β super-family of proteins, share substantial amino acid sequence homology in their C-terminal regions. The proteins are translated as a precursor, having an N-terminal signal peptide sequence, typically less than about 30 residues, followed by a "pro" domain that is cleaved to yield the mature sequence. The signal peptide is cleaved rapidly upon translation, at a cleavage site that can be predicted in a given sequence using the method of Von Heijne ((1986) Nucleic Acids Research 14:4683-4691.) Table I, below, describes the various morphogens identified to date, including their nomenclature as used herein, their Seq. ID references, and publication sources for the amino acid sequences for the full length proteins not included in the Seq. Listing. The disclosure of these publications is incorporated herein by reference.

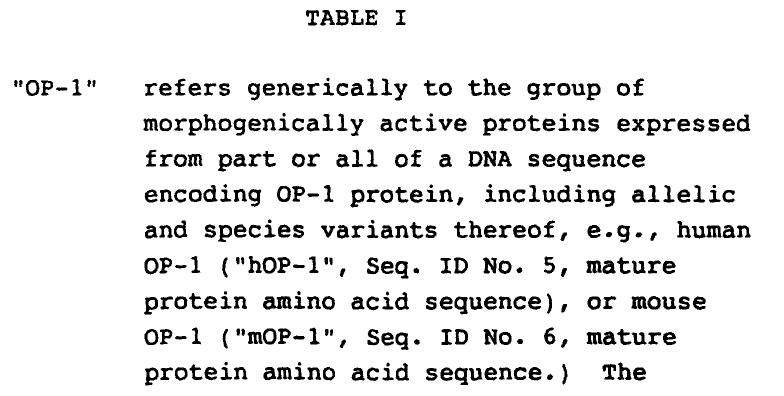

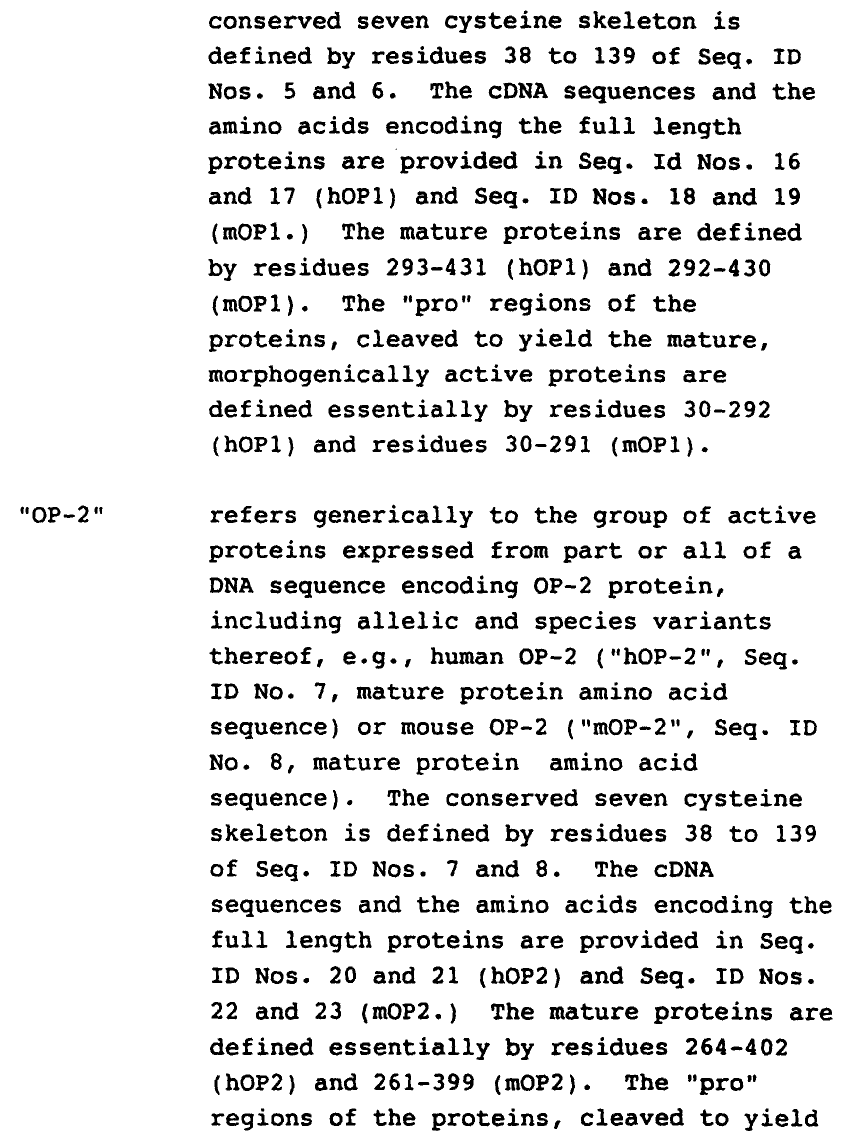

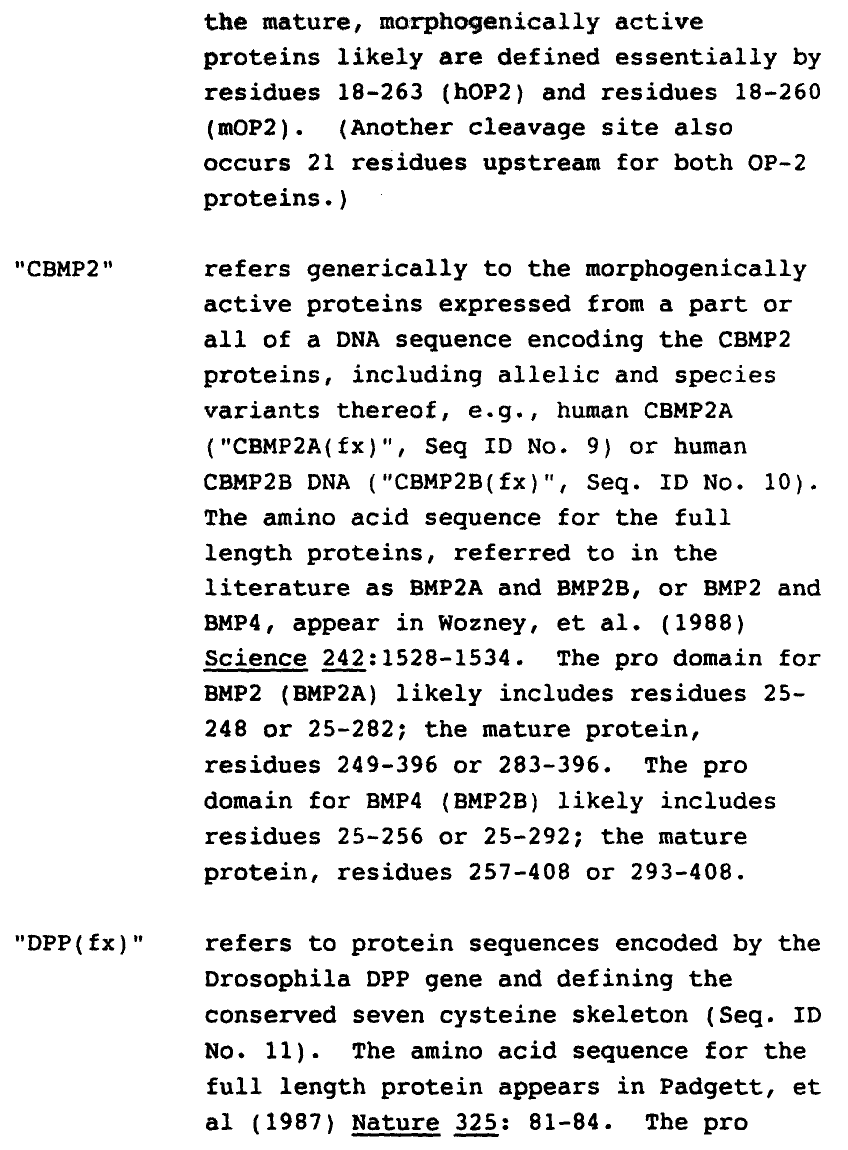

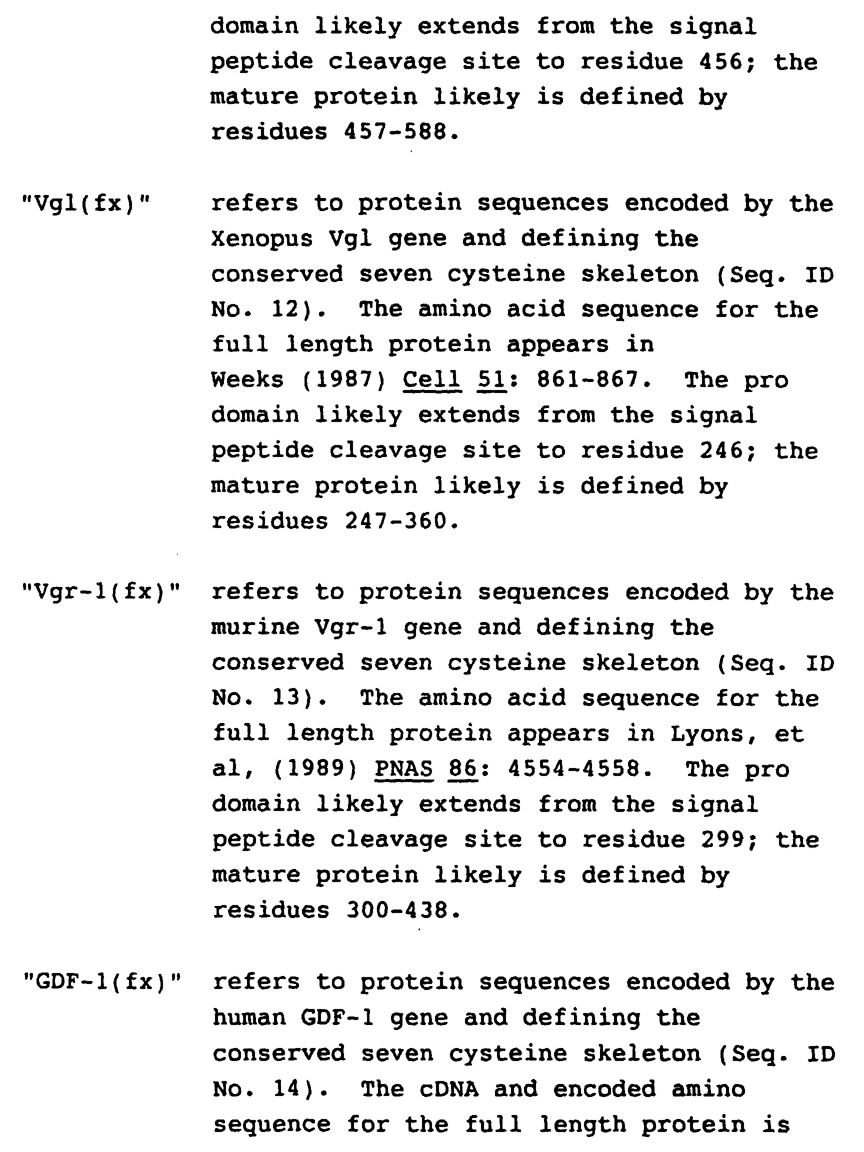

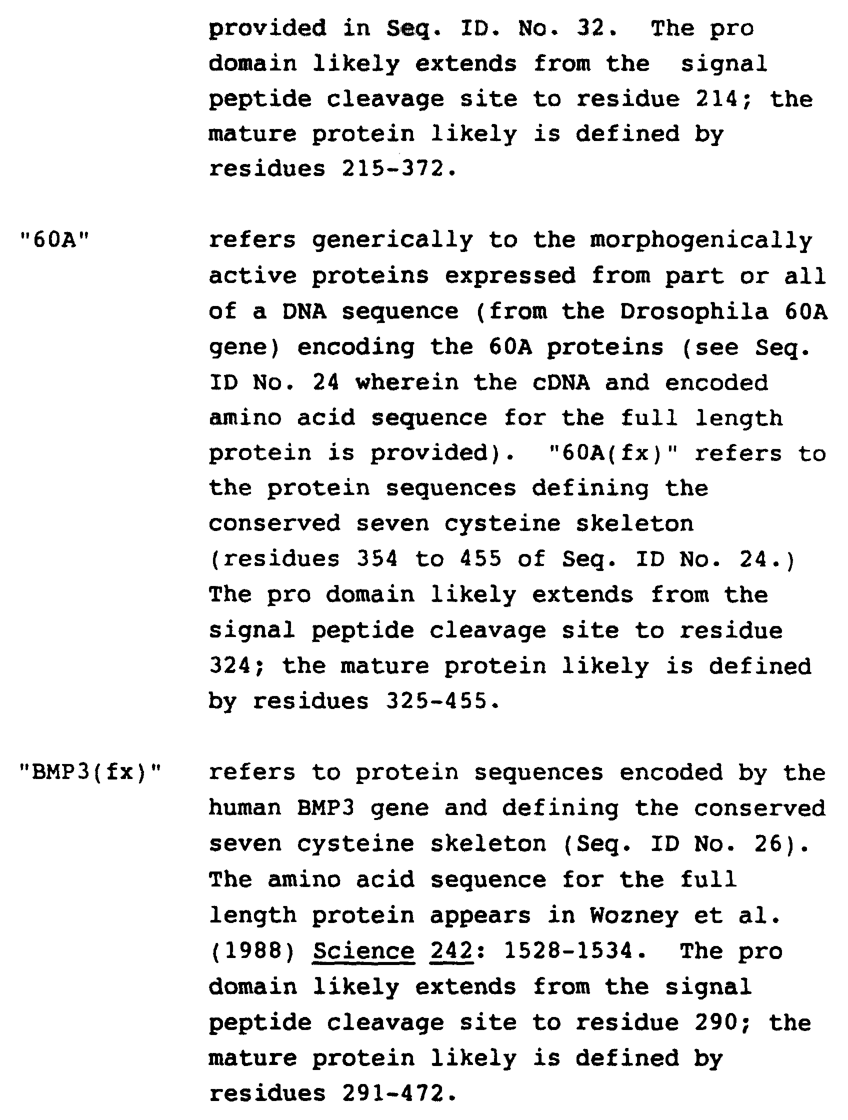

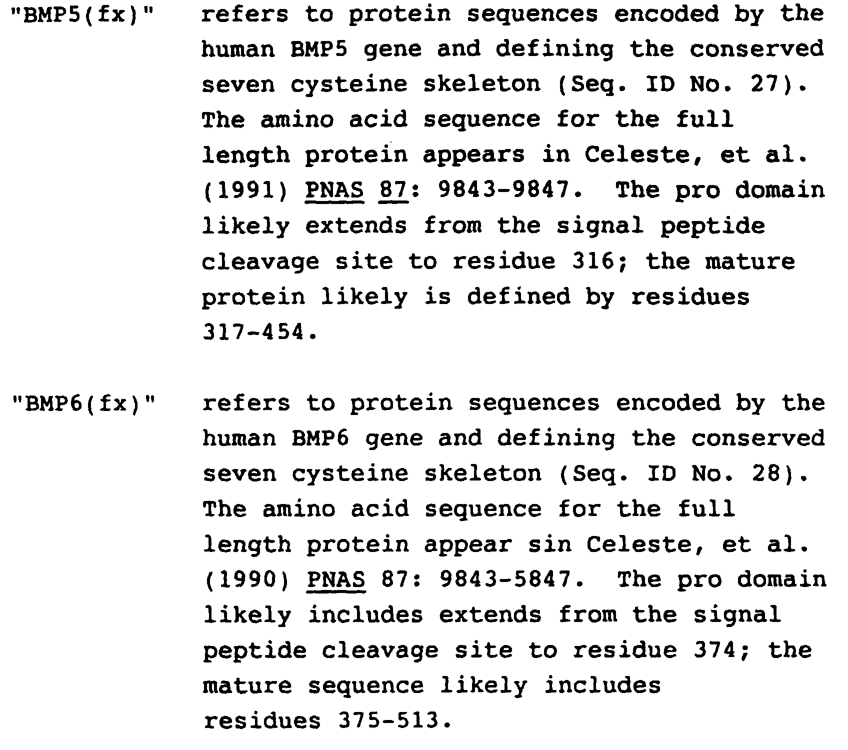

The OP-2 proteins have an additional cysteine residue in this region (e.g., see residue 41 of Seq. ID Nos. 7 and 8), in addition to the conserved cysteine skeleton in common with the other proteins in this family. The GDF-1 protein has a four amino acid insert within the conserved skeleton (residues 44-47 of Seq. ID No. 14) but this insert likely does not interfere with the relationship of the cysteines in the folded structure. In addition, the CBMP2 proteins are missing one amino acid residue within the cysteine skeleton.

The morphogens are inactive when reduced, but are active as oxidized homodimers and when oxidized in combination with other morphogens of this invention. Thus, as defined herein, a morphogen is a dimeric protein comprising a pair of polypeptide chains, wherein each polypeptide chain comprises at least the C-terminal six cysteine skeleton defined by residues 43-139 of Seq. ID No. 5, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra- and inter-chain disulfide bonds such that the protein is capable of acting as a morphogen as defined herein. Specifically, the morphogens generally are capable of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells. In addition, it is also anticipated that these morphogens are capable of inducing redifferentiation of committed cells under appropriate environmental conditions.

Morphogens useful in this invention comprise one of two species of generic amino acid sequences: Generic Sequence 1 (Seq. ID No. 1) or Generic Sequence 2 (Seq. ID No. 2); where each Xaa indicates one of the 20 naturally-occurring L-isomer, α-amino acids or a derivative thereof. Generic Sequence 1 comprises the conserved six cysteine skeleton and Generic Sequence 2 comprises the conserved six cysteine skeleton plus the additional cysteine identified in OP-2 (see residue 36, Seq. ID No. 2). In another preferred aspect, these sequences further comprise the following additional sequence at their N-terminus:

Cys Xaa Xaa Xaa Xaa (Seq. ID No. 15)
1 5

Preferred amino acid sequences within the foregoing generic sequences include: Generic Sequence 3 (Seq. ID No. 3), Generic Sequence 4 (Seq. ID No. 4), Generic Sequence 5 (Seq. ID No. 30) and Generic Sequence 6 (Seq. ID No. 31), listed below. These Generic Sequences accommodate the homologies shared among the various preferred members of this morphogen family identified in Table II, as well as the amino acid sequence variation among them. Specifically, Generic Sequences 3 and 4 are composite amino acid sequences of the following proteins presented in Table II and identified in Seq. ID Nos. 5-14: human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14.) The generic sequences include both the amino acid identity shared by the sequences in Table II, as well as alternative residues for the variable positions within the sequence. Note that these generic sequences allow for an additional cysteine at position 41 or 46 in Generic Sequences 3 or 4, respectively, providing an appropriate cysteine skeleton where inter- or intramolecular disulfide bonds can form, and contain certain critical amino acids which influence the tertiary structure of the proteins.

### Generic Sequence 3

Leu Tyr Val Xaa Phe
1               5
Xaa Xaa Xaa Gly Trp Xaa Xaa Trp Xaa
                10
Xaa Ala Pro Xaa Gly Xaa Xaa Ala
15                  20
Xaa Tyr Cys Xaa Gly Xaa Cys Xaa
        25                  30
Xaa Pro Xaa Xaa Xaa Xaa Xaa
                35

Xaa Xaa Xaa Asn His Ala Xaa Xaa
        40                  45
Xaa Xaa Leu Xaa Xaa Xaa Xaa Xaa
                50
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
70                  75
Xaa Xaa Xaa Xaa Val Xaa Leu Xaa
            80
Xaa Xaa Xaa Xaa Met Xaa Val Xaa
85                  90
Xaa Cys Gly Cys Xaa
        95

wherein each Xaa is independently selected from a group of one or more specified amino acids defined as follows: "Res." means "residue" and Xaa at res.4 = (Ser, Asp or Glu); Xaa at res.6 = (Arg, Gln, Ser or Lys); Xaa at res.7 = (Asp or Glu); Xaa at res.8 = (Leu or Val); Xaa at res.11 = (Gln, Leu, Asp, His or Asn); Xaa at res.12 = (Asp, Arg or Asn); Xaa at res.14 = (Ile or Val); Xaa at res.15 = (Ile or Val); Xaa at res.18 = (Glu, Gln, Leu, Lys, Pro or Arg); Xaa at res.20 = (Tyr or Phe); Xaa at res.21 = (Ala, Ser, Asp, Met, His, Leu or Gln); Xaa at res.23 = (Tyr, Asn or Phe); Xaa at res.26 = (Glu, His, Tyr, Asp or Gln); Xaa at res.28 = (Glu, Lys, Asp or Gln); Xaa at res.30 = (Ala, Ser, Pro or Gln); Xaa at res.31 = (Phe, Leu or Tyr); Xaa at res.33 = (Leu or Val); Xaa at res.34 = (Asn, Asp, Ala or Thr); Xaa at res.35 = (Ser, Asp, Glu, Leu or Ala); Xaa at res.36 = (Tyr, Cys, His, Ser or Ile); Xaa at res.37 = (Met, Phe, Gly or Leu); Xaa at res.38 = (Asn or Ser); Xaa at res.39 = (Ala, Ser or Gly); Xaa at res.40 = (Thr, Leu or Ser); Xaa at res.44 = (Ile or Val); Xaa at res.45 = (Val or Leu); Xaa at res.46 = (Gln or Arg); Xaa at res.47 = (Thr, Ala or Ser); Xaa at res.49 = (Val or Met); Xaa at res.50 = (His or Asn); Xaa at res.51 = (Phe, Leu, Asn, Ser, Ala or Val); Xaa at res.52 = (Ile, Met, Asn, Ala or Val); Xaa at res.53 = (Asn, Lys, Ala or Glu); Xaa at res.54 = (Pro or Ser); Xaa at res.55 = (Glu, Asp, Asn, or Gly); Xaa at res.56 = (Thr, Ala, Val, Lys, Asp, Tyr, Ser or Ala); Xaa at res.57 = (Val, Ala or Ile); Xaa at res.58 = (Pro or Asp); Xaa at res.59 = (Lys or Leu); Xaa at res.60 = (Pro or Ala); Xaa at res.63 = (Ala or Val); Xaa at res.65 = (Thr or Ala); Xaa at res.66 = (Gln, Lys, Arg or Glu); Xaa at res.67 = (Leu, Met or Val); Xaa at res.68 = (Asn, Ser or Asp); Xaa at res.69 = (Ala, Pro or Ser); Xaa at res.70 = (Ile, Thr or Val); Xaa at res.71 = (Ser or Ala); Xaa at res.72 = (Val or Met); Xaa at res.74 = (Tyr or Phe); Xaa at res.75 = (Phe, Tyr or Leu); Xaa at res.76 = (Asp or Asn); Xaa at res.77 = (Asp, Glu, Asn or Ser); Xaa at res.78 = (Ser, Gln, Asn or Tyr); Xaa at res.79 = (Ser, Asn, Asp or Glu); Xaa at res.80 = (Asn, Thr or Lys); Xaa at res.82 = (Ile or Val); Xaa at res.84 = (Lys or Arg); Xaa at res.85 = (Lys, Asn, Gln or His); Xaa at res.86 = (Tyr or His); Xaa at res.87 = (Arg, Gln or Glu); Xaa at res.88 = (Asn, Glu or Asp); Xaa at res.90 = (Val, Thr or Ala); Xaa at res.92 = (Arg, Lys, Val, Asp or Glu); Xaa at res.93 = (Ala, Gly or Glu); and Xaa at res.97 = (His or Arg);

### Generic Sequence 4

Cys Xaa Xaa Xaa Xaa Leu Tyr Val Xaa Phe
1               5                   10
Xaa Xaa Xaa Gly Trp Xaa Xaa Trp Xaa
                15
Xaa Ala Pro Xaa Gly Xaa Xaa Ala
20                  25
Xaa Tyr Cys Xaa Gly Xaa Cys Xaa
        30                  35
Xaa Pro Xaa Xaa Xaa Xaa Xaa
                40
Xaa Xaa Xaa Asn His Ala Xaa Xaa
        45                  50
Xaa Xaa Leu Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70
Xaa Xaa Xaa Leu Xaa Xaa Xaa
75                  80
Xaa Xaa Xaa Xaa Val Xaa Leu Xaa
            85
Xaa Xaa Xaa Xaa Met Xaa Val Xaa
90                  95

Xaa Cys Gly Cys Xaa
        100

wherein each Xaa is independently selected from a group of one or more specified amino acids as defined by the following: "Res." means "residue" and Xaa at res.2 = (Lys or Arg); Xaa at res.3 = (Lys or Arg); Xaa at res.4 = (His or Arg); Xaa at res.5 = (Glu, Ser, His, Gly, Arg or Pro); Xaa at res.9 = (Ser, Asp or Glu); Xaa at res.11 = (Arg, Gln, Ser or Lys); Xaa at res.12 = (Asp or Glu); Xaa at res.13 = (Leu or Val); Xaa at res.16 = (Gln, Leu, Asp, His or Asn); Xaa at res.17 = (Asp, Arg, or Asn); Xaa at res.19 = (Ile or Val); Xaa at res.20 = (Ile or Val); Xaa at res.23 = (Glu, Gln, Leu, Lys, Pro or Arg); Xaa at res.25 = (Tyr or Phe); Xaa at res.26 = (Ala, Ser, Asp, Met, His, Leu, or Gln); Xaa at res.28 = (Tyr, Asn or Phe); Xaa at res.31 = (Glu, His, Tyr, Asp or Gln); Xaa at res.33 = Glu, Lys, Asp or Gln); Xaa at res.35 = (Ala, Ser or Pro); Xaa at res.36 = (Phe, Leu or Tyr); Xaa at res.38 = (Leu or Val); Xaa at res.39 = (Asn, Asp, Ala or Thr); Xaa at res.40 = (Ser, Asp, Glu, Leu or Ala); Xaa at res.41 = (Tyr, Cys, His, Ser or Ile); Xaa at res.42 = (Met, Phe, Gly or Leu); Xaa at res.44 = (Ala, Ser or Gly); Xaa at res.45 = (Thr, Leu or Ser); Xaa at res.49 = (Ile or Val); Xaa at res.50 = (Val or Leu); Xaa at res.51 = (Gln or Arg); Xaa at res.52 = (Thr, Ala or Ser); Xaa at res.54 = (Val or Met); Xaa at res.55 = (His or Asn); Xaa at res.56 = (Phe, Leu, Asn, Ser, Ala or Val); Xaa at res.57 = (Ile, Met, Asn, Ala or Val); Xaa at res.58 = (Asn, Lys, Ala or Glu); Xaa at res.59 = (Pro or Ser); Xaa at res.60 = (Glu, Asp, or Gly); Xaa at res.61 = (Thr, Ala, Val, Lys, Asp, Tyr, Ser or Ala); Xaa at res.62 = (Val, Ala or Ile); Xaa at res.63 = (Pro or Asp); Xaa at res.64 = (Lys or Leu); Xaa at res.65 = (Pro or Ala); Xaa at res.68 = (Ala or Val); Xaa at res.70 = (Thr or Ala); Xaa at res.71 = (Gln, Lys, Arg or Glu); Xaa at res.72 = (Leu, Met or Val); Xaa at res.73 = (Asn, Ser or Asp); Xaa at res.74 = (Ala, Pro or Ser); Xaa at res.75 = (Ile, Thr or Val); Xaa at res.76 = (Ser or Ala); Xaa at res.77 = (Val or Met); Xaa at res.79 = (Tyr or Phe); Xaa at res.80 = (Phe, Tyr or Leu); Xaa at res.81 = (Asp or Asn); Xaa at res.82 = (Asp, Glu, Asn or Ser); Xaa at res.83 = (Ser, Gln, Asn or Tyr); Xaa at res.84 = (Ser, Asn, Asp or Glu); Xaa at res.85 = (Asn, Thr or Lys); Xaa at res.87 = (Ile or Val); Xaa at res.89 = (Lys or Arg); Xaa at res.90 = (Lys, Asn, Gln or His); Xaa at res.91 = (Tyr or His); Xaa at res.92 = (Arg, Gln or Glu); Xaa at res.93 = (Asn, Glu or Asp); Xaa at res.95 = (Val, Thr or Ala); Xaa at res.97 = (Arg, Lys, Val, Asp or Glu); Xaa at res.98 = (Ala, Gly or Glu); and Xaa at res.102 = (His or Arg).

Similarly, Generic Sequence 5 (Seq. ID No. 30) and Generic Sequence 6 (Seq. ID No. 31) accommodate the homologies shared among all the morphogen protein family members identified in Table II. Specifically, Generic Sequences 5 and 6 are composite amino acid sequences of human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14), human BMP3 (Seq. ID No. 26), human BMP5 (Seq. ID No. 27), human BMP6 (Seq. ID No. 28) and 60(A) (from Drosophila, Seq. ID Nos. 24-25). The generic sequences include both the amino acid identity shared by these sequences in the C-terminal domain, defined by the six and seven cysteine skeletons (Generic Sequences 5 and 6, respectively), as well as alternative residues for the variable positions within the sequence. As for Generic Sequences 3 and 4, Generic Sequences 5 and 6 allow for an additional cysteine at position 41 (Generic Sequence 5) or position 46 (Generic Sequence 6), providing an appropriate cysteine skeleton where inter- or intramolecular disulfide bonds can form, and containing certain critical amino acids which influence the tertiary structure of the proteins.

### Generic Sequence 5

Leu Xaa Xaa Xaa Phe
1               5
Xaa Xaa Xaa Gly Trp Xaa Xaa Trp Xaa
                10
Xaa Xaa Pro Xaa Xaa Xaa Xaa Ala
15                  20
Xaa Tyr Cys Xaa Gly Xaa Cys Xaa
        25                  30
Xaa Pro Xaa Xaa Xaa Xaa Xaa
                35

Xaa Xaa Xaa Asn His Ala Xaa Xaa
        40                  45
Xaa Xaa Xaa Xaa Xaa Xaa Xaa Xaa
                    50
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
70                  75
Xaa Xaa Xaa Xaa Val Xaa Leu Xaa
            80
Xaa Xaa Xaa Xaa Met Xaa Val Xaa
85                  90
Xaa Cys Xaa Cys Xaa
        95

wherein each Xaa is independently selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp, Glu or Lys); Xaa at res.8 = (Leu, Val or Ile); Xaa at res.11 = (Gln, Leu, Asp, His, Asn or Ser); Xaa at res.12 = (Asp, Arg, Asn or Glu); Xaa at res.14 = (Ile or Val); Xaa at res.15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln or Ser); Xaa at res.28 = (Glu, Lys, Asp, Gln or Ala); Xaa at res.30 = (Ala, Ser, Pro, Gln or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val or Leu); Xaa at res.53 = (Asn, Lys, Ala, Glu, Gly or Pbs); Xaa at res.54 = (Pro, Ser or Val); Xaa at res.55 = (Glu, Asp, Asn, Gly, Val or Lys); Xaa at res.56 = (Thr, Ala, Val, Lys, Asp, Tyr, Ser, Ala, Pro or His); Xaa at res.57 = (Val, Ala or Ile); Xaa at res.58 = (Pro or Asp); Xaa at res.59 = (Lys, Leu or Glu); Xaa at res.60 = (Pro or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Met or Ile); Xaa at res.74 = (Tyr or Phe); Xaa at res.75 = (Pbs, Tyr, Leu or His); Xaa at res.76 = (Asp, Asn or Leu); Xaa at res.77 = (Asp, Glu, Asn or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (Ile, Val or Asn); Xaa at res.84 = (Lys or Arg); Xaa at res.85 = (Lys, Asn, Gln, His or Val); Xaa at res.86 = (Tyr or His); Xaa at res.87 = (Arg, Gln, Glu or Pro); Xaa at res.88 = (Asn, Glu or Asp); Xaa at res.90 = (Val, Thr, Ala or Ile); Xaa at res.92 = (Arg, Lys, Val, Asp or Glu); Xaa at res.93 = (Ala, Gly, Glu or Ser); Xaa at res.95 = (Gly or Ala) and Xaa at res.97 = (His or Arg).

### Generic Sequence 6

Cys Xaa Xaa Xaa Xaa Leu Xaa Xaa Xaa Phe
1               5                   10
Xaa Xaa Xaa Gly Trp Xaa Xaa Trp Xaa
                15
Xaa Xaa Pro Xaa Xaa Xaa Xaa Ala
20                  25
Xaa Tyr Cys Xaa Gly Xaa Cys Xaa
        30                  35
Xaa Pro Xaa Xaa Xaa Xaa Xaa
                40
Xaa Xaa Xaa Asn His Ala Xaa Xaa
        45                  50
Xaa Xaa Xaa Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70

Xaa Xaa Xaa Leu Xaa Xaa Xaa
 75                  80
Xaa Xaa Xaa Xaa Val Xaa Leu Xaa
            85
Xaa Xaa Xaa Xaa Met Xaa Val Xaa
 90                  95
Xaa Cys Xaa Cys Xaa
        100

wherein each Xaa is independently selected from a group of one or more specified amino acids as defined by the following: "Res." means "residue" and Xaa at res.2 = (Lys, Arg, Ala or Gln); Xaa at res.3 = (Lys, Arg or Met); Xaa at res.4 = (His, Arg or Gln); Xaa at res.5 = (Glu, Ser, His, Gly, Arg, Pro, Thr, or Tyr); Xaa at res.7 = (Tyr or Lys); Xaa at res.8 = (Val or Ile); Xaa at res.9 = (Ser, Asp or Glu); Xaa at res.11 = (Arg, Gln, Ser, Lys or Ala); Xaa at res.12 = (Asp, Glu, or Lys); Xaa at res.13 = (Leu, Val or Ile); Xaa at res.16 = (Gln, Leu, Asp, His, Asn or Ser); Xaa at res.17 = (Asp, Arg, Asn or Glu); Xaa at res.19 = (Ile or Val); Xaa at res.20 = (Ile or Val); Xaa at res.21 = (Ala or Ser); Xaa at res.23 = (Glu, Gln, Leu, Lys, Pro or Arg); Xaa at res.24 = (Gly or Ser); Xaa at res.25 = (Tyr or Phe); Xaa at res.26 = (Ala, Ser, Asp, Met, His, Gln, Leu, or Gly); Xaa at res.28 = (Tyr, Asn or Phe); Xaa at res.31 = (Glu, His, Tyr, Asp, Gln or Ser); Xaa at res.33 = Glu, Lys, Asp, Gln or Ala); Xaa at res.35 = (Ala, Ser, Pro, Gln or Asn); Xaa at res.36 = (Phe, Leu or Tyr); Xaa at res.38 = (Leu, Val or Met); Xaa at res.39 = (Asn, Asp, Ala, Thr or Pro); Xaa at res.40 = (Ser, Asp, Glu, Leu, Ala or Lys); Xaa at res.41 = (Tyr, Cys, His, Ser or Ile); Xaa at res.42 = (Met, Phe, Gly or Leu); Xaa at res.43 = (Asn, Ser or Lys); Xaa at res.44 = (Ala, Ser, Gly or Pro); Xaa at res.45 = (Thr, Leu or Ser); Xaa at res.49 = (Ile, Val or Thr); Xaa at res.50 = (Val, Leu or Ile); Xaa at res.51 = (Gln or Arg); Xaa at res.52 = (Thr, Ala or Ser); Xaa at res.53 = (Leu or Ile); Xaa at res.54 = (Val or Met); Xaa at res.55 = (His, Asn or Arg); Xaa at res.56 = (Phe, Leu, Asn, Ser, Ala or Val); Xaa at res.57 = (Ile, Met, Asn, Ala, Val or Leu); Xaa at res.58 = (Asn, Lys, Ala, Glu, Gly or Phe); Xaa at res.59 = (Pro, Ser or Val); Xaa at res.60 = (Glu, Asp, Gly, Val or Lys); Xaa at res.61 = (Thr, Ala, Val, Lys, Asp, Tyr, Ser, Ala, Pro or His); Xaa at res.62 = (Val, Ala or Ile); Xaa at res.63 = (Pro or Asp); Xaa at res.64 = (Lys, Leu or Glu); Xaa at res.65 = (Pro or Ala); Xaa at res.68 = (Ala or Val); Xaa at res.70 = (Thr, Ala or Glu); Xaa at res.71 = (Gln, Lys, Arg or Glu); Xaa at res.72 = (Leu, Met or Val); Xaa at res.73 = (Asn, Ser, Asp or Gly); Xaa at res.74 = (Ala, Pro or Ser); Xaa at res.75 = (Ile, Thr, Val or Leu); Xaa at res.76 = (Ser, Ala or Pro); Xaa at res.77 = (Val, Met or Ile); Xaa at res.79 = (Tyr or Phe); Xaa at res.80 = (Phe, Tyr, Leu or His); Xaa at res.81 = (Asp, Asn or Leu); Xaa at res.82 = (Asp, Glu, Asn or Ser); Xaa at res.83 = (Ser, Gln, Asn, Tyr or Asp); Xaa at res.84 = (Ser, Asn, Asp, Glu or Lys); Xaa at res.85 = (Asn, Thr or Lys); Xaa at res.87 = (Ile, Val or Asn); Xaa at res.89 = (Lys or Arg); Xaa at res.90 = (Lys, Asn, Gln, His or Val); Xaa at res.91 = (Tyr or His); Xaa at res.92 = (Arg, Gln, Glu or Pro); Xaa at res.93 = (Asn, Glu or Asp); Xaa at res.95 = (Val, Thr, Ala or Ile); Xaa at res.97 = (Arg, Lys, Val, Asp or Glu); Xaa at res.98 = (Ala, Gly, Glu or Ser); Xaa at res.100 = (Gly or Ala); and Xaa at res.102 = (His or Arg).

Particularly useful sequences for use as morphogens in this invention include the C-terminal domains, e.g., the C-terminal 96-102 amino acid residues of Vgl, Vgr-1, DPP, OP-1, OP-2, CBMP-2A, CBMP-2B, GDF-1 (see Table II, below, and Seq. ID Nos. 5-14), as well as proteins comprising the C-terminal domains of 60A, BMP3, BMP5 and BMP6 (see Seq. ID Nos. 24-28), all of which include at least the conserved six or seven cysteine skeleton. In addition, biosynthetic constructs designed from the generic sequences, such as COP-1, 3-5, 7, 16, disclosed in U.S. Pat. No. 5,011,691, also are useful. Other sequences include the inhibins/activin proteins (see, for example, U.S. Pat. Nos. 4,968,590 and 5,011,691). Accordingly, other useful sequences are those sharing at least 70% amino acid sequence homology or "similarity", and preferably 80% homology or similarity with any of the sequences above. These are anticipated to include allelic and species variants and mutants, and biosynthetic muteins, as well as novel members of this morphogenic family of proteins. Particularly envisioned in the family of related proteins are those proteins exhibiting morphogenic activity and wherein the amino acid changes from the preferred sequences include conservative changes, e.g., those as defined by Dayoff et al., Atlas of Protein Sequence and Structure; vol. 5, Suppl. 3, pp. 345-362, (M.O. Dayoff, ed., Nat'l BioMed. Research Fdn., Washington, D.C. 1979). As used herein, potentially useful sequences are aligned with a known morphogen sequence using the method of Needleman et al. ((1970) J.Mol.Biol. 48:443-453) and identities calculated by the Align program (DNAstar, Inc.). "Homology" or "similarity" as used herein includes allowed conservative changes as defined by Dayoff et al.

Morphogen sequences which are detectable according to the methods of the invention include but are not limited to those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP-1 and OP-2 proteins, including the Drosophila 60A protein. Accordingly, morphogens which are detectable according to the invention include active proteins comprising species of polypeptide chains having the generic amino acid sequence herein referred to as "OPX", which accommodates the homologies between the various identified species of OP1 and OP2 (Seq. ID No. 29).

The morphogens detectable in the methods of this invention include proteins comprising any of the polypeptide chains described above, whether isolated from naturally-occurring sources, or produced by recombinant DNA or other synthetic techniques, and includes allelic and species variants of these proteins, naturally-occurring or biosynthetic mutants thereof, chimeric variants containing a domain(s) or region(s) of one family member functionally arranged with another domain(s) or regions(s) of a second family member, as well as various truncated and fusion constructs. Deletion or insertion or addition mutants also are envisioned to be active, including those which may alter the conserved C-terminal cysteine skeleton, provided that the alteration does not functionally disrupt the relationship of these cysteines in the folded structure. Accordingly, such active forms are considered the equivalent of the specifically described constructs disclosed herein. The proteins may include forms having varying glycosylation patterns, varying N-termini, a family of related proteins having regions of amino acid sequence homology, and active truncated or mutated forms of native or biosynthetic proteins, produced by expression of recombinant DNA in host cells.

The morphogenic proteins can be expressed from intact or truncated cDNA or from synthetic DNAs in procaryotic or eucaryotic host cells, and purified, cleaved, refolded, and dimerized to form morphogenically active compositions. Currently preferred host cells include E. coli or mammalian cells, such as CHO, COS or BSC cells. A detailed description of the morphogens detectable according to the methods of this invention is disclosed in WO 92/01968.

The screening method of the invention provides a simple method of determining a change in the level of morphogenic protein as a result of exposure of cultured epithelial cells to one or more compound(s). The level of a morphogenic protein in a given cell culture, or a change in that level resulting from exposure to one or more compound(s) indicates that direct application of the compound modulates the level of the morphogen expressed by the cultured cells. If, for example, a compound upregulated the production of OP-1 by a kidney cell line, it would then be desirable to test systemic administration of this compound in an animal model to determine if it upregulated the production of OP-1 in vivo. If this compound did upregulate the endogenous circulating levels of OP-1, it would be consistent with administration of the compound systemically for the purpose of correcting bone metabolism diseases such as osteoporosis. The level of morphogen in the body may be a result of a wide range of physical conditions, e.g., tissue degeneration such as occurs in diseases including arthritis, emphysema, osteoporosis, kidney diseases, lung diseases, cardiomyopathy, and cirrhosis of the liver. The level of morphogens in the body may also occur as a result of the normal process of aging. A compound selected by the screening method of the invention as, for example, one which increases the level of morphogen in a tissue, may be consistent with the administration of the compound systemically or locally to a tissue for the purpose of preventing some form of tissue degeneration or for restoring the degenerated tissue to its normal healthy level.

Other advantages of the invention include determining the tissue or tissues of origin of a given morphogen in order to administer a compound aimed at modulating the systemic level of morphogen for treatment of a disease or condition in which the level of morphogen production has become altered.

### Brief Description of the Drawings

Fig. 1 shows the fragments of OP-1, used as probes in Northern hybridizations useful in the processes of the invention.
Fig. 2 shows results of Northern blot analysis of RNA using different OP-1-specific probes.
Fig. 3 shows results of Northern blot analysis of RNA from different cells types probed with an OP-1 probe.

### Detailed Description

The invention is based on the discovery of a family of structurally related morphogenic proteins (BMPs), also called osteogenic proteins (OPs), and more particularly that various of these proteins play an important role, not only in embryogenesis, but also in tissue and organ maintenance and repair in juvenile and adult mammals. Morphogenic proteins which have been identified include BMP 2, 3, 4, 5, 6, OP-1 and OP-2 (murine and human), Vgr-1, Vgl, DPP, GDF-1, CMBP-2A, CMBP-2B, 60A, and the inhibin/activin class of proteins. Other recombinant proteins include COP1, COP3, COP4, COP5, COP7, and COP16. While, as explained herein, the morphogen have significant homologies and similarities in structure, it is hypothesized that variants within the morphogenic protein genes may have specific roles in specific tissue involving, for example, stimulation of progenitor cell multiplication, tissue specific or tissue preferred phenotype maintenance, and/or stimulation or modulation of the rate of differentiation, growth or replication of tissue cells characterized by high turnover. The effect on the long-term physiology, maintenance and repair of particular tissues by particular species of the morphogens is currently unknown in any significant detail. However, methods useful in determining which particular tissues express which particular morphogen(s), and for finding changes which stimulate or depress morphogen expression in vivo, would enable discovery and development of strategies for therapeutic treatment of a large number of diseased states, and provide drugs designed to implement the strategy.

This invention provides such methods and, more specifically, two generic processes for obtaining data which ultimately will permit determination of structure/activity relationships of specific naturally occurring mammalian morphogens and drugs capable of modulating their production. For example, using the assay of the invention, it has been determined that OP-1, first found in bone and demonstrated to be osteoinductive, is synthesized primarily in kidney, bladder, and adrenal tissue. This surprising discovery, coupled with the observation that patients with kidney disease often express loss of bone mass, suggests that the bone loss in these patients may be due to pathologic depression of OP-1 synthesis in kidney, and suggests that administration of OP-1 systemically or stimulation of OP-1 expression and secretion by the kidney may arrest bone loss, or effect remineralization through increased bone formation (i.e., osteogenesis).

The assay of the invention involves screening candidate compounds for their ability to modulate the effective systemic or local concentration of a morphogen by incubating the compound with a cell culture that produces the morphogen, and assaying the culture for a parameter indicative of a change in the production level of the morphogen. Useful candidate compounds then may be tested for in vivo efficacy in a suitable animal model. These compounds then may be used in vivo to modulate effective morphogen concentrating in the disease treatment.

### 1. Morphogen Tissue Distribution

Morphogens are broadly distributed in developing and adult tissue. For example, DPP and 60A are expressed in both embryonic and developing Drosophila tissue. Vgl has been identified in Xenopus embryonic tissue. Vgr-1 transcripts have been identified in a variety of murine tissues, including embryonic and developing brain, lung, liver, kidney and calvaria (dermal bone) tissue. In addition, both CBMP2B and CBMP3 have been identified in lung tissue. Recently, Vgr-1 transcripts also have been identified in adult murine lung, kidney, heart, and brain tissue, with particularly high levels in the lung (see infra). GDF-1 has been identified in human adult cerebellum and in fetal brain tissue. In addition, recent Northern blot analyses indicate that OP-1 is encoded by multiple transcripts in different tissues. This potential alternative splicing is consistent with the hypothesis that the longer transcripts may encoded additional proteins (e.g., bicistronic mRNA) and each form may be tissue or developmentally related.

OP-1 and the CBMP2 proteins, both first identified as bone morphogens, have been identified in mouse and human placenta, hippocampus, calvaria and osteosarcoma tissue as determined by identification of OP-1 and CMBP2-specific sequences in cDNA libraries constructed from these tissues.
Additionally, the OP-1 protein is present in a variety of embryonic and developing tissues including kidney, liver, heart and brain as determined by Western blot analysis and immunolocalization (see infra). OP-1-specific transcripts also have been identified in both embryonic and developing tissues, most abundantly in developing kidney, bladder, adrenal and (see infra). OP-1 also has been identified as a mesoderm inducing factor present during embryogenesis. Moreover, OP-1 has been shown to be associated with satellite cells in the muscle and associated with potential pluripotential stem cells in bone marrow following damage to adult murine endochondral bone, indicating its morphogenic role in tissue repair and regeneration. In addition, a novel protein GDF-1 comprising a 7 cysteine skeleton, has been identified in neural tissue (Lee, 1991, Proc. Nat. Aca. Sci. 88: 4250-4254).

Knowledge of the tissue distribution of a given morphogen may be useful in choosing a cell type for screening according to the invention, or for targeting that cell type or tissue type for treatment. The proteins (or their mRNA transcripts) are readily identified in different tissues using standard methodologies and minor modifications thereof in tissues where expression may be low. For example, protein distribution may be determined using standard Western blot analysis or immunocytochemical techniques, and antibodies specific to the morphogen or morphogens of interest. Similarly, the distribution of morphogen transcripts may be determined using standard Northern hybridization protocols and a transcript-specific probe and hybridization conditions.

### 2. Useful Morphogens

As defined herein a protein is morphogenic if it is capable of inducing the developmental cascade of cellular and molecular events that culminate in the formation of new, organ-specific tissue and comprises at least the conserved C-terminal six cysteine skeleton or its functional equivalent (see supra). Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells. Details of how the morphogens detectable according to the methods of this invention first were identified, as well as a description on how to make, use and test them for morphogenic activity are disclosed in WO 92/01968.
As disclosed therein, the morphogens may be purified from naturally-sourced material or recombinantly produced from procaryotic or eucaryotic host cells, using the genetic sequences disclosed therein. Alternatively, novel morphogenic sequences may be identified following the procedures disclosed therein.

Particularly useful proteins include those which comprise the naturally derived sequences disclosed in Table II. Other useful sequences include biosynthetic constructs such as those disclosed in U.S. Pat. 5,011,691.
(e.g., COP-1, COP-3, COP-4, COP-5, COP-7, and COP-16).

Accordingly, the morphogens detectable according to the methods and compositions of this invention also may be described by morphogenically active proteins having amino acid sequences sharing 70% or, preferably, 80% homology (similarity) with any of the sequences described above, where "homology" is as defined herein above.

The morphogens detectable according to the method of this invention also can be described by any of the 6 generic sequences described herein (Generic Sequences 1, 2, 3, 4, 5 and 6). Generic sequences 1 and 2 also may, include, at their N-terminus, the sequence

Cys Xaa Xaa Xaa Xaa (Seq. ID No. 15)
1 5

Table II, set forth below, compares the amino acid sequences of the active regions of native proteins that have been identified as morphogens, including human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-23), CBMP2A (Seq. ID No. 9), CEMP2B (Seq. ID No. 10), BMP3 (Seq. ID No. 26), DPP (from Drosophila, Seq. ID No. 11), Vgl, (from Xenopus, Seq. ID No. 12), Vgr-1 (from mouse, Seq. ID No. 13), GDF-1 (from mouse, Seq. ID Nos. 14, 32 and 33), 60A protein (from Drosophila, Seq. ID Nos. 24 and 25), BMP5 (Seq. ID No. 27) and BMP6 (Seq. ID No. 28). The sequences are aligned essentially following the method of Needleman et al. (1970) J. Mol. Biol., 48:443-453, calculated using the Align Program (DNAstar, Inc.) In the table, three dots indicates that the amino acid in that position is the same as the amino acid in hOP-1. Three dashes indicates that no amino acid is present in that position, and are included for purposes of illustrating homologies. For example, amino acid residue 60 of CBMP-2A and CBMP-2B is "missing". Of course, both these amino acid sequences in this region comprise Asn-Ser (residues 58, 59), with CBMP-2A then comprising Lys and Ile, whereas CBMP-2B comprises Ser and Ile.

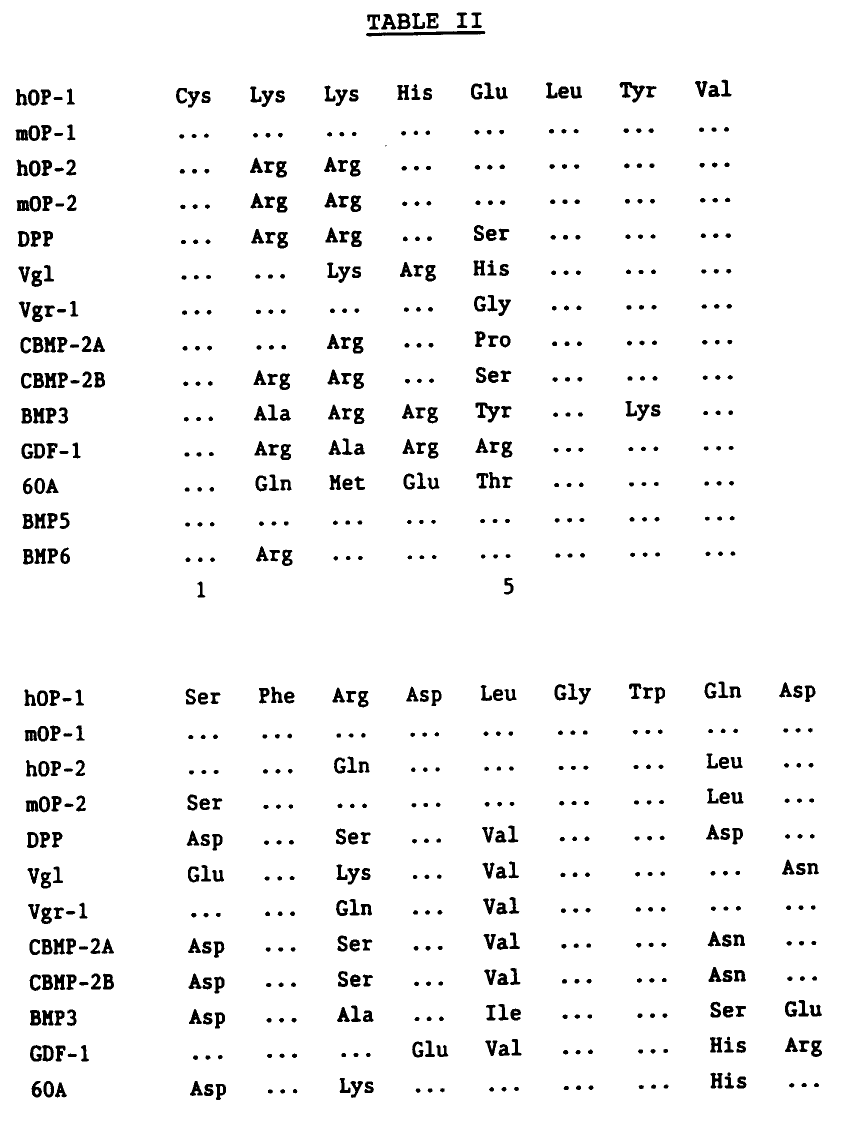

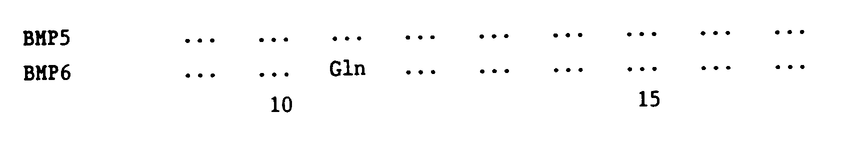

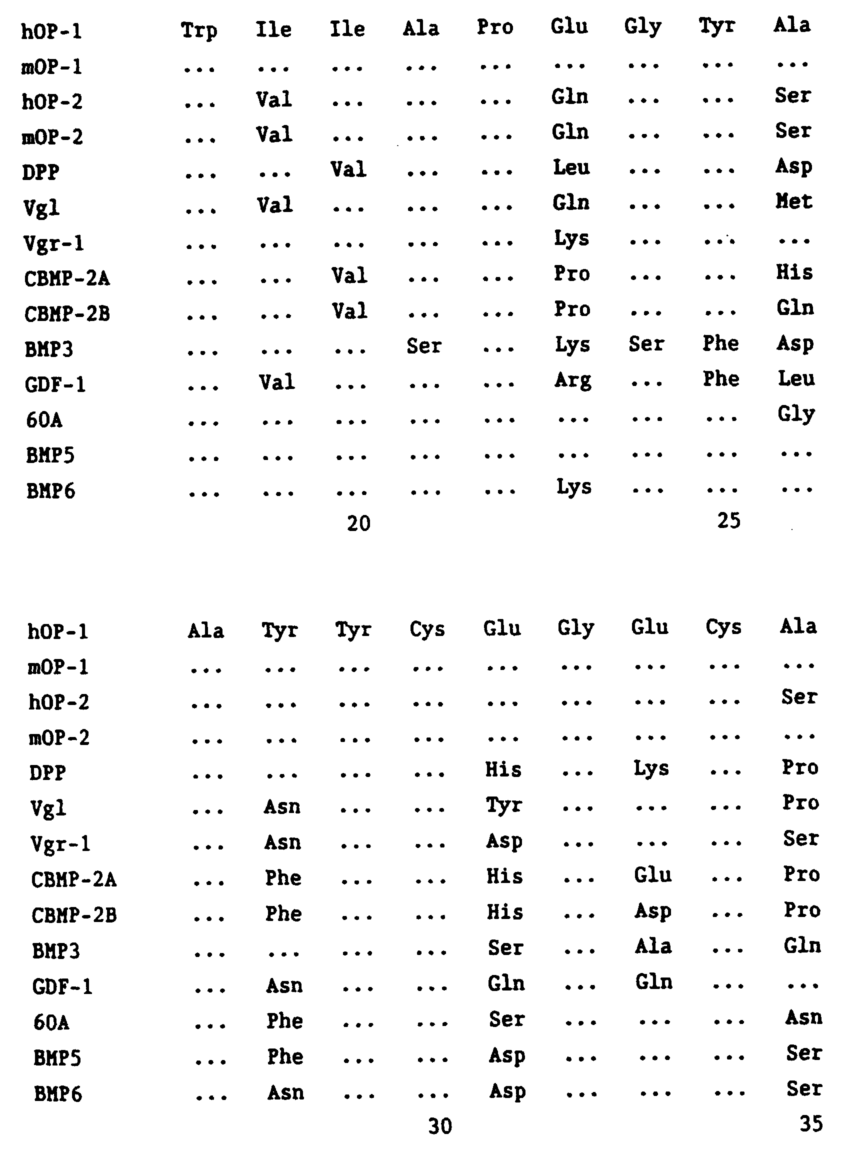

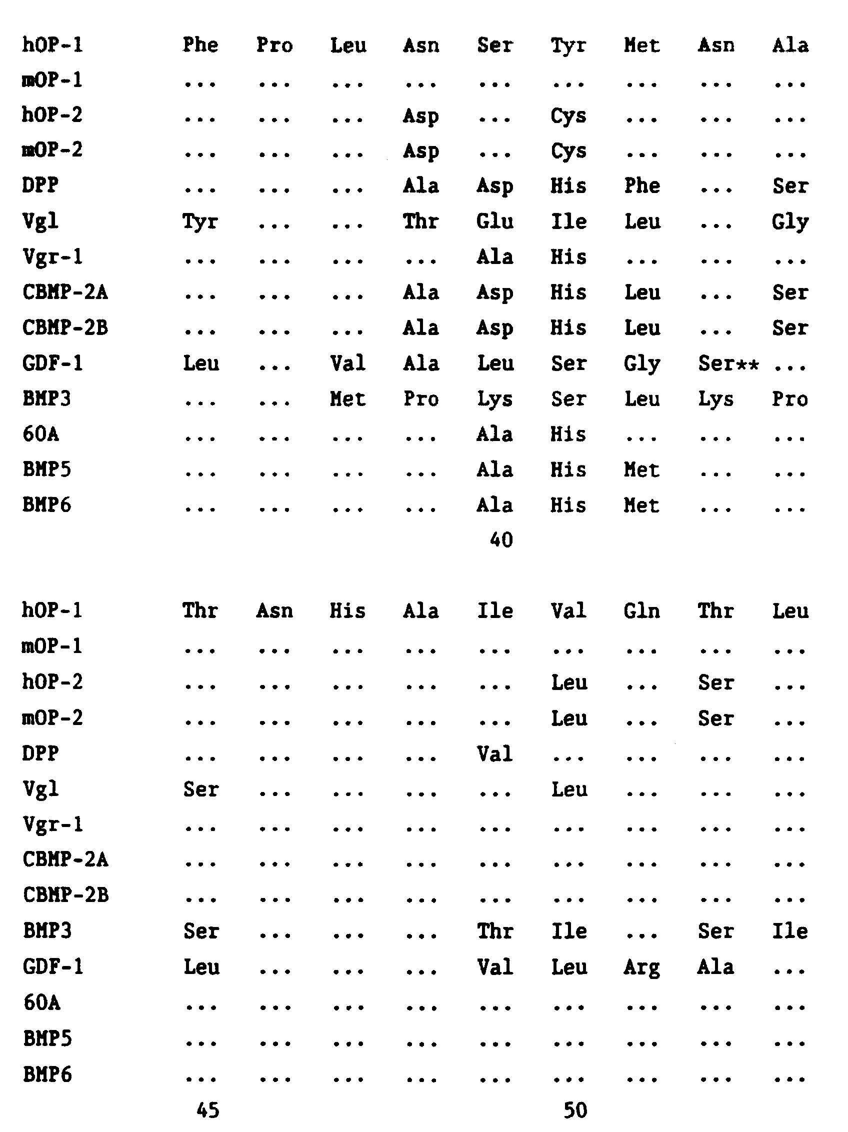

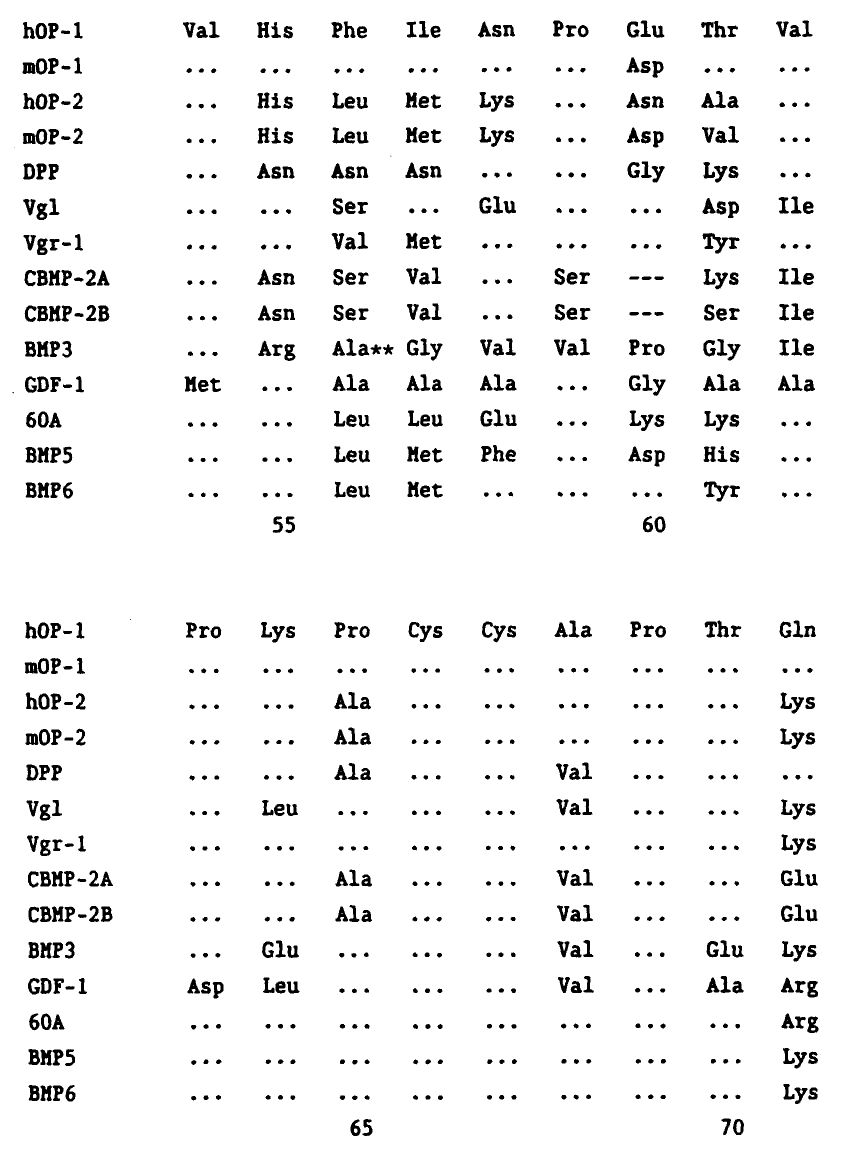

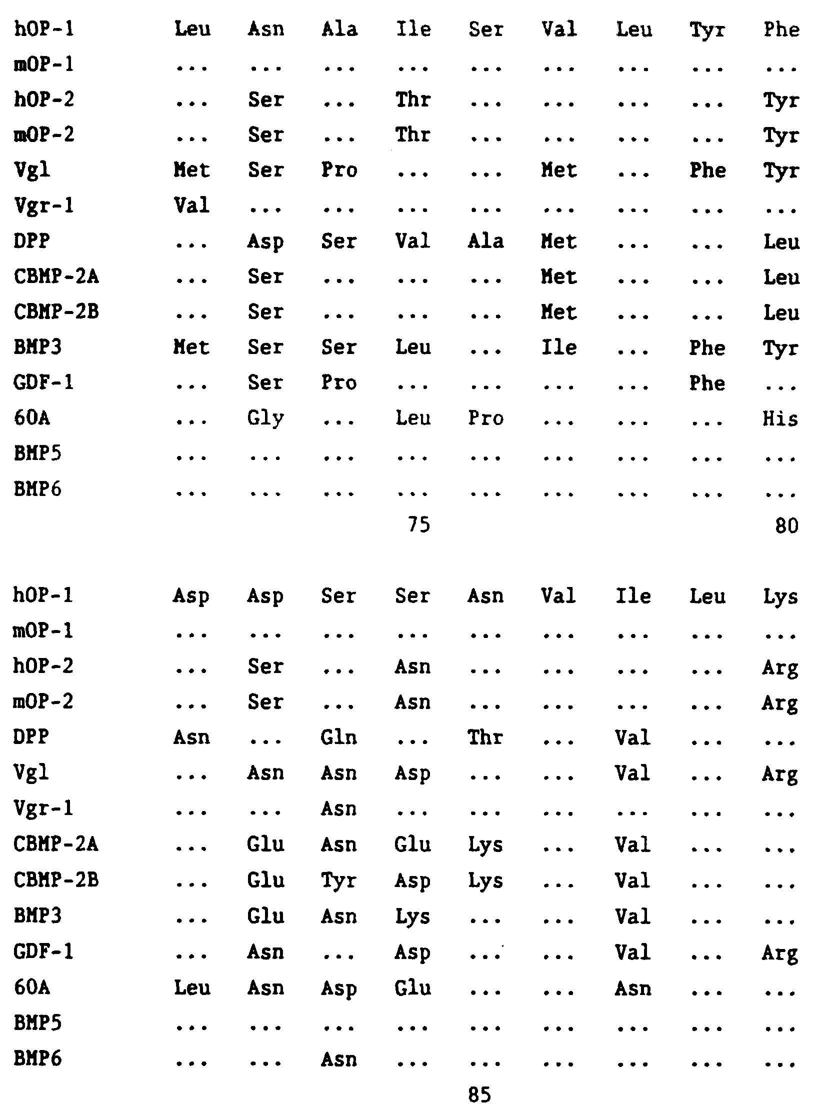

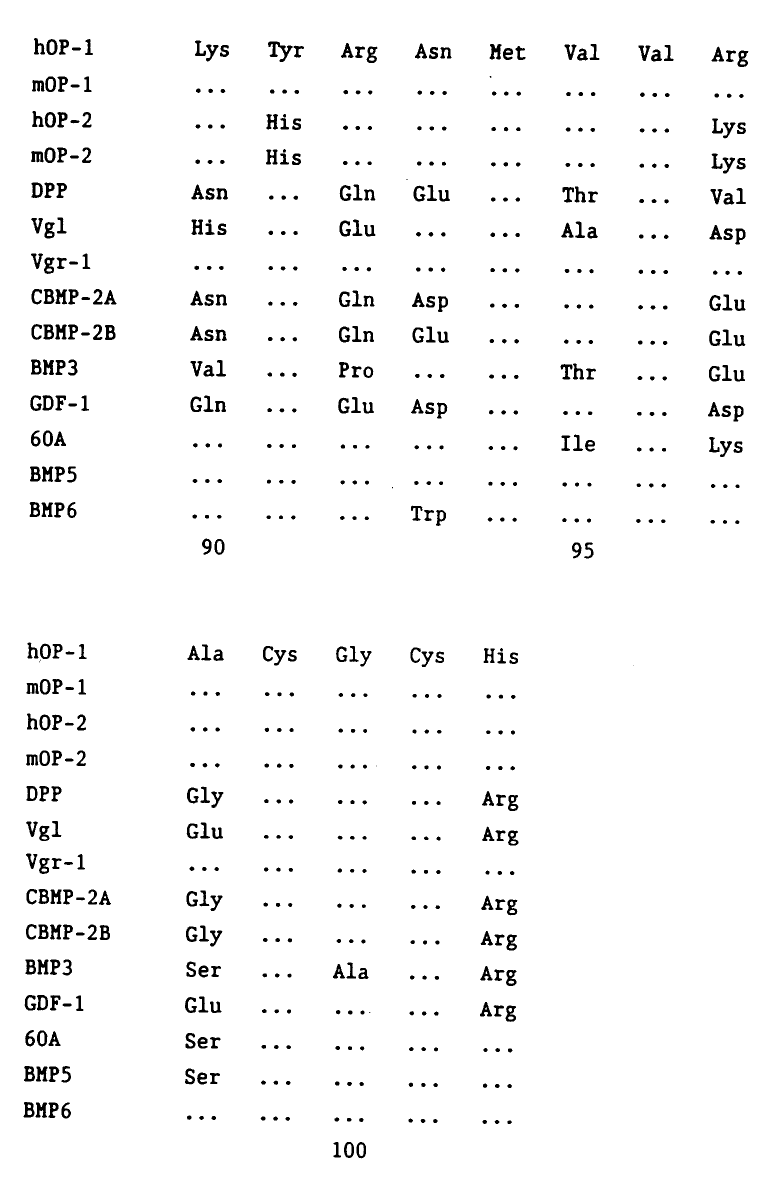

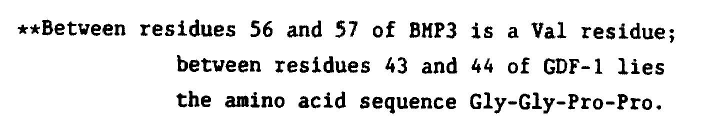

As is apparent from the foregoing amino acid sequence comparisons, significant amino acid changes can be made within the generic sequences while retaining the morphogenic activity. For example, while the GDF-1 protein sequence depicted in Table II shares only about 50% amino acid identity with the hOP1 sequence described therein, the GDF-1 sequence shares greater than 70% amino acid sequence homology (or "similarity") with the hOP1 sequence, where "homology" or "similarity" includes allowed conservative amino acid changes within the sequence as defined by Dayoff, et al., Atlas of Protein Sequence and Structure vol.5, supp.3, pp.345-362, (M.O. Dayoff, ed., Nat'l BioMed. Res. Fd'n, Washington D.C. 1979.)

The currently most preferred protein sequences detectable as morphogens in this invention include those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP-1 and OP-2 proteins, including the Drosophila 60A protein. Accordingly, in still another preferred aspect, the invention includes detection of morphogens comprising species of polypeptide chains having the generic amino acid sequence referred to herein as "OPX", which defines the seven cysteine skeleton and accommodates the identities between the various identified mouse and human OP1 and OP2 proteins. OPX is presented in Seq. ID No. 29. As described therein, each Xaa at a given position independently is selected from the residues occurring at the corresponding position in the C-terminal sequence of mouse or human OP1 or OP2 (see Seq. ID Nos. 5-8 and/or Seq. ID Nos. 16-23).

### 3. Tissue-Specific Expression of OP-1

Once a morphogen is identified in a tissue, its level may be determined either at the protein or nucleic acid level. By comparing the levels of production of a given morphogen among different tissues, it is possible to determine the tissue(s) of origin of that morphogen. The level of production of the morphogen OP-1 in different tissues is one example of a morphogen having a tissue of origin, i.e., the kidney, which contains a cell type that can also be used as the cell type which is used to screen, according to the invention, different compounds for their potential effects on morphogen (OP-1) production.

The level of OP-1 varies among different tissue types. In order to screen compounds for their effect on the production of OP-1 by a given cell type, it may be desirable to determine which tissues produce levels of OP-1 which are sufficiently high to show a potential decrease and sufficiently low to show a potential increase in production. Different tissues may be screened at the RNA level as follows.

Any probe capable of hybridizing specifically to a transcript, and distinguishing the transcript of interest from other, related transcripts may be used. Because the morphogens to be detected in the methods of this invention share such high sequence homology in their C-terminal domain, the tissue distribution of a specific morphogen transcript may best be determined using a probe specific for the "pro" region of the immature protein and/or the N-terminal heterogeneous region of the mature protein. Another useful probe sequence is the 3'non-coding region immediately following the stop codon. These portions of the sequence vary substantially among the morphogens of this invention, and accordingly, are specific for each protein. For example, a particularly useful Vgr-1-specific probe sequence is the PvuII-SacI fragment, a 265 bp fragment encoding both a portion of the pro region and the N-terminus of the mature sequence. Similarly, particularly useful mOP-1-specific probe sequences are the BstXI-BglI fragment, a 0.68kb sequence that covers approximately two-thirds of the mOP1 pro region; a StuI-StuI fragment, a 0.2 kb sequence immediately upstream of the 7-cysteine domain, and an EarI-PstI fragment, a 0.3kb fragment containing the 3'untranslated sequence. Similar approaches may be used, for example, with hOP-1 (SEQ. ID NO.16) or human or mouse OP-2 (SEQ. ID NOS.20 and 22).

Using morphogen-specific oligonucleotides probes, morphogen transcripts can be identified in mammalian tissues, using standard methodologies well known to those having ordinary skill in the art. Briefly, total RNA from mouse embryos and organs from post-natal animals is prepared using the acid guanidine thiocyanate-phenol-chloroform method (Chomczynski et al., Anal. Biochem. 162:156-159, 1987). The RNA may be dissolved in TES buffer (10 mM Tris-HCl, 1 mM EDTA, 0.1% SDS, pH 7.5) and treated with Proteinase K (approx. 1.5 mg per g tissue sample) at 45°C for 1 hr. Poly(A)⁺ RNA selection on oligo(dT)-cellulose (Type 7, Pharmacia LKB Biotechnology Inc., Piscataway, NJ) may be done in a batch procedure by mixing 0.1 g oligo(dT)-cellulose with 11 ml RNA solution (from 1 g tissue) in TES buffer and 0.5 M NaCl). Thereafter the oligo(dT) cellulose is washed in binding buffer (0.5 M NaCl, 10 mM Tris-HCl, 1 mM EDTA, pH 7.5) and poly(A)⁺ RNA is eluted with water. Poly(A)⁺ RNA (5 or 15 µg/lane) is fractionated on 1 or 1.2% agarose-formaldehyde gels (Selden, in Current Protocols in Molecular Biology, Ausubel et al. eds., pp. 1-4, 8, 9, Greene Publishing and Wiley-Interscience, New York, 1991). 1 µl of 400 µg/ml ethidium bromide is added to each sample prior to heat denaturation (Rosen et al., Focus 12:23-24, 1990). Following electrophoresis, the gels are photographed and the RNA is blotted overnight onto Nytran (RTM) nitrocellulose membranes (Schleicher & Schuell Inc., Keene, NH) with 10 x SSC. The membranes are baked at 80°C for 30-60 min. and irradiated with UV light (1 mW/cm² for 25 sec.). The Northern hybridization conditions may be as previously described (Ozkaynak et al., EMBO J. 9:2085-2093, 1990). For re-use, the filters may be deprobed in 1 mM Tris-HCl, 1 mM EDTA, 0.1% SDS, pH 7.5, at 90-95°C and exposed to film to assure complete removal of previous hybridization signals.

One probe which may be used to screen for transcripts encoding a morphogen includes a portion of or the complete OP-1 cDNA, which may be used to detect the presence of OP-1 mRNA or mRNAs of related morphogens. The sequence of the murine cDNA gene is set forth in SEQ ID NO:14.

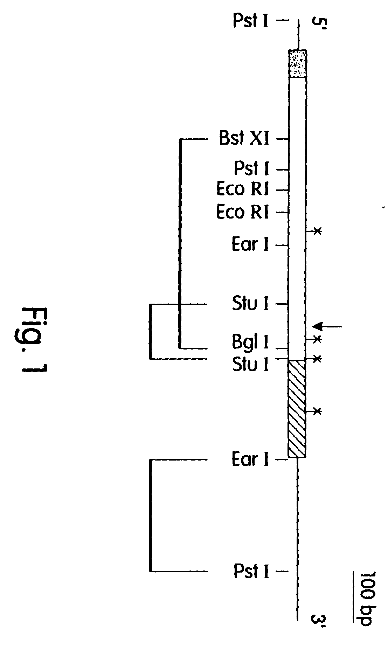

OP-1 mRNA expression was analyzed in 17 day mouse embryos and 3 day post-natal mice by sequentially hybridizing filters with various probes. Probes from regions other than the highly conserved 7-cysteine domain were selected because this region is highly variable among members of the TGF-β superfamily. Fig. 1 shows the fragments of OP-1, used as probes in the Northern hybridizations. The solid box indicates the putative signal peptide and the hatched box corresponds to the TGF-β-like domain that contains the seven cysteine residues. Asterisks indicate the potential N-glycosylation sites. The arrow marks the location of the cleavage site for OP-1 maturation. Three solid bars below the diagram indicate the OP-1 specific fragments used in making ³²P-labeled probes (0.68 kb BstXI - BglI fragment, 0.20 kb StuI - StuI fragment and 0.34 kb EarI - PstI non-coding fragment).

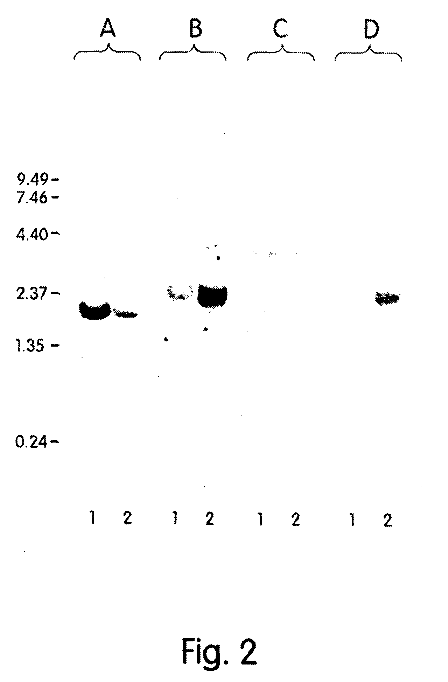

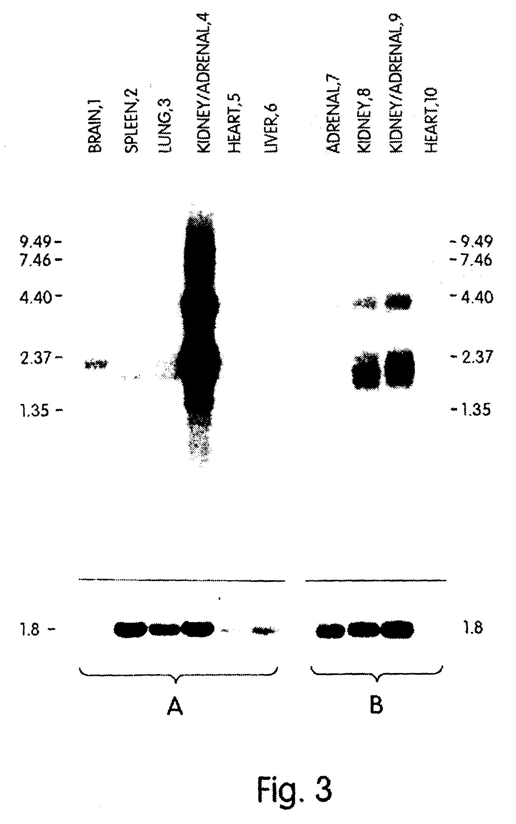

Hybridization with a probe that covers approximately two thirds of the pro region (the 0.68 kb BstXI-BglI fragment), reveals a 4 kb message and 3 messages at 1.8 kb, 2.2 kb and 2.4 kb (Fig. 2B and D, and Fig. 3). In the Northern hybridization of Fig. 2, equal amounts (15 µg) of poly(A)⁺ RNA were loaded into each lane, electrophoresed on a 1% agarose-formaldehyde gel, blotted and hybridized. A 0.24 - 9.49 kb RNA ladder (Bethesda Research Labs, Inc.) was used as size standard. The same filter was used for sequential hybridizations with labeled probes specific for OP-1 (Panels B and D), Vgr-1 (Panel C), and EF-Tu (Panel A). Panel A: the EF-Tu specific probe (a control) was the 0.4 kb HindIII-SacI fragment (part of the coding region), the SacI site used belonged to the vector; Panel B: the OP-1 specific probe was the 0.68 kb BstXI-BglI fragment (two thirds of the pro region and upstream sequences of the mature domain, not including any sequences from the 7-cysteine domain); Panel C: the Vgr-1 specific probe was the 0.26 kb PvuII-SacI fragment (part of the pro region and the amino-terminal sequences of the mature domain, including the first cysteine) (Lyons et al., 1989, Proc. Nat. Aca. Sci. 86: 4554, hereby incorporated by reference). Panel D: the OP-1 (3' flanking) specific probe was the 0.34 kb EarI-PstI fragment (3' untranslated sequences immediately following the sequences encoding OP-1).

In Fig. 3, the tissues to be used for RNA preparation were obtained from two week old mice (Panel A) or 5 week old mice (Panel B), with the exception of poly A+ RNA which was obtained from kidney adrenal gland of two week old mice (Panel B). Equal amounts of poly A+ RNA (15 µg for Panel A and 5 µg for Panel B) were loaded into each well. After electrophoresis (1.2% agarose-formaldehyde gels) and blotting, RNA was hybridized to the OP-1 specific 3' flanking probe described in the legend of Fig. 2 (Panel D). The 0.24-9.5 kb RNA ladder was used as size standard. The arrowheads indicate the OP-1 specific messages. The lower section of Panels A and B show the hybridization pattern obtained with the EF-Tu specific probe (a control).

Although the size of the Vgr-1 specific message is close to the 4 kb OP-1 species (Fig. 2 Panel C), the OP-1 4 kb mRNA is somewhat larger. To further rule out cross-hybridization with a non-OP-1 message, the 0.2 kb StuI-StuI fragment which represents the gene specific sequences immediately upstream of those encoding the 7-cysteine domain was used. This probe gave a hybridization pattern similar to the one shown in Fig. 2 Panel B (data not shown). A third probe, the 0.34 kb EarI-PstI fragment containing 3' untranslated sequences, also confirmed the pattern (Fig. 2 Panel D). Thus, the same four OP-1 specific messages were observed with three distinct probes.

The appearance of a new 4 kb OP-1 mRNA species was initially interpreted as cross hybridization of the OP-1 probe with Vgr-1 mRNA, which is approximately this size (Fig. 2 Panel C). However, the 4 kb message was detected with three different OP-1 specific probes, including one specific to the 3' untranslated region, and moreover it was separated from Vgr-1 message on the basis of size. Most likely, therefore, the 4 kb mRNA (and the three species of 1.8 kb, 2.2 kb and 2.4 kb) results from alternative splicing of OP-1 transcripts. The 4 kb OP-1 mRNA could also represent a bicistronic mRNA. The 4 kb message is a minor species in kidney, while it is more prominent in adrenal tissue.

The level of OP-1 expression was compared in different tissues using poly(A)⁺ RNA prepared from brain, spleen, lung, kidney and adrenal gland, heart, and liver of 13 day post-natal mice. The RNA was analyzed on Northern blots by hybridization to various probes (Fig. 3. Equal amounts of mRNA, as judged by optical density, were fractionated on agarose formaldehyde gels. Ethidium bromide staining of the gels revealed some residual ribosomal RNA in addition to the mRNA and provided another assurance that the mRNA was not degraded and that there was not significant quantitative or qualitative variation in the preparation. As control for mRNA recovery, EF-Tu (translational elongation factor) mRNA was probed (assuming uniform expression of EF-Tu in most tissues). A great variation in the level of OP-1 expression was observed in spleen, lung, kidney and adrenal tissues whereas EF-Tu mRNA levels appeared relatively constant in these tissues (Fig. 3 Panel A). The highest level of OP-1 mRNA was found in the kidneys. Uniformly lower levels of EF-Tu mRNA were found in brain, heart and liver (Fig. 3 Panel A). Additional analysis of OP-1 mRNA showed the presence of significant amounts of OP-1 mRNA in the bladder (data not shown). In summary, next to kidney, bladder and adrenal tissue, brain tissue contained the highest levels of OP-1 RNA, whereas heart and liver did not give detectable signals.

OP-1 mRNA patterns display qualitative changes in the various tissues. Of the four messages found in brain, the 2.2 kB message is most abundant whereas in lung and spleen the 1.8 kb message predominates. Levels of the 1.8-2.4 kb in the kidney OP-1 mRNA are approximately two times higher in 3 day post-natal mice than in 17 day embryos, perhaps reflecting phases in bone and/or kidney development. mRNA was also prepared from carefully separated renal and adrenal tissues of 5 week old mice. Northern blot analysis (Figure 4, Panel B) revealed that the high levels of 2.2 kb mRNA were derived from renal tissue whereas the 4 kb mRNA was more prominent in adrenal tissue.

The detection of of OP-1 message primarily in the kidney but also in bladder links OP-1 expression specifically with the urinary tract. Interestingly, the related Vgr-1 is also expressed at significant levels in kidney although its main site of expression in lung.

Once the tissue-specific expression of a given morphogen is known, cell types known to exist in that tissue or cell lines derived from that tissue can be screened, in a similar manner, to identify the cell type within that tissue that is actually responsible for the tissue specific synthesis and secretion of the morphogen. Once a cell type which produces the morphogen in an amount sufficient to detect increases or decreases in the production level of the morphogen upon exposure to a compound is identified, it may be used in tissue culture assay to rapidly screen for the ability of compound to upregulate or down regulate the synthesis and secretion of the morphogen. The level of morphogen production by the chosen cell type is determined with and without incubating the cell in culture with the compound, in order to assess the effects of the compound on the cell's ability to synthesize or secrete the morphogen. This can be accomplished by detection of the level of production of the morphogen either at the protein or mRNA level.

### 4. Growth of Cells in Culture

Cell cultures derived from kidney, adrenals, urinary bladder, brain, or other organs, may be prepared as described widely in the literature. For example, kidneys may be explanted from neonatal, new born, young or adult rodents (mouse or rat) and used in organ culture as whole or sliced (1-4 mm) tissues. Primary tissue cultures and established cell lines, also derived from kidney, adrenals, urinary, bladder, brain, or other tissues may be established in multiwell plates (6 well, 24 well, or 96 well) according to conventional cell culture techniques, and are cultured in the absence or presence of serum for a period of time (1-7 days). Cells may be cultured, for example, in Dulbecco's Modified Eagle medium (Gibco, Long Island, NY) containing serum (e.g., fetal calf serum at 1%-10%, Gibco) or in serum-deprived medium, as desired, or in defined medium (e.g., containing insulin, transferrin, glucose, albumin, or other growth factors).

Samples for testing the level of morphogen production include culture supernatants or cell lysates, collected periodically and evaluated for OP-1 production by immunoblot analysis of a portion of the cell culture itself, collected periodically and used to prepare polyA+ RNA for RNA analysis (Sambrook et al., eds., Molecular Cloning, 1989, Cold Spring Harbor Press, Cold Spring Harbor, NY). To monitor de novo OP-1 synthesis, some cultures are labeled with ³⁵S-methionine/³⁵S-cysteine mixture for 6-24 hours and then evaluated for morphogen production by conventional immunoprecipitation methods (Sambrook et al., eds., Molecular Cloning, 1989, Cold Spring Harbor Press, Cold Spring Harbor, NY). Alternatively, the production of morphogen or determination of the level of morphogen production may be ascertained using a simple assay for a parameter of cell growth, e.g., cellular proliferation or death. For example, where a morphogen is produced by a cultured cell line, the addition of antibody specific for the morphogen may result in relief from morphogen inhibition of cell growth. Thus, measurement of cellular proliferation can be used as an indication of morphogen production by a tissue.

### 5. Determination of Level of Morphogenic Protein

In order to quantitate the production of a morphogenic protein by a cell type, an immunoassay may be performed to detect the morphogen using a polyclonal or monoclonal antibody specific for that morphogen. For example, OP-1 may be detected using a polyclonal antibody specific for OP-1 in an ELISA, as follows.

1 µg/100 ul of affinity-purified polyclonal rabbit IgG specific for OP-1 is added to each well of a 96-well plate and incubated at 37°C for an hour. The wells are washed four times with 0.16M sodium borate buffer with 0.15 M NaCl (BSB), pH 8.2, containing 0.1% Tween 20. To minimize non-specific binding, the wells are blocked by filling completely with 1% bovine serum albumin (BSA) in BSB for 1 hour at 37°C. The wells are then washed four times with BSB containing 0.1% Tween 20. A 100 ul aliquot of an appropriate dilution of each of the test samples of cell culture supernatant is added to each well in triplicate and incubated at 37°C for 30 min. After incubation, 100 ul biotinylated rabbit anti-OP-1 serum (stock solution is about 1 mg/ml and diluted 1:400 in BSB containing 1% BSA before use) is added to each well and incubated at 37°C for 30 min. The wells are then washed four times with BSB containing 0.1% Tween 20 (RTM). 100 ul strepavidin-alkaline (Southern Biotechnology Associates, Inc. Birmingham, Alabama, diluted 1:2000 in BSB containing 0.1% Tween® 20 before use) is added to each well and incubated at 37°C for 30 min. The plates are washed four times with 0.5M Tris buffered Saline (TBS), pH 7.2. 50ul substrate (ELISA Amplification System Kit, Life Technologies, Inc., Bethesda, MD) are added to each well incubated at room temperature for 15 min. Then, 50 ul amplifier (from the same amplification system kit) is added and incubated for another 15 min at room temperature. The reaction is stopped by the addition of 50 ul 0.3 M sulphuric acid. The OD at 490 nm of the solution in each well is recorded. To quantitate OP-1 in culture media, a OP-1 standard curve is performed in parallel with the test samples.

### 6. Preparation of Polyclonal Antibody

Polyclonal antibody is prepared as follows. Each rabbit is given a primary immunization of 100 ug/500 ul E. coli-produced OP-1 monomer (amino acids 328-431 of SEQ. ID NO: 11) in 0.1% SDS mixed with 500 ul Complete Freund's Adjuvant. The antigen is injected subcutaneously at multiple sites on the back and flanks of the animal. The rabbit is boosted after a month in the same manner using incomplete Freund's Adjuvant. Test bleeds are taken from the ear vein seven days later. Two additional boosts and test bleeds are performed at monthly intervals until antibody against OP-1 is detected in the serum using an ELISA assay. Then, the rabbit is boosted monthly with 100 ug of antigen and bled (15 ml per bleed) at days seven and ten after boosting.

### 7. Preparation of Monoclonal Antibody and Neutralizing Mon oclonal Antibody

Monoclonal antibody specific for a given morphogen may be prepared as follows. A mouse is given two injections of E. coli produced OP-1 monomer (amino acids 328-431 in SEQ ID NO:11). The first injection contains 100ug of OP-1 in complete Freund's adjuvant and is given subcutaneously. The second injection contains 50 ug of OP-1 in incomplete adjuvant and is given intraperitoneally. The mouse then receives a total of 230 ug of OP-1 (amino acids 307-431 of SEQ ID NO:11) in four intraperitoneal injections at various times over an eight month period. One week prior to fusion, The mouse is boosted intraperitoneally with 100 ug of OP-1 (15-139) and 30 ug of the N-terminal peptide (Ser293-Asn309-Cys) conjugated through the added cys residue to bovine serum albumin with SMCC crosslinking agent. This boost is repeated five days (IP), four days (IP), three days (IP) and one day (IV) prior to fusion. The mouse spleen cells are then fused to myeloma (e.g., 653) cells at a ratio of 1:1 using PEG 1500 (Boehringer Mannheim), and the cell fusion is plated and screened for OP-1-specific antibodies using OP-1 (307-431) as antigen. The cell fusion and monoclonal screening are according to procedures widely available in the art. The neutralizing monoclonal is identified by its ability to block the biological activity of OP-1 when added to a cellular assay which responds biologically to added OP-1.

### 8. Identification of OP-1 Producing Cell Line Which Displays OP-1 Surface Receptors

During the process of routinely testing the effects of increasing concentrations of OP-1 and TGF-β on the proliferation of various cell lines, a cell line was identified which, surprising, appears not only to synthesize and secrete OP-1, but also to display cell surface receptors to which the secreted OP-1 binds and acts to inhibit proliferation of the cells. This cell line was identified after the following observations.Addition of increasing concentrations of OP-1 or TGF-β failed to increase or decrease the relatively low basal rate of proliferation of the cells. However, addition of a monoclonal antibody, which neutralizes the activity of Op-1, resulted in a large increase in the proliferation of the cells. In addition, simultaneous addition of the same quantity of OP-1 neutralizing monoclonal to a fixed amount of OP-1 resulted in an increase in proliferation which was intermediate between the low basal level observed with OP-1 alone and the high level observed with the monoclonal alone.This cell line, which is an epithelial cell line that was derived from a bladder cell carcinoma, may be used in an assay of the invention. The parameter to be tested according to the invention is cellular proliferation. Thus, a compound(s) that increases or decreases the level of OP-1 production may be tested on this cell line as follows..

### 9. Assay for Identifying Drugs Which Affect OP-1 Synthesis

A simple medium flux screening assay can be configured in a standard 24 or 96 well microtiter dish , in which each well contains a constant number of a cell line having the characteristics described above. Increasing concentrations of an OP-1 neutralizing monoclonal antibody is added from left to right across the dish. A constant amount of different test substances is added from top to bottom on the dish. An increase in the synthesis and secretion of OP-1 (over its constitutive (non-induced) level) will be indicated by an increase in the amount of OP-1 neutralizing antibody required to release the cells from the antimitogenic activity of OP-1. A decrease in the synthesis and secretion of OP-1 (below its constitutive (repressed) level) will be indicated by the observation that decreased concentrations of the OP-1 neutralizing monoclonal antibody will be required to release the cells from the antimitogenic activity of OP-1. One of the major advantages of this assay is that the end point, i.e., the dilution of antibody which has an effect on cell proliferation, is a measure of mitosis, or an increase in the number of cells per well. Because several convenient and rapid assays exist for quantitating cell numbers, this assay is faster and requires significantly fewer steps to perform.

The assay may be performed as follows. After addition of appropriate concentrations of the OP-1 neutralizing monoclonal antibody and test substances to the wells containing the cells, the dishes are placed in an incubator at 37°C for a period of 1-3 days. After completion of incubation/growth period, the dishes are removed and the cells in the individual wells are washed and stained with a vital stain, such as crystal violet. Washing and staining procedures are well-known in the art. The cells are then lysed and the stain dissolved in a constant amount of a solvent, such as ethanol. Quantitations of the dissolved stain, which is readily performed on an automated plate vendor, allows for direct quantitation of the number of cells in each well.

The above-described assay has the advantages of being rapid and easy to perform becaue it requires few steps. Another advantage is intrinsic to the assay; drugs which are screened according to this procedure that result in cell death (i.e., cytotoxic substances) are immediately, identifiable without the need of operator observation. In addition, although drugs that stop the growth of the cells (i.e., cytostatic substances) are scored as positive due to failure to see increases in cell numbers, they are automatically scored as suspect due to the failure of the highest concentrations of OP-1 neutralizing monoclonal antibody to release the cells from the antimitogenic activity of OP-1.

SEQUENCE LISTING

(1) GENERAL INFORMATION:

(i) APPLICANT: John Smart
Herman Oppermann
Engin Ozkaynak
Thangavel Kuberasampath
David C. Rueger
Roy H.L. Pang
Charles M. Cohen
(ii) TITLE OF INVENTION: MORPHOGENIC
PROTEIN SCREENING METHOD
(iii) NUMBER OF SEQUENCES: 33
(iv) CORRESPONDENCE ADDRESS:
(A) ADDRESSEE: Creative BioMolecules
(B) STREET: 35 South Street
(C) CITY: Hopkinton
(D) STATE: Massachusetts
(E) COUNTRY: U.S.A.
(F) ZIP: 01748
(v) COMPUTER READABLE FORM:
(A) MEDIUM TYPE: Diskette, 5.25,
360kb storage
(B) COMPUTER: IBM XT
(C) OPERATING SYSTEM: DOS 3.30
(D) SOFTWARE: ASC II TEXT
(vi) PRIOR APPLICATION DATA:
(A) APPLICATION NUMBER: US 667,274
(B) FILING DATE: March 11, 1991
(vii) PRIOR APPLICATION DATA:
(A) APPLICATION NUMBER: US 752,861
(B) FILING DATE: AUGUST 30, 1991

(viii)ATTORNEY/AGENT INFORMATION
(A) NAME: PITCHER, EDMUND R.
(B) REG. NO.: 27,829
(C) DOCKET NO.: CRP-058PC
(ix) TELEPHONE:
(A) 617/248-7000
(B) TELEFAX: 617/248-7100

(2)        INFORMATION FOR SEQ ID NO:1:

(i)   SEQUENCE CHARACTERISTICS:
      (A) LENGTH:  97 amino acids
      (B) TYPE:  amino acids
      (C) TOPOLOGY:  linear
(ii)  MOLECULE TYPE:  protein
(ix)  FEATURE:
      (A) NAME:  Generic Sequence 1
      (D) OTHER INFORMATION:  Each Xaa
          indicates one of the 20 naturally-
          occurring L-isomer, α-amino acids
          or a derivative thereof.
(xi)  SEQUENCE DESCRIPTION:  SEQ ID NO:1:
                    Xaa Xaa Xaa Xaa Xaa Xaa
                     1               5
Xaa Xaa Xaa Xaa Xaa Xaa Xaa Xaa Xaa Xaa Xaa
           10                  15
Xaa Xaa Xaa Xaa Xaa Xaa Xaa Cys Xaa Xaa Xaa
       20                  25
Cys Xaa Xaa Xaa Xaa Xaa Xaa Xaa Xaa Xaa Xaa
   30                  35
Xaa Xaa Xaa Xaa Xaa Xaa Xaa Xaa Xaa Xaa Xaa
40                  45                  50
Xaa Xaa Xaa Xaa Xaa Xaa Xaa Xaa Xaa Xaa Cys
               55                  60
Cys Xaa Xaa Xaa Xaa Xaa Xaa Xaa Xaa Xaa Xaa
           65                  70
Xaa Xaa Xaa Xaa Xaa Xaa Xaa Xaa Xaa Xaa Xaa
       75                  80
Xaa Xaa Xaa Xaa Xaa Xaa Xaa Xaa Xaa Xaa Cys
   85                  90
Xaa Cys Xaa
95

(2) INFORMATION FOR SEQ ID NO:2:

(i) SEQUENCE CHARACTERISTICS:
(A) LENGTH: 97 amino acids
(B) TYPE: amino acids
(C) TOPOLOGY: linear
(ii) MOLECULE TYPE: protein
(ix) FEATURE:
(A) NAME: Generic Sequence 2
(D) OTHER INFORMATION: Each Xaa
indicates one of the 20 naturally-
occurring L-isomer, α-amino acids
or a derivative thereof.
(xi) SEQUENCE DESCRIPTION: SEQ ID NO:2:

Xaa Xaa Xaa Xaa Xaa Xaa
1               5
Xaa Xaa Xaa Xaa Xaa Xaa Xaa Xaa Xaa Xaa Xaa
            10                  15
Xaa Xaa Xaa Xaa Xaa Xaa Xaa Cys Xaa Xaa Xaa
        20                  25
Cys Xaa Xaa Xaa Xaa Xaa Xaa Cys Xaa Xaa Xaa
    30                  35
Xaa Xaa Xaa Xaa Xaa Xaa Xaa Xaa Xaa Xaa Xaa
40                  45                  50
Xaa Xaa Xaa Xaa Xaa Xaa Xaa Xaa Xaa Xaa Cys
                55                  60
Cys Xaa Xaa Xaa Xaa Xaa Xaa Xaa Xaa Xaa Xaa
            65                  70
Xaa Xaa Xaa Xaa Xaa Xaa Xaa Xaa Xaa Xaa Xaa
        75                  80
Xaa Xaa Xaa Xaa Xaa Xaa Xaa Xaa Xaa Xaa Cys
    85                  90
Xaa Cys Xaa
95

(2)     INFORMATION FOR SEQ ID NO:3:

     (i)     SEQUENCE CHARACTERISTICS:
             (A) LENGTH:  97 amino acids
             (B) TYPE:  amino acids
             (C) TOPOLOGY:  linear
     (ii)    MOLECULE TYPE:  protein
     (ix)    FEATURE:
             (A) NAME: Generic Sequence 3
             (D) OTHER INFORMATION:  wherein each
                 Xaa is independently selected from
                 a group of one or more specified
                 amino acids as defined in the
                 specification.

     (xi)    SEQUENCE DESCRIPTION:  SEQ ID NO:3:

             Leu Tyr Val Xaa Phe
             1               5
     Xaa Xaa Xaa Gly Trp Xaa Xaa Trp Xaa
                         10
     Xaa Ala Pro Gly Xaa Xaa Xaa Ala
      15                     20
     Xaa Tyr Cys Xaa Gly Xaa Cys Xaa
             25                      30
     Xaa Pro Xaa Xaa Xaa Xaa Xaa
                         35
     Xaa Xaa Xaa Asn His Ala Xaa Xaa
             40                      45
     Xaa Xaa Leu Xaa Xaa Xaa Xaa Xaa
                         50
     Xaa Xaa Xaa Xaa Xaa Xaa Xaa Cys
         55                      60
     Cys Xaa Pro Xaa Xaa Xaa Xaa Xaa
                     65

Xaa Xaa Xaa Leu Xaa Xaa Xaa
70                    75
Xaa Xaa Xaa Xaa Val Xaa Leu Xaa
                80
Xaa Xaa Xaa Xaa Met Xaa Val Xaa
85                    90
Xaa Cys Gly Cys Xaa
            95

(2)     INFORMATION FOR SEQ ID NO:4:

        (i)    SEQUENCE CHARACTERISTICS:
               (A) LENGTH:  102 amino acids
               (B) TYPE:  amino acids
               (C) TOPOLOGY:  linear
        (ii)   MOLECULE TYPE:  protein
        (ix)   FEATURE:
               (A) NAME: Generic Sequence 4
               (D) OTHER INFORMATION:  wherein each
                   Xaa is independently selected from
                   a group of one or more specified
                   amino acids as defined in the
                   specification.

        (xi)   SEQUENCE DESCRIPTION:  SEQ ID NO:4:

Cys Xaa Xaa Xaa Xaa Leu Tyr Val Xaa Phe
1                   5                   10
Xaa Xaa Xaa Gly Trp Xaa Xaa Trp Xaa
                15
Xaa Ala Pro Xaa Gly Xaa Xaa Ala
20                    25
Xaa Tyr Cys Xaa Gly Xaa Cys Xaa
            30                    35
Xaa Pro Xaa Xaa Xaa Xaa Xaa
                40

Asn Xaa Xaa Asn His Ala Xaa Xaa
        45                  50
Xaa Xaa Leu Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70
Xaa Xaa Xaa Leu Xaa Xaa Xaa
75                  80
Xaa Xaa Xaa Xaa Val Xaa Leu Xaa
            85
Xaa Xaa Xaa Xaa Met Xaa Val Xaa
90                  95
Xaa Cys Gly Cys Xaa
        100

(2) INFORMATION FOR SEQ ID NO:5:
(i) SEQUENCE CHARACTERISTICS:
(A) LENGTH: 139 amino acids
(B) TYPE: amino acids
(C) TOPOLOGY: linear
(ii) MOLECULE TYPE: protein
(ix) FEATURE:
(A) NAME: hOP-1 (mature form)
(xi) SEQUENCE DESCRIPTION: SEQ ID NO:5:

Ser Thr Gly Ser Lys Gln Arg Ser Gln
1               5
Asn Arg Ser Lys Thr Pro Lys Asn Gln
10                  15
Glu Ala Leu Arg Met Ala Asn Val Ala
    20                  25
Glu Asn Ser Ser Ser Asp Gln Arg Gln
        30                  35

Ala Cys Lys Lys His Glu Leu Tyr Val
            40                  45
Ser Phe Arg Asp Leu Gly Trp Gln Asp
                50
Trp Ile Ile Ala Pro Glu Gly Tyr Ala
55                  60
Ala Tyr Tyr Cys Glu Gly Glu Cys Ala
    65                  70
Phe Pro Leu Asn Ser Tyr Met Asn Ala
        75                  80
Thr Asn His Ala Ile Val Gln Thr Leu
            85                  90
Val His Phe Ile Asn Pro Glu Thr Val
                95
Pro Lys Pro Cys Cys Ala Pro Thr Gln
100                 105
Leu Asn Ala Ile Ser Val Leu Tyr Phe
    110                 115
Asp Asp Ser Ser Asn Val Ile Leu Lys
        120                 125
Lys Tyr Arg Asn Met Val Val Arg Ala
            130                 135
Cys Gly Cys His

(2)    INFORMATION FOR SEQ ID NO:6:
       (i)   SEQUENCE CHARACTERISTICS:
             (A) LENGTH:  139 amino acids
             (B) TYPE:  amino acids
             (C) TOPOLOGY:  linear
       (ii)  MOLECULE TYPE:  protein
       (ix)  FEATURE:
             (A) NAME:  mOP-1 (mature form)
       (xi)  SEQUENCE DESCRIPTION:  SEQ ID NO:6:

Ser  Thr  Gly  Gly  Lys  Gln  Arg  Ser  Gln
 1                   5
Asn  Arg  Ser  Lys  Thr  Pro  Lys  Asn  Gln
 10                       15
Glu  Ala  Leu  Arg  Met  Ala  Ser  Val  Ala
      20                       25
Glu  Asn  Ser  Ser  Ser  Asp  Gln  Arg  Gln
           30                       35
Ala  Cys  Lys  Lys  His  Glu  Leu  Tyr  Val
                40                       45
Ser  Phe  Arg  Asp  Leu  Gly  Trp  Gln  Asp
                     50
Trp  Ile  Ile  Ala  Pro  Glu  Gly  Tyr  Ala
 55                       60
Ala  Tyr  Tyr  Cys  Glu  Gly  Glu  Cys  Ala
      65                       70
Phe  Pro  Leu  Asn  Ser  Tyr  Met  Asn  Ala
           75                       80
Thr  Asn  His  Ala  Ile  Val  Gln  Thr  Leu
                85                       90
Val  His  Phe  Ile  Asn  Pro  Asp  Thr  Val
                     95
Pro  Lys  Pro  Cys  Cys  Ala  Pro  Thr  Gln
100                      105
Leu  Asn  Ala  Ile  Ser  Val  Leu  Tyr  Phe
     110                      115

Asp Asp Ser Ser Asn Val Ile Leu Lys
        120                 125
Lys Tyr Arg Asn Met Val Val Arg Ala
            130                 135
Cys Gly Cys His

(2) INFORMATION FOR SEQ ID NO:7:
(i) SEQUENCE CHARACTERISTICS:
(A) LENGTH: 139 amino acids
(B) TYPE: amino acids
(C) TOPOLOGY: linear
(ii) MOLECULE TYPE: protein
(ix) FEATURE:
(A) NAME: hOP-2 (mature form)
(xi) SEQUENCE DESCRIPTION: SEQ ID NO:7:

Ala Val Arg Pro Leu Arg Arg Arg Gln
1               5
Pro Lys Lys Ser Asn Glu Leu Pro Gln
10                  15
Ala Asn Arg Leu Pro Gly Ile Phe Asp
    20                  25
Asp Val His Gly Ser His Gly Arg Gln
        30                  35
Val Cys Arg Arg His Glu Leu Tyr Val
            40                  45
Ser Phe Gln Asp Leu Gly Trp Leu Asp
                50
Trp Val Ile Ala Pro Gln Gly Tyr Ser
55                  60
Ala Tyr Tyr Cys Glu Gly Glu Cys Ser
    65                  70
Phe Pro Leu Asp Ser Cys Met Asn Ala
        75                  80
Thr Asn His Ala Ile Leu Gln Ser Leu
            85                  90

Val His Leu Met Lys Pro Asn Ala Val
                95
Pro Lys Ala Cys Cys Ala Pro Thr Lys
100                 105
Leu Ser Ala Thr Ser Val Leu Tyr Tyr
    110                 115
Asp Ser Ser Asn Asn Val Ile Leu Arg
        120                 125
Lys His Arg Asn Met Val Val Lys Ala
            130                 135
Cys Gly Cys His

(2) INFORMATION FOR SEQ ID NO:8:
(i) SEQUENCE CHARACTERISTICS:
(A) LENGTH: 139 amino acids
(B) TYPE: amino acids
(C) TOPOLOGY: linear
(ii) MOLECULE TYPE: protein
(ix) FEATURE:
(A) NAME: mOP-2 (mature form)
(xi) SEQUENCE DESCRIPTION: SEQ ID NO:8:

Ala Ala Arg Pro Leu Lys Arg Arg Gln
1               5
Pro Lys Lys Thr Asn Glu Leu Pro His
10                  15
Pro Asn Lys Leu Pro Gly Ile Phe Asp
    20                  25
Asp Gly His Gly Ser Arg Gly Arg Glu
        30                  35
Val Cys Arg Arg His Glu Leu Tyr Val
            40                  45
Ser Phe Arg Asp Leu Gly Trp Leu Asp
                50
Trp Val Ile Ala Pro Gln Gly Tyr Ser
55                  60

Ala Tyr Tyr Cys Glu Gly Glu Cys Ala
    65                  70
Phe Pro Leu Asp Ser Cys Met Asn Ala
        75                  80
Thr Asn His Ala Ile Leu Gln Ser Leu
            85                  90
Val His Leu Met Lys Pro Asp Val Val
                95
Pro Lys Ala Cys Cys Ala Pro Thr Lys
100                 105
Leu Ser Ala Thr Ser Val Leu Tyr Tyr
    110                 115
Asp Ser Ser Asn Asn Val Ile Leu Arg
        120                 125
Lys His Arg Asn Met Val Val Lys Ala
            130                 135
Cys Gly Cys His

(2)	INFORMATION FOR SEQ ID NO:9:
	(i)	SEQUENCE CHARACTERISTICS:
		(A) LENGTH:  96 amino acids
		(B) TYPE:  amino acids
		(C) TOPOLOGY:  linear
	(ii)	MOLECULE TYPE:  protein
	(ix)	FEATURE:
		(A) NAME:  CBMP-2A(fx)
	(xi)	SEQUENCE DESCRIPTION:  SEQ ID NO:9:

Cys Lys Arg His Pro Leu Tyr Val Asp Phe Ser
 1              5                   10
Asp Val Gly Trp Asn Asp Trp Ile Val Ala Pro
            15                  20
Pro Gly Tyr His Ala Phe Tyr Cys His Gly Glu
        25                  30
Cys Pro Phe Pro Leu Ala Asp His Leu Asn Ser
    35                  40
Thr Asn His Ala Ile Val Gln Thr Leu Val Asn
 45                 50                  55
Ser Val Asn Ser Lys Ile Pro Lys Ala Cys Cys
                60                  65
Val Pro Thr Glu Leu Ser Ala Ile Ser Met Leu
            70                  75
Tyr Leu Asp Glu Asn Glu Lys Val Val Leu Lys
        80                  85
Asn Tyr Gln Asp Met Val Val Glu Gly Cys Gly
    90                  95
Cys Arg
100

(2)	INFORMATION FOR SEQ ID NO:10:
	(i)	SEQUENCE CHARACTERISTICS:
		(A) LENGTH:  101 amino acids
		(B) TYPE:  amino acids
		(C) TOPOLOGY:  linear
	(ii)	MOLECULE TYPE:  protein
	(ix)	FEATURE:
		(A) NAME:  CBMP-2B(fx)
	(xi)	SEQUENCE DESCRIPTION:  SEQ ID NO:10:

Cys Arg Arg His Ser
1               5
Leu Tyr Val Asp Phe Ser Asp Val Gly Trp Asn
                10                  15
Asp Trp Ile Val Ala Pro Pro Gly Tyr Gln Ala
            20                  25
Phe Tyr Cys His Gly Asp Cys Pro Phe Pro Leu
        30                  35
Ala Asp His Leu Asn Ser Thr Asn His Ala Ile
    40                  45
Val Gln Thr Leu Val Asn Ser Val Asn Ser Ser
50                  55                  60
Ile Pro Lys Ala Cys Cys Val Pro Thr Glu Leu
                65                  70
Ser Ala Ile Ser Met Leu Tyr Leu Asp Glu Tyr
            75                  80
Asp Lys Val Val Leu Lys Asn Tyr Gln Glu Met
        85                  90
Val Val Glu Gly Cys Gly Cys Arg
    95                  100

(2)	INFORMATION FOR SEQ ID NO:11:
	(i)	SEQUENCE CHARACTERISTICS:
		(A) LENGTH:  102 amino acids
		(B) TYPE:  amino acids
		(C) TOPOLOGY:  linear
	(ii)	MOLECULE TYPE:  protein
	(ix)	FEATURE:
		(A) NAME:  DPP(fx)
	(xi)	SEQUENCE DESCRIPTION:  SEQ ID NO:11:

Cys Arg Arg His Ser Leu Tyr Val Asp Phe Ser
 1              5                   10
Asp Val Gly Trp Asp Asp Trp Ile Val Ala Pro
            15                  20
Leu Gly Tyr Asp Ala Tyr Tyr Cys His Gly Lys
        25                  30
Cys Pro Phe Pro Leu Ala Asp His Phe Asn Ser
    35                  40
Thr Asn His Ala Val Val Gln Thr Leu Val Asn
45                  50                  55
Asn Asn Asn Pro Gly Lys Val Pro Lys Ala Cys
                60                  65
Cys Val Pro Thr Gln Leu Asp Ser Val Ala Met
            70                  75
Leu Tyr Leu Asn Asp Gln Ser Thr Val Val Leu
        80                  85
Lys Asn Tyr Gln Glu Met Thr Val Val Gly Cys
    90                  95
Gly Cys Arg
100

(2)	INFORMATION FOR SEQ ID NO:12:
	(i)	SEQUENCE CHARACTERISTICS:
		(A) LENGTH:  102 amino acids
		(B) TYPE:  amino acids
		(C) TOPOLOGY:  linear
	(ii)	MOLECULE TYPE:  protein
	(ix)	FEATURE:
		(A) NAME:  Vgl(fx)
	(xi)	SEQUENCE DESCRIPTION:  SEQ ID NO:12:

Cys Lys Lys Arg His Leu Tyr Val Glu Phe Lys
  1               5                  10
Asp Val Gly Trp Gln Asn Trp Val Ile Ala Pro
            15                  20
Gln Gly Tyr Met Ala Asn Tyr Cys Tyr Gly Glu
        25                  30
Cys Pro Tyr Pro Leu Thr Glu Ile Leu Asn Gly
    35                  40
Ser Asn His Ala Ile Leu Gln Thr Leu Val His
 45                  50                  55
Ser Ile Glu Pro Glu Asp Ile Pro Leu Pro Cys
                60                  65
Cys Val Pro Thr Lys Met Ser Pro Ile Ser Met
            70                  75
Leu Phe Tyr Asp Asn Asn Asp Asn Val Val Leu
        80                  85
Arg His Tyr Glu Asn Met Ala Val Asp Glu Cys
    90                  95
Gly Cys Arg
100

(2)	INFORMATION FOR SEQ ID NO:13:
	(i)	SEQUENCE CHARACTERISTICS:
		(A) LENGTH:  102 amino acids
		(B) TYPE:  amino acids
		(C) TOPOLOGY:  linear
	(ii)	MOLECULE TYPE:  protein
	(ix)	FEATURE:
		(A) NAME:  Vgr-1(fx)
	(xi)	SEQUENCE DESCRIPTION:  SEQ ID NO:13:

Cys Lys Lys His Glu Leu Tyr Val Ser Phe Gln
1               5                   10
Asp Val Gly Trp Gln Asp Trp Ile Ile Ala Pro
            15                  20
Xaa Gly Tyr Ala Ala Asn Tyr Cys Asp Gly Glu
        25                  30
Cys Ser Phe Pro Leu Asn Ala His Met Asn Ala
    35                  40
Thr Asn His Ala Ile Val Gln Thr Leu Val His
45                  50                  55
Val Met Asn Pro Glu Tyr Val Pro Lys Pro Cys
                60                  65
Cys Ala Pro Thr Lys Val Asn Ala Ile Ser Val
            70                  75
Leu Tyr Phe Asp Asp Asn Ser Asn Val Ile Leu
        80                  85
Lys Lys Tyr Arg Asn Met Val Val Arg Ala Cys
    90                  95
Gly Cys His
100

(2) INFORMATION FOR SEQ ID NO:14:

(i) SEQUENCE CHARACTERISTICS:
(A) LENGTH: 106 amino acids
(B) TYPE: protein
(C) STRANDEDNESS: single
(D) TOPOLOGY: linear
(ii) MOLECULE TYPE: protein

(vi) ORIGINAL SOURCE:
(A) ORGANISM: human
(F) TISSUE TYPE: BRAIN

(ix) FEATURE:
(D) OTHER INFORMATION:
/product= "GDF-1 (fx)"

(xi) SEQUENCE DESCRIPTION: SEQ ID NO:14:

Cys Arg Ala Arg Arg Leu Tyr Val Ser Phe Arg Glu Val Gly
1               5                   10

Trp His Arg Trp Val Ile Ala Pro Arg Gly Phe Leu Ala Asn Tyr
15                  20                  25

Cys Gln Gly Gln Cys Ala Leu Pro Val Ala Leu Ser Gly Ser Gly
30                  35                  40

Gly Pro Pro Ala Leu Asn His Ala Val Leu Arg Ala Leu Met His
45                  50                  55

Ala Ala Ala Pro Gly Ala Ala Asp Leu Pro Cys Cys Val Pro Ala
60                  65                  70

Arg Leu Ser Pro Ile Ser Val Leu Phe Phe Asp Asn Ser Asp Asn
75                  80                  85

Val Val Leu Arg Gln Tyr Glu Asp Met Val Val Asp Glu Cys Gly
90                  95                  100

Cys Arg
105

(2) INFORMATION FOR SEQ ID NO:15:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:15:

Cys Xaa Xaa Xaa Xaa
1               5

(2) INFORMATION FOR SEQ ID NO:16:

(i) SEQUENCE CHARACTERISTICS:
(A) LENGTH: 1822 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: HOMO SAPIENS
(F) TISSUE TYPE: HIPPOCAMPUS

(ix) FEATURE:
(A) NAME/KEY: CDS
(B) LOCATION: 49..1341
(D) OTHER INFORMATION:/standard_name= "hOP1"

(xi) SEQUENCE DESCRIPTION: SEQ ID NO:16:

GGTGCGGGCC CGGAGCCCGG AGCCCGGGTA GCGCGTAGAG CCGGCGCG ATG CAC GTG 57
Met His Val
1

CGC TCA CTG CGA GCT GCG GCG CCG CAC AGC TTC GTG GCG CTC TGG GCA 105
Arg Ser Leu Arg Ala Ala Ala Pro His Ser Phe Val Ala Leu Trp Ala
    5                   10                  15

CCC CTG TTC CTG CTG CGC TCC GCC CTG GCC GAC TTC AGC CTG GAC AAC 153
Pro Leu Phe Leu Leu Arg Ser Ala Leu Ala Asp Phe Ser Leu Asp Asn
20                  25                  30                  35

GAG GTG CAC TCG AGC TTC ATC CAC CGG CGC CTC CGC AGC CAG GAG CGG 201
Glu Val His Ser Ser Phe Ile His Arg Arg Leu Arg Ser Gln Glu Arg
                40                  45                  50

CGG GAG ATG CAG CGC GAG ATC CTC TCC ATT TTG GGC TTG CCC CAC CGC 249
Arg Glu Met Gln Arg Glu Ile Leu Ser Ile Leu Gly Leu Pro His Arg
            55                  60                  65

CCG CGC CCG CAC CTC CAG GGC AAG CAC AAC TCG GCA CCC ATG TTC ATG 297
Pro Arg Pro His Leu Gln Gly Lys His Asn Ser Ala Pro Met Phe Met
        70                  75                  80

CTG GAC CTG TAC AAC GCC ATG GCG GTG GAG GAG GGC GGC GGG CCC GGC 345
Leu Asp Leu Tyr Asn Ala Met Ala Val Glu Glu Gly Gly Gly Pro Gly
    85                  90                  95

GGC CAG GGC TTC TCC TAC CCC TAC AAG GCC GTC TTC AGT ACC CAG GGC 393
Gly Gln Gly Phe Ser Tyr Pro Tyr Lys Ala Val Phe Ser Thr Gln Gly
100                 105                 110                 115

CCC CCT CTG GCC AGC CTG CAA GAT AGC CAT TTC CTC ACC GAC GCC GAC 441
Pro Pro Leu Ala Ser Leu Gln Asp Ser His Phe Leu Thr Asp Ala Asp
                120                 125                 130

ATG GTC ATG AGC TTC GTC AAC CTC GTG GAA CAT GAC AAG GAA TTC TTC 489
Met Val Met Ser Phe Val Asn Leu Val Glu His Asp Lys Glu Phe Phe
            135                 140                 145

CAC CCA CGC TAC CAC CAT CGA GAG TTC CGG TTT GAT CTT TCC AAG ATC 537
His Pro Arg Tyr His His Arg Glu Phe Arg Phe Asp Leu Ser Lys Ile
        150                 155                 160

CCA GAA GGG GAA GCT GTC ACG GCA GCC GAA TTC CGG ATC TAC AAG GAC 585
Pro Glu Gly Glu Ala Val Thr Ala Ala Glu Phe Arg Ile Tyr Lys Asp
    165                 170                 175

TAC ATC CGG GAA CGC TTC GAC AAT GAG ACG TTC CGG ATC AGC GTT TAT 633
Tyr Ile Arg Glu Arg Phe Asp Asn Glu Thr Phe Arg Ile Ser Val Tyr
180                 185                 190                 195

CAG GTG CTC CAG GAG CAC TTG GGC AGG GAA TCG GAT CTC TTC CTG CTC 681
Gln Val Leu Gln Glu His Leu Gly Arg Glu Ser Asp Leu Phe Leu Leu
                200                 205                 210

GAC AGC CGT ACC CTC TGG GCC TCG GAG GAG GGC TGG CTG GTG TTT GAC 729
Asp Ser Arg Thr Leu Trp Ala Ser Glu Glu Gly Trp Leu Val Phe Asp
            215                 220                 225

ATC ACA GCC ACC AGC AAC CAC TGG GTG GTC AAT CCG CGG CAC AAC CTG 777
Ile Thr Ala Thr Ser Asn His Trp Val Val Asn Pro Arg His Asn Leu
        230                 235                 240

GGC CTG CAG CTC TCG GTG GAG ACG CTG GAT GGG CAG AGC ATC AAC CCC 825
Gly Leu Gln Leu Ser Val Glu Thr Leu Asp Gly Gln Ser Ile Asn Pro
    245                 250                 255

AAG TTG GCG GGC CTG ATT GGG CGG CAC GGG CCC CAG AAC AAG CAG CCC 873
Lys Leu Ala Gly Leu Ile Gly Arg His Gly Pro Gln Asn Lys Gln Pro
260                 265                 270                 275

TTC ATG GTG GCT TTC TTC AAG GCC ACG GAG GTC CAC TTC CGC AGC ATC 921
Phe Met Val Ala Phe Phe Lys Ala Thr Glu Val His Phe Arg Ser Ile
                280                 285                 290

CGG TCC ACG GGG AGC AAA CAG CGC AGC CAG AAC CGC TCC AAG ACG CCC 969
Arg Ser Thr Gly Ser Lys Gln Arg Ser Gln Asn Arg Ser Lys Thr Pro
            295                 300                 305

AAG AAC CAG GAA GCC CTG CGG ATG GCC AAC GTG GCA GAG AAC AGC AGC 1017
Lys Asn Gln Glu Ala Leu Arg Met Ala Asn Val Ala Glu Asn Ser Ser
        310                 315                 320

AGC GAC CAG AGG CAG GCC TGT AAG AAG CAC GAG CTG TAT GTC AGC TTC 1065
Ser Asp Gln Arg Gln Ala Cys Lys Lys His Glu Leu Tyr Val Ser Phe
    325                 330                 335

CGA GAC CTG GGC TGG CAG GAC TGG ATC ATC GCG CCT GAA GGC TAC GCC 1113
Arg Asp Leu Gly Trp Gln Asp Trp Ile Ile Ala Pro Glu Gly Tyr Ala
340                 345                 350                 355

GCC TAC TAC TGT GAG GGG GAG TGT GCC TTC CCT CTG AAC TCC TAC ATG 1161
Ala Tyr Tyr Cys Glu Gly Glu Cys Ala Phe Pro Leu Asn Ser Tyr Met
                360                 365                 370

AAC GCC ACC AAC CAC GCC ATC GTG CAG ACG CTG GTC CAC TTC ATC AAC 1209
Asn Ala Thr Asn His Ala Ile Val Gln Thr Leu Val His Phe Ile Asn
            375                 380                 385

CCG GAA ACG GTG CCC AAG CCC TGC TGT GCG CCC ACG CAG CTC AAT GCC 1257
Pro Glu Thr Val Pro Lys Pro Cys Cys Ala Pro Thr Gln Leu Asn Ala
        390                 395                 400

ATC TCC GTC CTC TAC TTC GAT GAC AGC TCC AAC GTC ATC CTG AAG AAA 1305
Ile Ser Val Leu Tyr Phe Asp Asp Ser Ser Asn Val Ile Leu Lys Lys
    405                 410                 415

TAC AGA AAC ATG GTG GTC CGG GCC TGT GGC TGC CAC TAGCTCCTCC 1351
Tyr Arg Asn Met Val Val Arg Ala Cys Gly Cys His
420                 425                 430

GAGAATTCAG ACCCTTTGGG GCCAAGTTTT TCTGGATCCT CCATTGCTCG CCTTGGCCAG 1411

GAACCAGCAG ACCAACTGCC TTTTGTGAGA CCTTCCCCTC CCTATCCCCA ACTTTAAAGG 1471

TGTGAGAGTA TTAGGAAACA TGAGCAGCAT ATGGCTTTTG ATCAGTTTTT CAGTGGCAGC 1531

ATCCAATGAA CAAGATCCTA CAAGCTGTGC AGGCAAAACC TAGCAGGAAA AAAAAACAAC 1591

GCATAAAGAA AAATGGCCGG GCCAGGTCAT TGGCTGGGAA GTCTCAGCCA TGCACGGACT 1651

CGTTTCCAGA GGTAATTATG AGCGCCTACC AGCCAGGCCA CCCAGCCGTG GGAGGAAGGG 1711

GGCGTGGCAA GGGGTGGGCA CATTGGTGTC TGTGCGAAAG GAAAATTGAC CCGGAAGTTC 1771

CTGTAATAAA TGTCACAATA AAACGAATGA ATGAAAAAAA AAAAAAAAAA A 1822

(2) INFORMATION FOR SEQ ID NO:17:

(i) SEQUENCE CHARACTERISTICS:
(A) LENGTH: 431 amino acids
(B) TYPE: amino acid
(D) TOPOLOGY: linear

(ii) MOLECULE TYPE: protein

(ix) FEATURE:
(D) OTHER INFORMATION: /Product="OP1-PP"

(xi) SEQUENCE DESCRIPTION: SEQ ID NO:17:

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

(2) INFORMATION FOR SEQ ID NO:18:

(i) SEQUENCE CHARACTERISTICS:
(A) LENGTH: 1873 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: MURIDAE
(F) TISSUE TYPE: EMBRYO

(ix) FEATURE:
(A) NAME/KEY: CDS
(B) LOCATION: 104..1393
(D) OTHER INFORMATION: /note= "MOP1 (CDNA)"

(xi) SEQUENCE DESCRIPTION: SEQ ID NO:18:

CTGCAGCAAG TGACCTCGGG TCGTGGACCG CTGCCCTGCC CCCTCCGCTG CCACCTGGGG 60

CGGCGCGGGC CCGGTGCCCC GGATCGCGCG TAGAGCCGGC GCG ATG CAC GTG CGC 115
Met His Val Arg
1

TCG CTG CGC GCT GCG GCG CCA CAC AGC TTC GTG GCG CTC TGG GCG CCT 163
Ser Leu Arg Ala Ala Ala Pro His Ser Phe Val Ala Leu Trp Ala Pro
5                   10                  15                  20

CTG TTC TTG CTG CGC TCC GCC CTG GCC GAT TTC AGC CTG GAC AAC GAG 211
Leu Phe Leu Leu Arg Ser Ala Leu Ala Asp Phe Ser Leu Asp Asn Glu
                25                  30                  35

GTG CAC TCC AGC TTC ATC CAC CGG CGC CTC CGC AGC CAG GAG CGG CGG 259
Val His Ser Ser Phe Ile His Arg Arg Leu Arg Ser Gln Glu Arg Arg
            40                  45                  50

GAG ATG CAG CGG GAG ATC CTG TCC ATC TTA GGG TTG CCC CAT CGC CCG 307
Glu Met Gln Arg Glu Ile Leu Ser Ile Leu Gly Leu Pro His Arg Pro
        55                  60                  65

CGC CCG CAC CTC CAG GGA AAG CAT AAT TCG GCG CCC ATG TTC ATG TTG 355
Arg Pro His Leu Gln Gly Lys His Asn Ser Ala Pro Met Phe Met Leu
    70                  75                  80

GAC CTG TAC AAC GCC ATG GCG GTG GAG GAG AGC GGG CCG GAC GGA CAG 403
Asp Leu Tyr Asn Ala Met Ala Val Glu Glu Ser Gly Pro Asp Gly Gln
85                  90                  95                  100

GGC TTC TCC TAC CCC TAC AAG GCC GTC TTC AGT ACC CAG GGC CCC CCT 451
Gly Phe Ser Tyr Pro Tyr Lys Ala Val Phe Ser Thr Gln Gly Pro Pro
                105                 110                 115

TTA GCC AGC CTG CAG GAC AGC CAT TTC CTC ACT GAC GCC GAC ATG GTC 499
Leu Ala Ser Leu Gln Asp Ser His Phe Leu Thr Asp Ala Asp Met Val
            120                 125                 130

ATG AGC TTC GTC AAC CTA GTG GAA CAT GAC AAA GAA TTC TTC CAC CCT 547
Met Ser Phe Val Asn Leu Val Glu His Asp Lys Glu Phe Phe His Pro
        135                 140                 145
CGA TAC CAC CAT CGG GAG TTC CGG TTT GAT CTT TCC AAG ATC CCC GAG 595
Arg Tyr His His Arg Glu Phe Arg Phe Asp Leu Ser Lys Ile Pro Glu
    150                 155                 160

GGC GAA CGG GTG ACC GCA GCC GAA TTC AGG ATC TAT AAG GAC TAC ATC 643
Gly Glu Arg Val Thr Ala Ala Glu Phe Arg Ile Tyr Lys Asp Tyr Ile
165                 170                 175                 180

CGG GAG CGA TTT GAC AAC GAG ACC TTC CAG ATC ACA GTC TAT CAG GTG 691
Arg Glu Arg Phe Asp Asn Glu Thr Phe Gln Ile Thr Val Tyr Gln Val
                185                 190                 195

CTC CAG GAG CAC TCA GGC AGG GAG TCG GAC CTC TTC TTG CTG GAC AGC 739
Leu Gln Glu His Ser Gly Arg Glu Ser Asp Leu Phe Leu Leu Asp Ser
            200                 205                 210

CGC ACC ATC TGG GCT TCT GAG GAG GGC TGG TTG GTG TTT GAT ATC ACA 787
Arg Thr Ile Trp Ala Ser Glu Glu Gly Trp Leu Val Phe Asp Ile Thr
        215                 220                 225

GCC ACC AGC AAC CAC TGG GTG GTC AAC CCT CGG CAC AAC CTG GGC TTA 835
Ala Thr Ser Asn His Trp Val Val Asn Pro Arg His Asn Leu Gly Leu
    230                 235                 240

CAG CTC TCT GTG GAG ACC CTG GAT GGG CAG AGC ATC AAC CCC AAG TTG 883
Gln Leu Ser Val Glu Thr Leu Asp Gly Gln Ser Ile Asn Pro Lys Leu
245                 250                 255                 260

GCA GGC CTG ATT GGA CGG CAT GGA CCC CAG AAC AAG CAA CCC TTC ATG 931
Ala Gly Leu Ile Gly Arg His Gly Pro Gln Asn Lys Gln Pro Phe Met
                265                 270                 275

GTG GCC TTC TTC AAG GCC ACG GAA GTC CAT CTC CGT AGT ATC CGG TCC 979
Val Ala Phe Phe Lys Ala Thr Glu Val His Leu Arg Ser Ile Arg Ser
            280                 285                 290

ACG GGG GGC AAG CAG CGC AGC CAG AAT CGC TCC AAG ACG CCA AAG AAC 1027
Thr Gly Gly Lys Gln Arg Ser Gln Asn Arg Ser Lys Thr Pro Lys Asn
        295                 300                 305

CAA GAG GCC CTG AGG ATG GCC AGT GTG GCA GAA AAC AGC AGC AGT GAC 1075
Gln Glu Ala Leu Arg Met Ala Ser Val Ala Glu Asn Ser Ser Ser Asp
    310                 315                 320

CAG AGG CAG GCC TGC AAG AAA CAT GAG CTG TAC GTC AGC TTC CGA GAC 1123
Gln Arg Gln Ala Cys Lys Lys His Glu Leu Tyr Val Ser Phe Arg Asp
325                 330                 335                 340

CTT GGC TGG CAG GAC TGG ATC ATT GCA CCT GAA GGC TAT GCT GCC TAC 1171
Leu Gly Trp Gln Asp Trp Ile Ile Ala Pro Glu Gly Tyr Ala Ala Tyr
                345                 350                 355

TAC TGT GAG GGA GAG TGC GCC TTC CCT CTG AAC TCC TAC ATG AAC GCC 1219
Tyr Cys Glu Gly Glu Cys Ala Phe Pro Leu Asn Ser Tyr Met Asn Ala
            360                 365                 370

ACC AAC CAC GCC ATC GTC CAG ACA CTG GTT CAC TTC ATC AAC CCA GAC 1267
Thr Asn His Ala Ile Val Gln Thr Leu Val His Phe Ile Asn Pro Asp
        375                 380                 385

ACA GTA CCC AAG CCC TGC TGT GCG CCC ACC CAG CTC AAC GCC ATC TCT 1315
Thr Val Pro Lys Pro Cys Cys Ala Pro Thr Gln Leu Asn Ala Ile Ser
    390                 395                 400

GTC CTC TAC TTC GAC GAC AGC TCT AAT GTC ATC CTG AAG AAG TAC AGA 1363
Val Leu Tyr Phe Asp Asp Ser Ser Asn Val Ile Leu Lys Lys Tyr Arg
405                 410                 415                 420

AAC ATG GTG GTC CGG GCC TGT GGC TGC CAC TAGCTCTTCC TGAGACCCTG 1413
Asn Met Val Val Arg Ala Cys Gly Cys His
                425                 430

ACCTTTGCGG GGCCACACCT TTCCAAATCT TCGATGTCTC ACCATCTAAG TCTCTCACTG 1473

CCCACCTTGG CGAGGAGAAC AGACCAACCT CTCCTGAGCC TTCCCTCACC TCCCAACCGG 1533

AAGCATGTAA GGGTTCCAGA AACCTGAGCG TGCAGCAGCT GATGAGCGCC CTTTCCTTCT 1593

GGCACGTGAC GGACAAGATC CTACCAGCTA CCACAGCAAA CGCCTAAGAG CAGGAAAAAT 1653

GTCTGCCAGG AAAGTGTCCA GTGTCCACAT GGCCCCTGGC GCTCTGAGTC TTTGAGGAGT 1713

AATCGCAAGC CTCGTTCAGC TGCAGCAGAA GGAAGGGCTT AGCCAGGGTG GGCGCTGGCG 1773

TCTGTGTTGA AGGGAAACCA AGCAGAAGCC ACTGTAATGA TATGTCACAA TAAAACCCAT 1833

GAATGAAAAA AAAAAAAAAA AAAAAAAAAA AAAAGAATTC 1873

20) INFORMATION FOR SEQ ID NO:19:

(i) SEQUENCE CHARACTERISTICS:
(A) LENGTH: 430 amino acids
(B) TYPE: amino acid
(D) TOPOLOGY: linear

(ii) MOLECULE TYPE: protein

(ix) FEATURE:
(D) OTHER INFORMATION: /product= "mOP1-PP"

(xi) SEQUENCE DESCRIPTION: SEQ ID NO:19:

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Ser Gly
                85                  90                  95

Pro Asp Gly Gln Gly Phe Ser Tyr Pro Tyr Lys Ala Val Phe Ser Thr
            100                 105                 110

Gln Gly Pro Pro Leu Ala Ser Leu Gln Asp Ser His Phe Leu Thr Asp
        115                 120                 125

Ala Asp Met Val Met Ser Phe Val Asn Leu Val Glu His Asp Lys Glu
    130                 135                 140

Phe Phe His Pro Arg Tyr His His Arg Glu Phe Arg Phe Asp Leu Ser
145                 150                 155                 160

Lys Ile Pro Glu Gly Glu Arg Val Thr Ala Ala Glu Phe Arg Ile Tyr
                165                 170                 175

Lys Asp Tyr Ile Arg Glu Arg Phe Asp Asn Glu Thr Phe Gln Ile Thr
            180                 185                 190

Val Tyr Gln Val Leu Gln Glu His Ser Gly Arg Glu Ser Asp Leu Phe
        195                 200                 205

Leu Leu Asp Ser Arg Thr Ile Trp Ala Ser Glu Glu Gly Trp Leu Val
    210                 215                 220

Phe Asp Ile Thr Ala Thr Ser Asn His Trp Val Val Asn Pro Arg His
225                 230                 235                 240

Asn Leu Gly Leu Gln Leu Ser Val Glu Thr Leu Asp Gly Gln Ser Ile
                245                 250                 255

Asn Pro Lys Leu Ala Gly Leu Ile Gly Arg His Gly Pro Gln Asn Lys
            260                 265                 270

Gln Pro Phe Met Val Ala Phe Phe Lys Ala Thr Glu Val His Leu Arg
        275                 280                 285

Ser Ile Arg Ser Thr Gly Gly Lys Gln Arg Ser Gln Asn Arg Ser Lys
    290                 295                 300

Thr Pro Lys Asn Gln Glu Ala Leu Arg Met Ala Ser Val Ala Glu Asn
305                 310                 315                 320

Ser Ser Ser Asp Gln Arg Gln Ala Cys Lys Lys His Glu Leu Tyr Val
                325                 330                 335

Ser Phe Arg Asp Leu Gly Trp Gln Asp Trp Ile Ile Ala Pro Glu Gly
            340                 345                 350

Tyr Ala Ala Tyr Tyr Cys Glu Gly Glu Cys Ala Phe Pro Leu Asn Ser
        355                 360                 365

Tyr Met Asn Ala Thr Asn His Ala Ile Val Gln Thr Leu Val His Phe
    370                 375                 380

Ile Asn Pro Asp Thr Val Pro Lys Pro Cys Cys Ala Pro Thr Gln Leu
385                 390                 395                 400

Asn Ala Ile Ser Val Leu Tyr Phe Asp Asp Ser Ser Asn Val Ile Leu
                405                 410                 415

Lys Lys Tyr Arg Asn Met Val Val Arg Ala Cys Gly Cys His
            420                 425                 430

(2) INFORMATION FOR SEQ ID NO:20:

(i)SEQUENCE CHARACTERISTICS:
(A) LENGTH: 1723 base pairs
(B) TYPE: nucleic acid
(C) STRANDEDNESS: single
(D) TOPOLOGY: linear

(ii)MOLECULE TYPE: cDNA

(vi)ORIGINAL SOURCE:
(A) ORGANISM: Homo sapiens
(F) TISSUE TYPE: HIPPOCAMPUS

(ix)FEATURE:
(A) NAME/KEY: CDS
(B) LOCATION: 490..1696
(D) OTHER INFORMATION: /note= "hOP2 (cDNA)"

(xi)SEQUENCE DESCRIPTION: SEQ ID NO:20:
GGCGCCGGCA GAGCAGGAGT GGCTGGAGGA GCTGTGGTTG GAGCAGGAGG TGGCACGGCA 60
GGGCTGGAGG GCTCCCTATG AGTGGCGGAG ACGGCCCAGG AGGCGCTGGA GCAACAGCTC 120
CCACACCGCA CCAAGCGGTG GCTGCAGGAG CTCGCCCATC GCCCCTGCGC TGCTCGGACC 180
GCGGCCACAG CCGGACTGGC GGGTACGGCG GCGACAGAGG CATTGGCCGA GAGTCCCAGT 240
CCGCAGAGTA GCCCCGGCCT CGAGGCGGTG GCGTCCCGGT CCTCTCCGTC CAGGAGCCAG 300
GACAGGTGTC GCGCGGCGGG GCTCCAGGGA CCGCGCCTGA GGCCGGCTGC CCGCCCGTCC 360
CGCCCCGCCC CGCCGCCCGC CGCCCGCCGA GCCCAGCCTC CTTGCCGTCG GGGCGTCCCC 420
AGGCCCTGGG TCGGCCGCGG AGCCGATGCG CGCCCGCTGA GCGCCCCAGC TGAGCGCCCC 480
CGGCCTGCC ATG ACC GCG CTC CCC GGC CCG CTC TGG CTC CTG GGC CTG 528
GCG CTA TGC GCG CTG GGC GGG GGC GGC CCC GGC CTG CGA CCC CCG CCC 576
GGC TGT CCC CAG CGA CGT CTG GGC GCG CGC GAG CGC CGG GAC GTG CAG 624
CGC GAG ATC CTG GCG GTG CTC GGG CTG CCT GGG CGG CCC CGG CCC CGC 672
GCG CCA CCC GCC GCC TCC CGG CTG CCC GCG TCC GCG CCG CTC TTC ATG 720
CTG GAC CTG TAC CAC GCC ATG GCC GGC GAC GAC GAC GAG GAC GGC GCG 768
CCC GCG GAG CGG CGC CTG GGC CGC GCC GAC CTG GTC ATG AGC TTC GTT 816
AAC ATG GTG GAG CGA GAC CGT GCC CTG GGC CAC CAG GAG CCC CAT TGG 864
AAG GAG TTC CGC TTT GAC CTG ACC CAG ATC CCG GCT GGG GAG GCG GTC 912
ACA GCT GCG GAG TTC CGG ATT TAC AAG GTG CCC AGC ATC CAC CTG CTC 960
AAC AGG ACC CTC CAC GTC AGC ATG TTC CAG GTG GTC CAG GAG CAG TCC 1008
AAC AGG GAG TCT GAC TTG TTC TTT TTG GAT CTT CAG ACG CTC CGA GCT 1056
GGA GAC GAG GGC TGG CTG GTG CTG GAT GTC ACA GCA GCC AGT GAC TGC 1104
TGG TTG CTG AAG CGT CAC AAG GAC CTG GGA CTC CGC CTC TAT GTG GAG 1152
ACT GAG GAC GGG CAC AGC GTG GAT CCT GGC CTG GCC GGC CTG CTG GGT 1200
CAA CGG GCC CCA CGC TCC CAA CAG CCT TTC GTG GTC ACT TTC TTC AGG 1248
GCC AGT CCG AGT CCC ATC CGC ACC CCT CGG GCA GTG AGG CCA CTG AGG 1296
AGG AGG CAG CCG AAG AAA AGC AAC GAG CTG CCG CAG GCC AAC CGA CTC 1344
CCA GGG ATC TTT GAT GAC GTC CAC GGC TCC CAC GGC CGG CAG GTC TGC 1392
CGT CGG CAC GAG CTC TAC GTC AGC TTC CAG GAC CTC GGC TGG CTG GAC 1440
TGG GTC ATC GCT CCC CAA GGC TAC TCG GCC TAT TAC TGT GAG GGG GAG 1488
TGC TCC TTC CCA CTG GAC TCC TGC ATG AAT GCC ACC AAC CAC GCC ATC 1536
CTG CAG TCC CTG GTG CAC CTG ATG AAG CCA AAC GCA GTC CCC AAG GCG 1584
TGC TGT GCA CCC ACC AAG CTG AGC GCC ACC TCT GTG CTC TAC TAT GAC 1632
AGC AGC AAC AAC GTC ATC CTG CGC AAA CAC CGC AAC ATG GTG GTC AAG 1680
GCC TGC GGC TGC CAC T GAGTCAGCCC GCCCAGCCCT ACTGCAG 1723

(2) INFORMATION FOR SEQ ID NO:21:

(i)SEQUENCE CHARACTERISTICS:
(A) LENGTH: 402 amino acids
(B) TYPE: amino acid
(D) TOPOLOGY: linear

(ii)MOLECULE TYPE: protein

(ix)FEATURE:
(A)OTHER INFORMATION: /product= "hOP2-PP"

(xi)SEQUENCE DESCRIPTION: SEQ ID NO:21:

(2) INFORMATION FOR SEQ ID NO:22:

(i) SEQUENCE CHARACTERISTICS:
(A) LENGTH: 1926 base pairs
(B) TYPE: nucleic acid
(C) STRANDEDNESS: single
(D TOPOLOGY: linear

(ii) MOLECULE TYPE: cDNA

(vi) ORIGINAL SOURCE:
(A) ORGANISM: MURIDAE
(F) TISSUE TYPE: EMBRYO

(ix) FEATURE:
(A) NAME/KEY: CDS
(B) LOCATION: 93..1289
(D) OTHER INFORMATION: /note= "mOP2 cDNA"

(xi) SEQUENCE DESCRIPTION: SEQ ID NO:22:

GCCAGGCACA GGTGCGCCGT CTGGTCCTCC CCGTCTGGCG TCAGCCGAGC 50

CCGACCAGCT ACCAGTGGAT GCGCGCCGGC TGAAAGTCCG AG ATG GCT ATG CGT 104
Met Ala Met Arg
1

CCC GGG CCA CTC TGG CTA TTG GGC CTT GCT CTG TGC GCG CTG GGA GGC 152
Pro Gly Pro Leu Trp Leu Leu Gly Leu Ala Leu Cys Ala Leu Gly Gly
5                   10                  15                  20

GGC CAC GGT CCG CGT CCC CCG CAC ACC TGT CCC CAG CGT CGC CTG GGA 200
Gly His Gly Pro Arg Pro Pro His Thr Cys Pro Gln Arg Arg Leu Gly
                25                  30                  35

GCG CGC GAG CGC CGC GAC ATG CAG CGT GAA ATC CTG GCG GTG CTC GGG 248
Ala Arg Glu Arg Arg Asp Met Gln Arg Glu Ile Leu Ala Val Leu Gly
            40                  45                  50

CTA CCG GGA CGG CCC CGA CCC CGT GCA CAA CCC GCG GCT GCC CGG CAG 296
Leu Pro Gly Arg Pro Arg Pro Arg Ala Gln Pro Ala Ala Ala Arg Gln
        55                  60                  65

CCA GCG TCC GCG CCC CTC TTC ATG TTG GAC CTA TAC CAC GCC ATG ACC 344
Pro Ala Ser Ala Pro Leu Phe Met Leu Asp Leu Tyr His Ala Met Thr
    70                  75                  80

GAT GAC GAC GAC GGC GGG CCA CCA CAG GCT CAC TTA GGC CGT GCC GAC 392
Asp Asp Asp Asp Gly Gly Pro Pro Gln Ala His Leu Gly Arg Ala Asp
85                  90                  95                  100

CTG GTC ATG AGC TTC GTC AAC ATG GTG GAA CGC GAC CGT ACC CTG GGC 440
Leu Val Met Ser Phe Val Asn Met Val Glu Arg Asp Arg Thr Leu Gly
                105                 110                 115

TAC CAG GAG CCA CAC TGG AAG GAA TTC CAC TTT GAC CTA ACC CAG ATC 488
Tyr Gln Glu Pro His Trp Lys Glu Phe His Phe Asp Leu Thr Gln Ile
            120                 125                 130

CCT GCT GGG GAG GCT GTC ACA GCT GCT GAG TTC CGG ATC TAC AAA GAA 536
Pro Ala Gly Glu Ala Val Thr Ala Ala Glu Phe Arg Ile Tyr Lys Glu
        135                 140                 145

CCC AGC ACC CAC CCG CTC AAC ACA ACC CTC CAC ATC AGC ATG TTC GAA 584
Pro Ser Thr His Pro Leu Asn Thr Thr Leu His Ile Ser Met Phe Glu
    150                 155                 160

GTG GTC CAA GAG CAC TCC AAC AGG GAG TCT GAC TTG TTC TTT TTG GAT 632
Val Val Gln Glu His Ser Asn Arg Glu Ser Asp Leu Phe Phe Leu Asp
165                 170                 175                 180

CTT CAG ACG CTC CGA TCT GGG GAC GAG GGC TGG CTG GTG CTG GAC ATC 680
Leu Gln Thr Leu Arg Ser Gly Asp Glu Gly Trp Leu Val Leu Asp Ile
                185                 190                 195

ACA GCA GCC AGT GAC CGA TGG CTG CTG AAC CAT CAC AAG GAC CTG GGA 728
Thr Ala Ala Ser Asp Arg Trp Leu Leu Asn His His Lys Asp Leu Gly
            200                 205                 210

CTC CGC CTC TAT GTG GAA ACC GCG GAT GGG CAC AGC ATG GAT CCT GGC 776
Leu Arg Leu Tyr Val Glu Thr Ala Asp Gly His Ser Met Asp Pro Gly
        215                 220                 225

CTG GCT GGT CTG CTT GGA CGA CAA GCA CCA CGC TCC AGA CAG CCT TTC 824
Leu Ala Gly Leu Leu Gly Arg Gln Ala Pro Arg Ser Arg Gln Pro Phe
    230                 235                 240

ATG GTA ACC TTC TTC AGG GCC AGC CAG AGT CCT GTG CGG GCC CCT CGG 872
Met Val Thr Phe Phe Arg Ala Ser Gln Ser Pro Val Arg Ala Pro Arg
245                 250                 255                 260

GCA GCG AGA CCA CTG AAG AGG AGG CAG CCA AAG AAA ACG AAC GAG CTT 920
Ala Ala Arg Pro Leu Lys Arg Arg Gln Pro Lys Lys Thr Asn Glu Leu
                265                 270                 275

CCG CAC CCC AAC AAA CTC CCA GGG ATC TTT GAT GAT GGC CAC GGT TCC 968
Pro His Pro Asn Lys Leu Pro Gly Ile Phe Asp Asp Gly His Gly Ser
            280                 285                 290

CGC GGC AGA GAG GTT TGC CGC AGG CAT GAG CTC TAC GTC AGC TTC CGT 1016
Arg Gly Arg Glu Val Cys Arg Arg His Glu Leu Tyr Val Ser Phe Arg
        295                 300                 305

GAC CTT GGC TGG CTG GAC TGG GTC ATC GCC CCC CAG GGC TAC TCT GCC 1064
Asp Leu Gly Trp Leu Asp Trp Val Ile Ala Pro Gln Gly Tyr Ser Ala
    310                 315                 320

TAT TAC TGT GAG GGG GAG TGT GCT TTC CCA CTG GAC TCC TGT ATG AAC 1112
Tyr Tyr Cys Glu Gly Glu Cys Ala Phe Pro Leu Asp Ser Cys Met Asn
325                 330                 335                 340

GCC ACC AAC CAT GCC ATC TTG CAG TCT CTG GTG CAC CTG ATG AAG CCA 1160
Ala Thr Asn His Ala Ile Leu Gln Ser Leu Val His Leu Met Lys Pro
                345                 350                 355

GAT GTT GTC CCC AAG GCA TGC TGT GCA CCC ACC AAA CTG AGT GCC ACC 1208
Asp Val Val Pro Lys Ala Cys Cys Ala Pro Thr Lys Leu Ser Ala Thr
            360                 365                 370

TCT GTG CTG TAC TAT GAC AGC AGC AAC AAT GTC ATC CTG CGT AAA CAC 1256
Ser Val Leu Tyr Tyr Asp Ser Ser Asn Asn Val Ile Leu Arg Lys His
        375                 380                 385

CGT AAC ATG GTG GTC AAG GCC TGT GGC TGC CAC TGAGGCCCCG CCCAGCATCC 1309
Arg Asn Met Val Val Lys Ala Cys Gly Cys His
    390                 395

TGCTTCTACT ACCTTACCAT CTGGCCGGGC CCCTCTCCAG AGGCAGAAAC CCTTCTATGT 1369

TATCATAGCT CAGACAGGGG CAATGGGAGG CCCTTCACTT CCCCTGGCCA CTTCCTGCTA 1429

AAATTCTGGT CTTTCCCAGT TCCTCTGTCC TTCATGGGGT TTCGGGGCTA TCACCCCGCC 1489

CTCTCCATCC TCCTACCCCA AGCATAGACT GAATGCACAC AGCATCCCAG AGCTATGCTA 1549

ACTGAGAGGT CTGGGGTCAG CACTGAAGGC CCACATGAGG AAGACTGATC CTTGGCCATC 1609

CTCAGCCCAC AATGGCAAAT TCTGGATGGT CTAAGAAGGC CGTGGAATTC TAAACTAGAT 1669

GATCTGGGCT CTCTGCACCA TTCATTGTGG CAGTTGGGAC ATTTTTAGGT ATAACAGACA 1729

CATACACTTA GATCAATGCA TCGCTGTACT CCTTGAAATC AGAGCTAGCT TGTTAGAAAA 1789

AGAATCAGAG CCAGGTATAG CGGTGCATGT CATTAATCCC AGCGCTAAAG AGACAGAGAC 1849

AGGAGAATCT CTGTGAGTTC AAGGCCACAT AGAAAGAGCC TGTCTCGGGA GCAGGAAAAA 1909

AAAAAAAAAC GGAATTC 1926

(2) INFORMATION FOR SEQ ID NO:23:

(i) SEQUENCE CHARACTERISTICS:
(A) LENGTH: 399 amino acids
(B) TYPE: amino acid
(D) TOPOLOGY: linear

(ii) MOLECULE TYPE: protein

(ix) FEATURE:
(D) OTHER INFORMATION: /product= "mOP2-PP"

(xi) SEQUENCE DESCRIPTION: SEQ ID NO:23:

Met Ala Met Arg Pro Gly Pro Leu Trp Leu Leu Gly Leu Ala Leu Cys
1               5                   10                  15

Ala Leu Gly Gly Gly His Gly Pro Arg Pro Pro His Thr Cys Pro Gln
            20                  25                  30

Arg Arg Leu Gly Ala Arg Glu Arg Arg Asp Met Gln Arg Glu Ile Leu Ala
        35                  40                  45

Val Leu Gly Leu Pro Gly Arg Pro Arg Pro Arg Ala Gln Pro Ala Ala
50                  55                  60                  65

Ala Arg Gln Pro Ala Ser Ala Pro Leu Phe Met Leu Asp Leu Tyr His Ala
                70                  75                  80

Met Thr Asp Asp Asp Asp Gly Gly Pro Pro Gln Ala His Leu Gly Arg
        85                  90                  95

Ala Asp Leu Val Met Ser Phe Val Asn Met Val Glu Arg Asp Arg Thr
    100                 105                 110

Leu Gly Tyr Gln Glu Pro His Trp Lys Glu Phe His Phe Asp Leu Thr
115                 120                 125                 130

Gln Ile Pro Ala Gly Glu Ala Val Thr Ala Ala Glu Phe Arg Ile Tyr
                135                 140                 145

Lys Glu Pro Ser Thr His Pro Leu Asn Thr Thr Leu His Ile Ser Met
            150                 155                 160

Phe Glu Val Val Gln Glu His Ser Asn Arg Glu Ser Asp Leu Phe Phe
        165                 170                 175

Leu Asp Leu Gln Thr Leu Arg Ser Gly Asp Glu Gly Trp Leu Val Leu
    180                 185                 190

Asp Ile Thr Ala Ala Ser Asp Arg Trp Leu Leu Asn His His Lys Asp
195                 200                 205                 210

Leu Gly Leu Arg Leu Tyr Val Glu Thr Ala Asp Gly His Ser Met Asp
                215                 220                 225

Pro Gly Leu Ala Gly Leu Leu Gly Arg Gln Ala Pro Arg Ser Arg Gln
            230                 235                 240

Pro Phe Met Val Thr Phe Phe Arg Ala Ser Gln Ser Pro Val Arg Ala
        245                 250                 255

Pro Arg Ala Ala Arg Pro Leu Lys Arg Arg Gln Pro Lys Lys Thr Asn
    260                 265                 270

Glu Leu Pro His Pro Asn Lys Leu Pro Gly Ile Phe Asp Asp Gly His
275                 280                 285                 290

Gly Ser Arg Gly Arg Glu Val Cys Arg Arg His Glu Leu Tyr Val Ser
                295                 300                 305

Phe Arg Asp Leu Gly Trp Leu Asp Trp Val Ile Ala Pro Gln Gly Tyr
            310                 315                 320

Ser Ala Tyr Tyr Cys Glu Gly Glu Cys Ala Phe Pro Leu Asp Ser Cys
        325                 330                 335

Met Asn Ala Thr Asn His Ala Ile Leu Gln Ser Leu Val His Leu Met
    340                 345                 350

Lys Pro Asp Val Val Pro Lys Ala Cys Cys Ala Pro Thr Lys Leu Ser
355                 360                 365                 370

Ala Thr Ser Val Leu Tyr Tyr Asp Ser Ser Asn Asn Val Ile Leu Arg
                375                 380                 385

Lys His Arg Asn Met Val Val Lys Ala Cys Gly Cys His
            390                 395

(2)INFORMATION FOR SEQ ID NO:24:

(i) SEQUENCE CHARACTERISTICS:
(A) LENGTH: 1368 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..1368
(D) OTHER INFORMATION:/STANDARD NAME="60A"

(x) PUBLICATION INFORMATION:
(A) AUTHORS: WHARTON, KRISTI A.; THOMSEN, GERALD H.;
GELBERT, WILLIAM M.
(B) TITLE: DROSOPHILA 60A GENE...
(C) JOURNAL: PROC. NAT'L ACAD. SCI. USA
(D) VOLUME: 88
(E) RELEVANT RESIDUES IN SEQ ID NO:3: FROM 1 TO 1368
(F) PAGES: 9214-9218
(G) DATE: OCT - 1991

(xi) SEQUENCE DESCRIPTION: SEQ ID NO:24:

ATG TCG GGA CTG CGA AAC ACC TCG GAG GCC GTT GCA GTG CTC GCC TCC 48
Met Ser Gly Leu Arg Asn Thr Ser Glu Ala Val Ala Val Leu Ala Ser
1               5                   10                  15

CTG GGA CTC GGA ATG GTT CTG CTC ATG TTC GTG GCG ACC ACG CCG CCG 96
Leu Gly Leu Gly Met Val Leu Leu Met Phe Val Ala Thr Thr Pro Pro
            20                  25                  30

GCC GTT GAG GCC ACC CAG TCG GGG ATT TAC ATA GAC AAC GGC AAG GAC 144
Ala Val Glu Ala Thr Gln Ser Gly Ile Tyr Ile Asp Asn Gly Lys Asp
        35                  40                  45

CAG ACG ATC ATG CAC AGA GTG CTG AGC GAG GAC GAC AAG CTG GAC GTC 192
Gln Thr Ile Met His Arg Val Leu Ser Glu Asp Asp Lys Leu Asp Val
    50                  55                  60

TCG TAC GAG ATC CTC GAG TTC CTG GGC ATC GCC GAA CGG CCG ACG CAC 240
Ser Tyr Glu Ile Leu Glu Phe Leu Gly Ile Ala Glu Arg Pro Thr His
65                  70                  75                  80

CTG AGC AGC CAC CAG TTG TCG CTG AGG AAG TCG GCT CCC AAG TTC CTG 288
Leu Ser Ser His Gln Leu Ser Leu Arg Lys Ser Ala Pro Lys Phe Leu
                85                  90                  95

CTG GAC GTC TAC CAC CGC ATC ACG GCG GAG GAG GGT CTC AGC GAT CAG 336
Leu Asp Val Tyr His Arg Ile Thr Ala Glu Glu Gly Leu Ser Asp Gln
            100                 105                 110

GAT GAG GAC GAC GAC TAC GAA CGC GGC CAT CGG TCC AGG AGG AGC GCC 384
Asp Glu Asp Asp Asp Tyr Glu Arg Gly His Arg Ser Arg Arg Ser Ala
        115                 120                 125

GAC CTC GAG GAG GAT GAG GGC GAG CAG CAG AAG AAC TTC ATC ACC GAC 432
Asp Leu Glu Glu Asp Glu Gly Glu Gln Gln Lys Asn Phe Ile Thr Asp
    130                 135                 140

CTG GAC AAG CGG GCC ATC GAC GAG AGC GAC ATC ATC ATG ACC TTC CTG 480
Leu Asp Lys Arg Ala Ile Asp Glu Ser Asp Ile Ile Met Thr Phe Leu
145                 150                 155                 160

AAC AAG CGC CAC CAC AAT GTG GAC GAA CTG CGT CAC GAG CAC GGC CGT 528
Asn Lys Arg His His Asn Val Asp Glu Leu Arg His Glu His Gly Arg
                165                 170                 175

CGC CTG TGG TTC GAC GTC TCC AAC GTG CCC AAC GAC AAC TAC CTG GTG 576
Arg Leu Trp Phe Asp Val Ser Asn Val Pro Asn Asp Asn Tyr Leu Val
            180                 185                 190

ATG GCC GAG CTG CGC ATC TAT CAG AAC GCC AAC GAG GGC AAG TGG CTG 624
Met Ala Glu Leu Arg Ile Tyr Gln Asn Ala Asn Glu Gly Lys Trp Leu
        195                 200                 205

ACC GCC AAC AGG GAG TTC ACC ATC ACG GTA TAC GCC ATT GGC ACC GGC 672
Thr Ala Asn Arg Glu Phe Thr Ile Thr Val Tyr Ala Ile Gly Thr Gly
    210                 215                 220

ACG CTG GGC CAG CAC ACC ATG GAG CCG CTG TCC TCG GTG AAC ACC ACC 720
Thr Leu Gly Gln His Thr Met Glu Pro Leu Ser Ser Val Asn Thr Thr
225                 230                 235                 240

GGG GAC TAC GTG GGC TGG TTG GAG CTC AAC GTG ACC GAG GGC CTG CAC 768
Gly Asp Tyr Val Gly Trp Leu Glu Leu Asn Val Thr Glu Gly Leu His
                245                 250                 255

GAG TGG CTG GTC AAG TCG AAG GAC AAT CAT GGC ATC TAC ATT GGA GCA 816
Glu Trp Leu Val Lys Ser Lys Asp Asn His Gly Ile Tyr Ile Gly Ala
            260                 265                 270

CAC GCT GTC AAC CGA CCC GAC CGC GAG GTG AAG CTG GAC GAC ATT GGA 864
His Ala Val Asn Arg Pro Asp Arg Glu Val Lys Leu Asp Asp Ile Gly
        275                 280                 285

CTG ATC CAC CGC AAG GTG GAC GAC GAG TTC CAG CCC TTC ATG ATC GGC 912
Leu Ile His Arg Lys Val Asp Asp Glu Phe Gln Pro Phe Met Ile Gly
    290                 295                 300

TTC TTC CGC GGA CCG GAG CTG ATC AAG GCG ACG GCC CAC AGC AGC CAC 960
Phe Phe Arg Gly Pro Glu Leu Ile Lys Ala Thr Ala His Ser Ser His
305                 310                 315                 320

CAC AGG AGC AAG CGA AGC GCC AGC CAT CCA CGC AAG CGC AAG AAG TCG 1008
His Arg Ser Lys Arg Ser Ala Ser His Pro Arg Lys Arg Lys Lys Ser
                325                 330                 335

GTG TCG CCC AAC AAC GTG CCG CTG CTG GAA CCG ATG GAG AGC ACG CGC 1056
Val Ser Pro Asn Asn Val Pro Leu Leu Glu Pro Met Glu Ser Thr Arg
            340                 345                 350

AGC TGC CAG ATG CAG ACC CTG TAC ATA GAC TTC AAG GAT CTG GGC TGG 1104
Ser Cys Gln Met Gln Thr Leu Tyr Ile Asp Phe Lys Asp Leu Gly Trp
        355                 360                 365

CAT GAC TGG ATC ATC GCA CCA GAG GGC TAT GGC GCC TTC TAC TGC AGC 1152
His Asp Trp Ile Ile Ala Pro Glu Gly Tyr Gly Ala Phe Tyr Cys Ser
    370                 375                 380

GGC GAG TGC AAT TTC CCG CTC AAT GCG CAC ATG AAC GCC ACG AAC CAT 1200
Gly Glu Cys Asn Phe Pro Leu Asn Ala His Met Asn Ala Thr Asn His
385                 390                 395                 400

GCG ATC GTC CAG ACC CTG GTC CAC CTG CTG GAG CCC AAG AAG GTG CCC 1248
Ala Ile Val Gln Thr Leu Val His Leu Leu Glu Pro Lys Lys Val Pro
                405                 410                 415

AAG CCC TGC TGC GCT CCG ACC AGG CTG GGA GCA CTA CCC GTT CTG TAC 1296
Lys Pro Cys Cys Ala Pro Thr Arg Leu Gly Ala Leu Pro Val Leu Tyr
            420                 425                 430

CAC CTG AAC GAC GAG AAT GTG AAC CTG AAA AAG TAT AGA AAC ATG ATT 1344
His Leu Asn Asp Glu Asn Val Asn Leu Lys Lys Tyr Arg Asn Met Ile
        435                 440                 445

GTG AAA TCC TGC GGG TGC CAT TGA 1368
Val Lys Ser Cys Gly Cys His
    450                 455

(2)INFORMATION FOR SEQ ID NO:25:

(i) SEQUENCE CHARACTERISTICS:
(A) LENGTH: 455 amino acids
(B) TYPE: amino acid
(D) TOPOLOGY: linear

(ii) MOLECULE TYPE: protein

(xi) SEQUENCE DESCRIPTION: SEQ ID NO:25:

Met Ser Gly Leu Arg Asn Thr Ser Glu Ala Val Ala Val Leu Ala Ser
1               5                   10                  15

Leu Gly Leu Gly Met Val Leu Leu Met Phe Val Ala Thr Thr Pro Pro
            20                  25                  30

Ala Val Glu Ala Thr Gln Ser Gly Ile Tyr Ile Asp Asn Gly Lys Asp
        35                  40                  45

Gln Thr Ile Met His Arg Val Leu Ser Glu Asp Asp Lys Leu Asp Val
    50                  55                  60

Ser Tyr Glu Ile Leu Glu Phe Leu Gly Ile Ala Glu Arg Pro Thr His
65                  70                  75                  80

Leu Ser Ser His Gln Leu Ser Leu Arg Lys Ser Ala Pro Lys Phe Leu
                85                  90                  95

Leu Asp Val Tyr His Arg Ile Thr Ala Glu Glu Gly Leu Ser Asp Gln
            100                 105                 110

Asp Glu Asp Asp Asp Tyr Glu Arg Gly His Arg Ser Arg Arg Ser Ala
        115                 120                 125

Asp Leu Glu Glu Asp Glu Gly Glu Gln Gln Lys Asn Phe Ile Thr Asp
    130                 135                 140

Leu Asp Lys Arg Ala Ile Asp Glu Ser Asp Ile Ile Met Thr Phe Leu
145                 150                 155                 160

Asn Lys Arg His His Asn Val Asp Glu Leu Arg His Glu His Gly Arg
                165                 170                 175

Arg Leu Trp Phe Asp Val Ser Asn Val Pro Asn Asp Asn Tyr Leu Val
            180                 185                 190

Met Ala Glu Leu Arg Ile Tyr Gln Asn Ala Asn Glu Gly Lys Trp Leu
        195                 200                 205

Thr Ala Asn Arg Glu Phe Thr Ile Thr Val Tyr Ala Ile Gly Thr Gly
    210                 215                 220

Thr Leu Gly Gln His Thr Met Glu Pro Leu Ser Ser Val Asn Thr Thr
225                 230                 235                 240

Gly Asp Tyr Val Gly Trp Leu Glu Leu Asn Val Thr Glu Gly Leu His
                245                 250                 255

Glu Trp Leu Val Lys Ser Lys Asp Asn His Gly Ile Tyr Ile Gly Ala
            260                 265                 270

His Ala Val Asn Arg Pro Asp Arg Glu Val Lys Leu Asp Asp Ile Gly
        275                 280                 285

Leu Ile His Arg Lys Val Asp Asp Glu Phe Gln Pro Phe Met Ile Gly
    290                 295                 300

Phe Phe Arg Gly Pro Glu Leu Ile Lys Ala Thr Ala His Ser Ser His
305                 310                 315                 320

His Arg Ser Lys Arg Ser Ala Ser His Pro Arg Lys Arg Lys Lys Ser
                325                 330                 335

Val Ser Pro Asn Asn Val Pro Leu Leu Glu Pro Met Glu Ser Thr Arg
            340                 345                 350

Ser Cys Gln Met Gln Thr Leu Tyr Ile Asp Phe Lys Asp Leu Gly Trp
        355                 360                 365

His Asp Trp Ile Ile Ala Pro Glu Gly Tyr Gly Ala Phe Tyr Cys Ser
    370                 375                 380

Gly Glu Cys Asn Phe Pro Leu Asn Ala His Met Asn Ala Thr Asn His
385                 390                 395                 400

Ala Ile Val Gln Thr Leu Val His Leu Leu Glu Pro Lys Lys Val Pro
                405                 410                 415

Lys Pro Cys Cys Ala Pro Thr Arg Leu Gly Ala Leu Pro Val Leu Tyr
            420                 425                 430

His Leu Asn Asp Glu Asn Val Asn Leu Lys Lys Tyr Arg Asn Met Ile
        435                 440                 445

Val Lys Ser Cys Gly Cys His
    450                 455

(2) INFORMATION FOR SEQ ID NO:26:

(i) SEQUENCE CHARACTERISTICS:
(A) LENGTH: amino acids
(B) TYPE: amino acid
(D) TOPOLOGY: linear

(ii) MOLECULE TYPE: protein

(iii) ORIGINAL SOURCE:
(A) ORGANISM: Homo Sapiens

(ix) FEATURE:
(A) NAME/KEY: Protein
(B) LOCATION: 1..102
(D) OTHER INFORMATION: /note="BMP3"

(xi) SEQUENCE DESCRIPTION: SEQ ID NO:26:

(i)SEQUENCE CHARACTERISTICS:
(A) LENGTH: 104 amino acids
(B) TYPE: amino acid
(C) STRANDEDNESS: single
(D) TOPOLOGY: linear

(ii)MOLECULE TYPE: protein

(ix)FEATURE:
(A) NAME/KEY: Protein
(B) LOCATION: 1..104
(D) OTHER INFORMATION: /note="BMP3"

(xi)SEQUENCE DESCRIPTION: SEQ ID NO:26:

Cys Ala Arg Arg Tyr Leu Lys Val Asp Phe Ala Asp Ile Gly Trp Ser
1               5                   10                  15

Glu Trp Ile Ile Ser Pro Lys Ser Phe Asp Ala Tyr Try Cys Ser Gly
            20                  25                  30

Ala Cys Gln Phe Pro Met Pro Lys Ser Leu Lys Pro Ser Asn His Ala
        35                  40                  45

Thr Ile Gln Ser Ile Val Ala Arg Ala Val Gly Val Val Pro Gly Ile
    50                  55                  60

Pro Glu Pro Cys Cys Val Pro Glu Lys Met Ser Ser Leu Ser Ile Leu
65                  70                  75                  80

Phe Phe Asp Glu Asn Lys Asn Val Val Leu Lys Val Tyr Pro Asn Met
                85                  90                  95

Thr Val Glu Ser Cys Ala Cys Arg
            100

(2) INFORMATION FOR SEQ ID NO:27:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS

(ix) FEATURE:
(A) NAME/KEY: Protein
(B) LOCATION: 1..102
(D) OTHER INFORMATION: /note= "BMP5"

(xi) SEQUENCE DESCRIPTION: SEQ ID NO:27:

Cys Lys Lys His Glu Leu Tyr Val Ser Phe Arg Asp Leu Gly Trp Gln
1               5                   10                  15

Asp Trp Ile Ile Ala Pro Glu Gly Tyr Ala Ala Phe Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Leu Met Phe Pro Asp His Val Pro Lys
    50                  55                  60

Pro Cys Cys Ala Pro Thr Lys Leu Asn Ala Ile Ser Val Leu Tyr Phe
65                  70                  75                  80

Asp Asp Ser Ser Asn Val Ile Leu Lys Lys Tyr Arg Asn Met Val Val
                85                  90                  95

Arg Ser Cys Gly Cys His
            100

(2) INFORMATION FOR SEQ ID NO:28:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS

(ix) FEATURE:
(A) NAME/KEY: Protein
(B) LOCATION: 1..102
(D) OTHER INFORMATION: /note= "BMP6"

(xi) SEQUENCE DESCRIPTION: SEQ ID NO:28:

Cys Arg Lys His Glu Leu Tyr Val Ser Phe Gln Asp Leu Gly Trp Gln
1               5                   10                  15

Asp Trp Ile Ile Ala Pro Lys Gly Tyr Ala Ala Asn Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Leu Met Asn Pro Glu Tyr Val Pro Lys
    50                  55                  60

Pro Cys Cys Ala Pro Thr Lys Leu Asn Ala Ile Ser Val Leu Tyr Phe
65                  70                  75                  80

Asp Asp Asn Ser Asn Val Ile Leu Lys Lys Tyr Arg Trp Met Val Val
                85                  90                  95

Arg Ala Cys Gly Cys His
            100

(2) INFORMATION FOR SEQ ID NO:29:

(i) SEQUENCE CHARACTERISTICS:
(A) LENGTH: 102 amino acids
(B) TYPE: amino acid
(D) TOPOLOGY: linear

(ii) MOLECULE TYPE: protein

(ix) FEATURE:
(A) NAME/KEY: Protein
(B) LOCATION: 1..102
(D) OTHER INFORMATION: /label= OPX
/note= "WHEREIN XAA AT EACH POS'N IS INDEPENDENTLY
SELECTED FROM THE RESIDUES OCCURRING AT THE
CORRESPONDING POS'N IN THE C-TERMINAL SEQUENCE OF MOUSE
OR HUMAN OP1 OR OP2 (SEE SEQ. ID NOS. 5,6,7 and 8 or
16,18,20 and 22.)"

(xi) SEQUENCE DESCRIPTION: SEQ ID NO:29:

Cys Xaa Xaa His Glu Leu Tyr Val Xaa Phe Xaa Asp Leu Gly Trp Xaa
1               5                   10                  15

Asp Trp Xaa Ile Ala Pro Xaa Gly Tyr Xaa Ala Tyr Tyr Cys Glu Gly
            20                  25                  30

Glu Cys Xaa Phe Pro Leu Xaa Ser Xaa Met Asn Ala Thr Asn His Ala
        35                  40                  45

Ile Xaa Gln Xaa Leu Val His Xaa Xaa Xaa Pro Xaa Xaa Val Pro Lys
    50                  55                  60

Xaa Cys Cys Ala Pro Thr Xaa Leu Xaa Ala Xaa Ser Val Leu Tyr Xaa
65                  70                  75                  80

Asp Xaa Ser Xaa Asn Val Xaa Leu Xaa Lys Xaa Arg Asn Met Val Val
                85                  90                  95

Xaa Ala Cys Gly Cys His
            100

(2) INFORMATION FOR SEQ ID NO:30:

(i)SEQUENCE CHARACTERISTICS:
(A) LENGTH: 97 amino acids
(B) TYPE: amino acids
(C) TOPOLOGY: linear
(ii)MOLECULE TYPE: protein
(ix)FEATURE:
(A) NAME: Generic Sequence 5
(D) OTHER INFORMATION: wherein each Xaa is independently
selected from a group of one or more specified amino acids as
defined in the specification.

(xi)SEQUENCE DESCRIPTION: SEQ ID NO:30:

Leu Xaa Xaa Xaa Phe
1               5
Xaa Xaa Xaa Gly Trp Xaa Xaa Trp Xaa
                10
Xaa Xaa Pro Xaa Xaa Xaa Xaa Ala
15                  20
Xaa Tyr Cys Xaa Gly Xaa Cys Xaa
        25                  30
Xaa Pro Xaa Xaa Xaa Xaa Xaa
                35
Xaa Xaa Xaa Asn His Ala Xaa Xaa
        40                  45
Xaa Xaa Xaa Xaa Xaa Xaa Xaa Xaa
                50
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
70                  75
Xaa Xaa Xaa Xaa Val Xaa Leu Xaa
            80
Xaa Xaa Xaa Xaa Met Xaa Val Xaa
85                  90
Xaa Cys Xaa Cys Xaa
        95

(2) INFORMATION FOR SEQ ID NO:31:

(i)SEQUENCE CHARACTERISTICS:
(A) LENGTH: 102 amino acids
(B) TYPE: amino acids
(C) TOPOLOGY: linear
(ii)MOLECULE TYPE: protein
(ix)FEATURE:
(A) NAME: Generic Sequence 6
(D) OTHER INFORMATION: wherein each Xaa is independently
selected from a group of one or more specified amino acids as
defined in the specification.

(xi) SEQUENCE DESCRIPTION: SEQ ID NO:31:

Cys Xaa Xaa Xaa Xaa Leu Xaa Xaa Xaa Phe
1               5                   10
Xaa Xaa Xaa Gly Trp Xaa Xaa Trp Xaa
                15
Xaa Xaa Pro Xaa Xaa Xaa Xaa Ala
20                  25
Xaa Tyr Cys Xaa Gly Xaa Cys Xaa
        30                  35
Xaa Pro Xaa Xaa Xaa Xaa Xaa
                40
Xaa Xaa Xaa Asn His Ala Xaa Xaa
        45                  50
Xaa Xaa Xaa Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70
Xaa Xaa Xaa Leu Xaa Xaa Xaa
75                  80
Xaa Xaa Xaa Xaa Val Xaa Leu Xaa
            85
Xaa Xaa Xaa Xaa Met Xaa Val Xaa
90                  95
Xaa Cys Xaa Cys Xaa
        100

(2) INFORMATION FOR SEQ ID NO:32:

(i) SEQUENCE CHARACTERISTICS:
(A) LENGTH: 1238 base pairs, 372 amino acids
(B) TYPE: nucleic acid, amino acid
(C) STRANDEDNESS: single
(D) TOPOLOGY: linear

(ii) MOLECULE TYPE: cDNA

(iii) ORIGINAL SOURCE:
(A) ORGANISM: human
(F) TISSUE TYPE: BRAIN

(iv) FEATURE:
(A) NAME/KEY: CDS
(B) LOCATION:
(D) OTHER INFORMATION:
/product= "GDF-1"
/note= "GDF-1 CDNA"

(x) PUBLICATION INFORMATION:
(A) AUTHORS: Lee, Se-Jin
(B) TITTLE: Expression of Growth/Differentiation Factor 1
(C) JOURNAL: Proc. Nat'l Acad. Sci.
(D) VOLUME: 88
(E) RELEVANT RESIDUES: 1-1238
(F) PAGES: 4250-4254
(G) DATE: May-1991
(xi) SEQUENCE DESCRIPTION: SEQ ID NO:32:

GGGGACACCG GCCCCGCCCT CAGCCCACTG GTCCCGGGCC GCCGCGGACC CTGCGCACTC 60

TCTGGTCATC GCCTGGGAGG AAG ATG CCA CCG CCG CAG CAA GGT CCC TGC GGC 113
Met Pro Pro Pro Gln Gln Gly Pro Cys Gly
1               5                   10

CAC CAC CTC CTC CTC CTC CTG GCC CTG CTG CTG CCC TCG CTG CCC 158
His His Leu Leu Leu Leu Leu Ala Leu Leu Leu Pro Ser Leu Pro
                15                  20                  25

CTG ACC CGC GCC CCC GTG CCC CCA GGC CCA GCC GCC GCC CTG CTC 203
Leu Thr Arg Ala Pro Val Pro Pro Gly Pro Ala Ala Ala Leu Leu
                30                  35                  40

CAG GCT CTA GGA CTG CGC GAT GAG CCC CAG GGT GCC CCC AGG CTC 248
Gln Ala Leu Gly Leu Arg Asp Glu Pro Gln Gly Ala Pro Arg Leu
                45                  50                  55

CGG CCG GTT CCC CCG GTC ATG TGG CGC CTG TTT CGA CGC CGG GAC     293
Arg Pro Val Pro Pro Val Met Trp Arg Leu Phe Arg Arg Arg Asp
                    60                  65                  70

CCC CAG GAG ACC AGG TCT GGC TCG CGG CGG ACG TCC CCA GGG GTC     338
Pro Gln Glu Thr Arg Ser Gly Ser Arg Arg Thr Ser Pro Gly Val
                    75                  80                  85

ACC CTG CAA CCG TGC CAC GTG GAG GAG CTG GGG GTC GCC GGA AAC     383
Thr Leu Gln Pro Cyc His Val Glu Glu Leu Gly Val Ala Gly Asn
                    90                  95                 100

ATC GTG CGC CAC ATC CCG GAC CGC GGT GCG CCC ACC CGG GCC TCG     428
Ile Val Arg His Ile Pro Asp Arg Gly Ala Pro Thr Arg Ala Ser
                   105                 110                 115

GAG CCT GTC TCG GCC GCG GGG CAT TGC CCT GAG TGG ACA GTC GTC     473
Glu Pro Val Ser Ala Ala Gly His Cys Pro Glu Trp Thr Val Val
                   120                 125                 130

TTC GAC CTG TCG GCT GTG GAA CCC GCT GAG CGC CCG AGC CGG GCC     518
Phe Asp Leu Ser Ala Val Glu Pro Ala Glu Arg Pro Ser Arg Ala
                   135                 140                 145

CGC CTG GAG CTG CGT TTC GCG GCG GCG GCG GCG GCA GCC CCG GAG     563
Arg Leu Glu Leu Arg Phe Ala Ala Ala Ala Ala Ala Ala Pro Glu
                   150                 155                 160

GGC GGC TGG GAG CTG AGC GTG GCG CAA GCG GGC CAG GGC GCG GGC     608
Gly Gly Trp Glu Leu Ser Val Ala Gln Ala Gly Gln Gly Ala Gly
                   165                 170                 175

GCG GAC CCC GGG CCG GTG CTG CTC CGC CAG TTG GTG CCC GCC CTG     653
Ala Asp Pro Gly Pro Val Leu Leu Arg Gln Leu Val Pro Ala Leu
                   180                 185                 190

GGG CCG CCA GTG CGC GCG GAG CTG CTG GGC GCC GCT TGG GCT CGC     698
Gly Pro Pro Val Arg Ala Glu Leu Leu Gly Ala Ala Trp Ala Arg
                   195                 200                 205

AAC GCC TCA TGG CCG CGC AGC CTC CGC CTG GCG CTG GCG CTA CGC     743
Asn Ala Ser Trp Pro Arg Ser Leu Arg Leu Ala Leu Ala Leu Arg
                   210                 215                 220

CCC CGG GCC CCT GCC GCC TGC GCG CGC CTG GCC GAG GCC TCG CTG     788
Pro Arg Ala Pro Ala Ala Cys Ala Arg Leu Ala Glu Ala Ser Leu
                   225                 230                 235

CTG CTG GTG ACC CTC GAC CCG CGC CTG TGC CAC CCC CTG GCC CGG     833
Leu Leu Val Thr Leu Asp Pro Arg Leu Cys His Pro Leu Ala Arg
                   240                 245                 250

CCG CGG CGC GAC GCC GAA CCC GTG TTG GGC GGC GGC CCC GGG GGC     878
Pro Arg Arg Asp Ala Glu Pro Val Leu Gly Gly Gly Pro Gly Gly
                255                 260                 265

GCT TGT CGC GCG CGG CGG CTG TAC GTG AGC TTC CGC CAG GTG GGC     923
Ala Cys Arg Ala Arg Arg Leu Tyr Val Ser Phe Arg Glu Val Gly
                270                 275                 280

TGG CAC CGC TGG GTC ATC GCG CCG CGC CCC TTC CTG GCC AAC TAC     968
Trp His Arg Trp Val Ile Arg Pro Arg Gly Phe Leu Ala Asn Tyr
                285                 290                 295

TGC CAG GGT CAG TGC GCG CTG CCC GTC GCG CTG TCG GGG TCC GGG    1013
Cys Gln Gly Gln Cys Ala Leu Pro Val Ala Leu Ser Gly Ser Gly
                300                 305                 310

GGG CCG CCG GCG CTC AAC CAC GCT GTG CTG CGC GCG CTC ATG CAC    1058
Gly Pro Pro Ala Leu Asn His Ala Val Leu Arg Ala Leu Met His
                315                 320                 325

GCG GCC GCC CCG GGA GCC GCC GAC CTG CCC TGC TGC GTG CCC GCG    1103
Ala Ala Ala Pro Gly Ala Ala Asp Leu Pro Cys Cys Val Pro Ala
                330                 335                 340

CGC CTG TCG CCC ATC TCC GTG CTC TTC TTT GAC AAC AGC GAC AAC    1148
Arg Leu Ser Pro Ile Ser Val Leu Phe Phe Asp Asn Ser Asp Asn
                345                 350                 355

GTG GTG CTG CGG CAG TAT GAG GAC ATG GTG GTG GAC GAG TGC GGC    1193
Val Val Leu Arg Gln Tyr Glu Asp Met Val Val Asp Glu Cys Gly
                360                 365                 370

TGC CGC TAACCCGGGG CGGGCAGGGA CCCGGCCCA ACAATAAATG CCGCGTGG    1238
Cys Arg
    372

(34)   INFORMATION FOR SEQ ID NO:33:

(i)   SEQUENCE CHARACTERISTICS:
(A)   LENGTH: 372 amino acids
(B)   TYPE: amino acid
(C)   STRANDEDNESS: single
(D)   TOPOLOGY: linear

(ii)  MOLECULE TYPE: cDNA

(iii) HYPOTHETICAL: NO

(iv)  ANTI-SENSE: NO

(vi)  ORIGINAL SOURCE:
(A)   ORGANISM: human
(F)   TISSUE TYPE: BRAIN

(ix)  FEATURE:
(A)   NAME/KEY: CDS
(B)   LOCATION:
(D)   OTHER INFORMATION: /function=
/product= "GDF-1"

(xi) SEQUENCE DESCRIPTION: SEQ ID NO:33:

Met Pro Pro Pro Gln Gln Gly Pro Cys Gly
1               5                   10

His His Leu Leu Leu Leu Leu Ala Leu Leu Leu Pro Ser Leu Pro
                15                  20                  25

Leu Thr Arg Ala Pro Val Pro Pro Gly Pro Ala Ala Ala Leu Leu
                30                  35                  40

Gln Ala Leu Gly Leu Arg Asp Glu Pro Gln Gly Ala Pro Arg Leu
                45                  50                  55

Arg Pro Val Pro Pro Val Met Trp Arg Leu Phe Arg Arg Arg Asp
                60                  65                  70

Pro Gln Glu Thr Arg Ser Gly Ser Arg Arg Thr Ser Pro Gly Val
                75                  80                  85

Thr Leu Gln Pro Cyc His Val Glu Glu Leu Gly Val Ala Gly Asn
                90                  95                  100

Ile Val Arg His Ile Pro Asp Arg Gly Ala Pro Thr Arg Ala Ser
                105                 110                 115

Glu Pro Val Ser Ala Ala Gly His Cys Pro Glu Trp Thr Val Val
                120                 125                 130

Phe Asp Leu Ser Ala Val Glu Pro Ala Glu Arg Pro Ser Arg Ala
                135                 140                 145

Arg Leu Glu Leu Arg Phe Ala Ala Ala Ala Ala Ala Ala Pro Glu
                150                 155                 160

Gly Gly Trp Glu Leu Ser Val Ala Gln Ala Gly Gln Gly Ala Gly
                165                 170                 175

Ala Asp Pro Gly Pro Val Leu Leu Arg Gln Leu Val Pro Ala Leu
                180                 185                 190

Gly Pro Pro Val Arg Ala Glu Leu Leu Gly Ala Ala Trp Ala Arg
                195                 200                 205

Asn Ala Ser Trp Pro Arg Ser Leu Arg Leu Ala Leu Ala Leu Arg
                210                 215                 220

Pro Arg Ala Pro Ala Ala Cys Ala Arg Leu Ala Glu Ala Ser Leu
                225                 230                 235

Leu Leu Val Thr Leu Asp Pro Arg Leu Cys His Pro Leu Ala Arg
                240                 245                 250

Pro Arg Arg Asp Ala Glu Pro Val Leu Gly Gly Gly Pro Gly Gly
                255                 260                 265

Ala Cys Arg Ala Arg Arg Leu Tyr Val Ser Phe Arg Glu Val Gly
                270                 275                 280

Trp His Arg Trp Val Ile Arg Pro Arg Gly Phe Leu Ala Asn Tyr
                285                 290                 295

Cys Gln Gly Gln Cys Ala Leu Pro Val Ala Leu Ser Gly Ser Gly
                300                 305                 310

Gly Pro Pro Ala Leu Asn His Ala Val Leu Arg Ala Leu Met His
                315                 320                 325

Ala Ala Ala Pro Gly Ala Ala Asp Leu Pro Cys Cys Val Pro Ala
                330                 335                 340

Arg Leu Ser Pro Ile Ser Val Leu Phe Phe Asp Asn Ser Asp Asn
                345                 350                 355

Val Val Leu Arg Gln Tyr Glu Asp Met Val Val Asp Glu Cys Gly
                360                 365                 370

Cys Arg
    372

## Claims

1. A method of screening candidate compounds for the ability to modulate expression of a cellular gene encoding a polypeptide, the amino acid sequence of said polypeptide comprising
(I) a sequence having at least 70% amino acid sequence homology with the C-terminal seven cysteine domain of human OP-1, residues 38 to 139 of SEQ ID No. 5, or
(II) a sequence defined by Generic Sequence 6, SEQ ID No. 31, such that said polypeptide, when dimerized with a second said polypeptide, forms a protein having the property of inducing a developmental cascade of tissue-specific morphogenesis in a mammal, said method comprising the steps of:
(a) incubating a candidate compound with epithelial cells, which express said protein, for a time sufficient to allow said compound to affect the expression of a gene present in said cells and encoding said polypeptide; and
(b) assaying said epithelial cells for the presence or amount of an expression product of said gene, wherein a change in the level of said expression product relative to the level thereof in said epithelial cells in the absence of said candidate compound is indicative of the ability of said compound to modulate expression of said gene in said epithelial cells.

2. The method of Claim 1 wherein the amino acid sequence of said polypeptide comprises a sequence defined by OPX, SEQ ID No. 29.

3. The method of Claim 1 or Claim 2 wherein said epithelial cells are cultured from brain or renal tissue.

4. The method of Claim 1 or Claim 2 wherein said epithelial cells are cultured from kidney, bladder or adrenal tissue.

5. The method of Claim 1 or Claim 2 wherein said epithelial cells are epithelial carcinoma cells.

6. The method of Claim 5 wherein said epithelial cells are an immortalized human bladder carcinoma cell line.

7. The method of Claim 1 wherein the amino acid sequence of said polypeptide comprises a sequence having at least 80% homology with the C-terminal seven cysteine domain of human OP-1, residues 38 to 139 of SEQ ID No. 5.

8. The method of Claim 1 wherein the amino acid sequence of said polypeptide comprises a sequence having at least 60% amino acid identity with the C-terminal seven cysteine domain of human OP-1, residues 38 to 139 of SEQ ID No. 5.

9. The method of Claim 1 wherein the amino acid sequence of said polypeptide comprises a sequence having at least 65% amino acid identity with the C-terminal seven cysteine domain of human OP-1, residues 38 to 139 of SEQ ID No. 5.

10. The method of Claim 1 or Claim 9 wherein the presence or amount of said expression product is assayed with antibody selectively reactive with a polypeptide encoded by said gene.

11. The method of Claim 1 or Claim 9 wherein the presence or amount of said expression product is assayed by measuring cellular proliferation in cells which are sensitive to the concentration of said protein having morphogenic activity secreted by said incubated epithelial cells.

12. The method of Claim 1 or Claim 9 wherein the presence or amount of said expression product is assayed using a nucleic acid probe that hybridizes, under stringent conditions, with RNA transcribed from said gene.

13. The method of Claim 12 wherein said nucleic acid probe hybridizes with an mRNA transcript having a nucleic acid sequence comprising a sequence encoding a polypeptide region N-terminal to residue 38 of human OP-1, SEQ ID No. 5.

14. The method of Claim 12 wherein said nucleic acid probe hybridizes with an mRNA transcript having a nucleic acid sequence comprising a sequence transcribed from a gene region 3' to the gene region encoding residue 139 of human OP-1, SEQ ID No. 5.

## Patentansprüche

1. Verfahren zum Screenen von Test-Verbindungen auf die Fähigkeit, die Expression eines zellulären Gens, das ein Polypeptid kodiert, zu modulieren, wobei die Aminosäure-Sequenz des Polypeptids folgendes umfaßt:
(i) eine Sequenz mit zumindest 70 % Aminosäuresequenz-Homologie mit der C-terminalen Sieben-Cystein-Domäne von menschlichem OP-1, Reste 38 bis 139 von SEQ ID Nr. 5, oder
(ii) eine durch Gattungssequenz 6, SEQ ID Nr. 31 definierte Sequenz, derartig, daß das Polypeptid, wenn es mit einem zweiten derartigen Polypeptid dimerisiert wird, ein Protein mit der Eigenschaft bildet, eine Entwicklungs-Kaskade einer gewebespezifischen Morphogenese bei einem Säugetier zu induzieren, wobei das Verfahren die folgenden Schritte umfaßt:
(a) Inkubieren einer Test-Verbindung mit Epithelzellen, die das Protein exprimieren, für einen Zeitraum, der dazu ausreicht, der Verbindung zu ermöglichen, die Expression eines Gens zu beeinflussen, das in den Zellen vorliegt, und das das Polypeptid kodiert; und
(b) Untersuchen der Epithelzellen auf das Vorhandensein oder die Menge eines Expressionsprodukts des Gens, wobei eine Veränderung im Spiegel des Expressionsproduktes bezüglich dessen Spiegel in den Epithelzellen bei Fehlen der Testverbindung, auf die Fähigkeit der Verbindung hinweist, eine Expression des Gens in den Epithelzellen zu modulieren.

2. Verfahren nach Anspruch 1,
bei dem die Aminosäure-Sequenz des Polypeptids eine durch OPX, SEQ ID Nr. 29 definierte Sequenz umfaßt.

3. Verfahren nach Anspruch 1 oder 2,
bei dem die Epithelzellen aus Gehirn- oder Nierengewebe gezüchtet werden.

4. Verfahren nach Anspruch 1 oder Anspruch 2,
bei dem die Epithelzellen aus Nieren-, Blasen- oder Nebennieren-Gewebe gezüchtet werden.

5. Verfahren nach Anspruch 1 oder Anspruch 2,
bei dem die Epithelzellen Epithel-Karzinomzellen sind.

6. Verfahren nach Anspruch 5,
bei dem die Epithelzellen eine unsterbliche menschliche Blasen-Karzinom-Zell-Linie sind.

7. Verfahren nach Anspruch 1,
bei dem die Aminosäure-Sequenz des Polypeptids eine Sequenz mit zumindest 80 % Homologie mit der C-terminalen Sieben-Cystein-Domäne von menschlichem OP-1, Reste 38 bis 139 von SEQ ID Nr. 5 umfaßt.

8. Verfahren nach Anspruch 1,
bei dem die Aminosäure-Sequenz des Polypeptids eine Sequenz mit zumindest 60 % Aminosäure-Identität mit der C-terminalen Sieben-Cystein-Domäne von menschlichem OP-1, Reste 38 bis 139 von SEQ ID Nr. 5 umfaßt.

9. Verfahren nach Anspruch 1,
bei dem die Aminosäure-Sequenz des Polypeptids eine Sequenz mit zumindest 65 % Aminosäure-Identität mit der C-terminalen Sieben-Cystein-Domäne von menschlichem OP-1, Reste 38 bis 139 von SEQ ID Nr. 5 umfaßt.

10. Verfahren nach Anspruch 1 oder Anspruch 9,
bei dem das Vorhandensein oder die Menge des Expressionsprodukts mit einem Antikörper untersucht wird, der mit einem von dem Gen kodierten Polypeptid selektiv reaktiv ist.

11. Verfahren nach Anspruch 1 oder Anspruch 9,
bei dem das Vorhandensein oder die Menge des Expressionsprodukts untersucht wird, indem die Zellproliferation bei Zellen gemessen wird, die gegenüber der Konzentration des Proteins, das eine morphogenetische Aktivität aufweist, und das durch die inkubierten Epithelzellen sezerniert wird, empfindlich sind.

12. Verfahren nach Anspruch 1 oder Anspruch 9,
bei dem das Vorhandensein oder die Menge des Expressionsprodukts untersucht wird, indem eine Nukleinsäure-Sonde verwendet wird, die, unter stringenten Bedingungen, mit von dem Gen transkribierter RNA hybridisiert.

13. Verfahren nach Anspruch 12,
bei dem die Nukleinsäure-Sonde mit einem mRNA-Transcript mit einer Nukleinsäure-Sequenz hybridisiert, die einen zu Rest 38 von menschlichem OP-1, SEQ ID Nr. 5 N-terminalen Bereich eines Polypeptids kodiert.

14. Verfahren nach Anspruch 12,
bei dem die Nukleinsäure-Sonde mit einem mRNA Transcript mit einer Nukleinsäure-Sequenz hybridisiert, die eine von einem Gen-Bereich transkribierte Sequenz umfaßt, in die 3'-Richtung zu der Gen-Region liegt, die Rest 139 von menschlichem OP-1, SEQ ID Nr. 5 kodiert.

## Revendications

1. Procédé de criblage de composés candidats pour l'aptitude à moduler l'expression d'un gène cellulaire codant un polypetitde, la séquence d'acides aminés dudit polypeptide comprenant:
(I) une séquence ayant au moins 70 % d'homologie de séquence d'acides aminés avec le domaine à sept cystéines C-terminal de OP-1 humain, résidus 38 à 139 de SEQ. ID n° 5, ou
(II) une séquence définie par la séquence générique 6, SEQ. ID n° 31, telle que ledit polypeptide, quand il est dimérisé avec un second polypeptide de ce type, forme une protéine ayant la propriété d'induire une cascade développementale de morphogenèse spécifique de tissu dans un mammifère, ledit procédé comprenant les étapes de :
(a) incubation d'un composé candidat avec des cellules épithéliales qui expriment ladite protéine, pendant une durée suffisante pour permettre audit composé d'affecter l'expression d'un gène présent dans lesdites cellules et codant ledit polypeptide ; et
(b) test desdites cellules épithéliales pour la présence ou la quantité d'un produit d'expression dudit gène, où une variation du niveau dudit produit d'expression par rapport à son niveau dans lesdites cellules épithéliales en l'absence dudit composé candidat indique l'aptitude dudit composé à moduler l'expression dudit gène dans lesdites cellules épithéliales.

2. Procédé selon la revendication 1, où la séquence d'acides aminés dudit polypeptide comprend une séquence définie par OPX, SEQ. ID n° 29.

3. Procédé selon la revendication 1 ou la revendication 2, où lesdites cellules épithéliales sont cultivées à partir de tissu cérébral ou rénal.

4. Procédé selon la revendication 1 ou la revendication 2, où lesdites cellules épithéliales sont cultivées à partir de tissu rénal, vésical ou surrénal.

5. Procédé selon la revendication 1 ou la revendication 2, où lesdites cellules épithéliales sont des cellules de carcinome épithélial.

6. Procédé selon la revendication 5, où lesdites cellules épithéliales sont une lignée cellulaire de carcinome vésical humain immortalisée.

7. Procédé selon la revendication 1, où la séquence d'acides aminés dudit polypeptide comprend une séquence ayant au moins 80 % d'homologie avec le domaine à sept cystéines C-terminal de OP-1 humain, résidus 38 à 139 de SEQ. ID n° 5.

8. Procédé selon la revendication 1, où la séquence d'acides aminés dudit polypeptide comprend une séquence ayant au moins 60% d'identité d'acides aminés avec le domaine à sept cystéines C-terminal de OP-1 humain, résidus 38 à 139 de SEQ. ID n° 5.

9. Procédé selon la revendication 1, où la séquence d'acides aminés dudit polypeptide comprend une séquence ayant au moins 65 % d'identité d'acides aminés avec le domaine à sept cystéines C-terminal de OP-1 humain, résidus 38 à 139 de SEQ. ID n° 5.

10. Procédé selon la revendication 1 ou la revendication 9, où la présence ou la quantité dudit produit d'expression est testée avec un anticorps réactif sélectivement avec un polypeptide codé par ledit gène.

11. Procédé selon la revendication 1 ou la revendication 9, où la présence ou la quantité dudit produit d'expression est testée par mesure de la prolifération cellulaire dans des cellules qui sont sensibles à la concentration de ladite protéine ayant une activité morphogène sécrétée par lesdites cellules épithéliales incubées.

12. Procédé selon la revendication 1 ou la revendication 9, où la présence ou la quantité dudit produit d'expression est testée au moyen d'une sonde d'acide nucléique qui s'hybride, dans des conditions stringentes, avec un ARN transcrit à partir dudit gène.

13. Procédé selon la revendication 12, où ladite sonde d'acide nucléique s'hybride avec un transcrit d'ARNm ayant une séquence d'acide nucléique comprenant une séquence codant une région polypeptidique N-terminale jusqu'au résidu 38 de OP-1 humain, SEQ. ID n° 5.

14. Procédé selon la revendication 12, où ladite sonde d'acide nucléique s'hybride avec un transcrit d'ARNm ayant une séquence d'acide nucléique comprenant une séquence transcrite d'une région 3' de gène à la région de gène codant le résidu 139 de OP-1 humain, SEQ. ID n° 5.
